# EUROPEAN PATENT APPLICATION

(11) **EP 4 578 960 A1**
(43) Date of publication of application: **02.07.2025**
(21) Application number: 24221378.3
(22) Date of filing: 19.12.2024
(51) Int. Cl.: C12Q 1/6841

(54) **IN SITU DETECTION OF COPY NUMBER VARIATIONS IN BIOLOGICAL SAMPLES**

(30) Priority: 20.12.2023 US 202363612716 P
(71) Applicant: 10x Genomics, Inc., Pleasanton, CA 94588-3260 (US)
(72) Inventor: SCHNALL-LEVIN, Michael, Pleasanton, 94588-3260 (US)
(74) Representative: J A Kemp LLP

(57) **Abstract**

The present disclosure relates in some aspects to methods, systems, and kits for analyzing copy number of a genomic region, including at spatial regions in a biological sample. The methods can comprise contacting the biological sample with a plurality of probes for detecting RNA expression from a plurality of subregions within a genomic region, wherein the plurality of probes comprises at least two different probes capable of hybridizing to at least two different RNA molecules expressed from different subregions within the genomic region, and wherein the plurality of probes individually comprise a common barcode sequence that identifies the genomic region. Detection of the common barcode sequence for the genomic region can be used to infer copy number.

## Description

### CROSS-REFERENCE TO RELATED APPLICATIONS

This application claims priority to U.S. Provisional Patent Application No. 63/612,716, filed December 20th, 2023, entitled "IN SITU DETECTION OF COPY NUMBER VARIATIONS IN BIOLOGICAL SAMPLES," which is herein incorporated by reference in its entirety for all purposes.

### FIELD

The present disclosure relates in some aspects to methods for *in situ* detection of RNA to analyze copy number variation in a biological sample.

### BACKGROUND

Genetic variants, such as insertions, deletions, substitutions, rearrangements and copy number variants may be correlated with disease and response to therapeutic intervention. In many cases, specific genes and/or critical diagnostic markers have been identified in portions of the genome that are present at abnormal copy numbers. For example, in prenatal diagnosis, extra or missing copies of whole chromosomes are the frequently occurring genetic lesions. In cancer, deletion or multiplication of copies of whole chromosomes or chromosomal segments, and higher level amplifications of specific regions of the genome, are common occurrences. Improved methods are needed for analyzing copy-number variation, including *in situ* in cell or tissue samples. The present application addresses these and other needs.

### SUMMARY

Whole-transcriptome RNA expression analysis from single-cell-based or spatial-array-based transcriptomic analyses can be used to infer large-scale CNVs. Analyzing copy number variations based on RNA expression *in situ,* however, is challenging in part due to current limitations in multiplexing RNA detection *in situ.* In some aspects, the present application addresses the need for improved methods for detecting copy number variations.

While next generation sequencing-based approaches for transcriptomic analysis used to infer copy number can detect many thousands of different genes, practical limitations on image resolution to detect different nucleic acid sequences (e.g., barcode sequences) *in situ* mean that only a few hundred different nucleic barcode sequences can be detected in the same imaging round. Additional imaging rounds can be used to detect different subsets of RNA transcripts, expanding the number of different barcodes detected by reducing optical crowding. However, this increases the time required for the assay. And with more modest plex experiments, it can be challenging to sufficiently average over many genes to achieve good representation of large scale genomic events. For instance, if 400 different barcodes are available and that is divided evenly among 400 genes, that's only about 20 genes per chromosome in humans. This may substantially limit the ability to call CNVs since there will still be substantial variability in the sum of 20 gene counts, due to changes in gene expression in those genes. Therefore, improved methods are needed for analyzing copy number variation *in situ* in a biological sample, including methods that are compatible with experiments involving a lower degree of multiplexing.

The present application addresses these and other needs in part by using a common barcode sequence (or set of barcode sequences) to encode multiple genes on the same chromosome or genomic location. The same barcode sequence or set of barcodes can encode multiple genes, averaging out the signal across multiple genes from the same region. Additionally, the use of common barcode sequences to encode genomic regions can be combined with the use of different barcodes to encode different analytes in the biological sample, allowing detection of both copy number variations and other gene expression signatures. For example, detection of gene expression can be used in applications such as cell-typing, optionally wherein the gene expression information is combined with inferred copy number variations.

In some aspects, provided herein is a method for analyzing copy number of a genomic region in a biological sample, the method comprising: (a) contacting the biological sample with a plurality of probes for detecting RNA expression from a plurality of subregions within a genomic region, wherein the biological sample is a cell or tissue sample, wherein the plurality of probes comprises at least two different probes capable of hybridizing to at least two different RNA molecules expressed from different subregions within the genomic region, wherein the plurality of probes individually comprise a common barcode sequence that identifies the genomic region; (b) detecting the common barcode sequence or a complement thereof in hybridized probes of the plurality of probes or in products thereof, wherein detecting the barcode sequence or the complement thereof provides a barcode count corresponding to the genomic region for a cell in the biological sample; and (c) analyzing the copy number of the genomic region based on the barcode count corresponding to the genomic region for the cell in the biological sample.

In some aspects, provided herein is a method for analyzing copy number of a genomic region in a biological sample, the method comprising contacting the biological sample with a plurality of probes for detecting RNA expression from a plurality of subregions within a genomic region, wherein the biological sample is a cell or tissue sample, wherein the plurality of probes comprises at least two different probes capable of hybridizing to at least two different RNA molecules expressed from different subregions within the genomic region, wherein the plurality of probes individually comprise a common barcode sequence that identifies the genomic region; and detecting the common barcode sequence or a complement thereof in hybridized probes of the plurality of probes or in products thereof, wherein detecting the barcode sequence or the complement thereof provides a barcode count corresponding to the genomic region for a cell in the biological sample. In some aspects, the copy number of the genomic region for the cell is based on the barcode count.

In any of the embodiments herein, analyzing the copy number of the genomic region comprises inferring a copy number variation for the genomic region in the cell based on the barcode count corresponding to the genomic region. In any of the embodiments herein, the cell is a cancer cell in the biological sample.

In any of the embodiments herein, the biological sample further comprises a non-cancer cell, and detecting the common barcode sequence or a complement thereof in hybridized probes of the plurality of probes or in products thereof provides a barcode count corresponding to the genomic region for the non-cancer cell in the biological sample.

In any of the embodiments herein, the biological sample comprises a non-cancer cell, and wherein detecting the common barcode sequence or a complement thereof provides a barcode count corresponding to the genomic region for the non-cancer cell in the biological sample. In some embodiments, the method comprises identifying the cancer cell and the non-cancer cell based on RNA expression of one or more cancer biomarker detected *in situ* in the biological sample. In any of the embodiments herein, analyzing the copy number of the genomic region comprises comparing the barcode count for the genomic region in the cancer cell with the barcode count for the genomic region in the non-cancer cell. In any of the embodiments herein, analyzing the copy number of the genomic region comprises comparing the barcode count for the genomic region in the cancer cell with the barcode count for the genomic region in a cell of a healthy control sample.

In any of the embodiments herein, the copy number variation is gain or loss of a chromosome. In any of the embodiments herein, the copy number variation is gain or deletion of a chromosomal segment. In any of the embodiments herein, wherein the copy number variation is gain of chromosome 7 or loss of chromosome 10.

In any of the embodiments herein, the different subregions of the contiguous genomic region is separated by at least one gene. In any of the embodiments herein, the different subregions comprises housekeeping genes within the genomic region. In any of the embodiments herein, the different subregions spans at least 1, at least 10, at least 20, or at least 50 megabases within the genomic region.

In any of the embodiments herein, the barcode count for the genomic region is at least 10, at least 20, or at least 50. In any of the embodiments herein, the barcode count for the genomic region is between 10 and 400.

In any of the embodiments herein, the plurality of probes comprises at least 5, at least 10, at least 15, or at least 20 different probes capable of hybridizing to different RNA molecules expressed from different subregions within the genomic region. In any of the embodiments herein, the individual probes of the plurality of probes further comprises a gene-specific barcode sequence. In any of the embodiments herein, the plurality of probes does not comprise a gene-specific barcode sequence.

In any of the embodiments herein, the genomic region comprises a first genomic region and the plurality of probes is a first plurality of probes, and wherein contacting the biological sample with a plurality of probes further comprises contacting the biological sample with a second plurality of probes for detecting RNA expression from a plurality of subregions within a second genomic region, wherein the second plurality of probes comprises at least two different probes capable of hybridizing to different RNA molecules expressed from different subregions within the second genomic region, and wherein the second plurality of probes individually comprises a second common barcode sequence that identifies the second genomic region. In any of the embodiments herein, the method comprises analyzing the copy number of the second genomic region based on the barcode count corresponding to the second genomic region for the cell in the biological sample. In any of the embodiments herein, the second genomic region is on a different chromosome than the first genomic region.

In some aspects, provided herein is a method for detecting a copy number variation in a cancer cell in a biological sample, the method comprising: (a) contacting the biological sample with a plurality of probes for detecting RNA expression from a plurality of subregions within a genomic region, wherein the biological sample is a cell or tissue sample comprising the cancer cell and a non-cancer cell, wherein the plurality of probes comprises at least two different probes capable of hybridizing to at least two different RNA molecules expressed from different subregions within the genomic region, wherein the plurality of probes individually comprise a common barcode sequence that identifies the genomic region; (b) detecting the common barcode sequence or a complement thereof in hybridized probes of the plurality of probes or in products thereof, wherein detecting the barcode sequence or the complement thereof provides a barcode count corresponding to the genomic region for the cancer cell and for the non-cancer cell in the biological sample; and (c) detecting the copy number variation in the cancer cell by comparing the barcode count corresponding to the genomic region for the cancer cell to the barcode count corresponding to the genomic region for the non-cancer cell.

In some aspects, provided herein is a method for detecting a copy number variation in a cancer cell in a biological sample, the method comprising: (a) contacting the biological sample with a plurality of probes for detecting RNA expression from a plurality of subregions within a genomic region, wherein the biological sample is a cell or tissue sample comprising the cancer cell and a non-cancer cell, wherein the plurality of probes comprises at least two different probes capable of hybridizing to at least two different RNA molecules expressed from different subregions within the genomic region, wherein the plurality of probes individually comprise a common barcode sequence that identifies the genomic region; and (b) detecting the common barcode sequence or a complement thereof in hybridized probes of the plurality of probes or in products thereof, wherein detecting the barcode sequence or the complement thereof provides a barcode count corresponding to the genomic region for the cancer cell and for the non-cancer cell in the biological sample. In some cases, copy number variation in the cancer cell is determined by comparing the barcode count corresponding to the genomic region for the cancer cell to the barcode count corresponding to the genomic region for the non-cancer cell.

In any of the embodiments herein, the plurality of probes comprises circular probes or circularizable probes or probe sets capable of hybridizing to each of the different RNA molecules expressed from the different subregions within the genomic region. In any of the embodiments herein, the method comprises performing rolling circle amplification to generate rolling circle amplification products of the hybridized circular probes or of circularized probes generated from the hybridized circularizable probes or probe sets in the biological sample. In any of the embodiments herein, detecting the common barcode sequence or a complement thereof comprises detecting the complement of the common barcode sequence in the rolling circle amplification products. In any of the embodiments herein, the plurality of probes comprises at least two different probe sets, wherein each probe set is capable of hybridizing to a different RNA molecule. In any of the embodiments herein, each probe set comprises at least 10, at least 20, or at least 30 probes that hybridize to tiled regions of an individual RNA molecule.

In any of the embodiments herein, the biological sample comprises a fixed and/or permeabilized biological sample. In any of the embodiments herein, the biological sample is a tissue sample. In any of the embodiments herein, the biological sample is a frozen tissue sample or a fresh tissue sample. In any of the embodiments herein, the tissue sample is a tissue slice between about 1 µm and about 50 µm in thickness, optionally wherein the tissue slice is between about 5 µm and about 35 µm in thickness. In any of the embodiments herein, the biological sample is crosslinked. In any of the embodiments, the biological sample is embedded in a hydrogel matrix. In any of the embodiments herein, the biological sample is cleared. In any of the embodiments herein, the biological sample is a biological sample that is not embedded in a hydrogel matrix.

Provided herein is a system comprising a first plurality of probes for detecting RNA expression from a plurality of subregions within a first genomic region, wherein the first plurality of probes comprises at least two different probes capable of hybridizing to at least two different RNA molecules expressed from different subregions within the first genomic region, and wherein the first plurality of probes individually comprise a first common barcode sequence that identifies the first genomic region; and a second plurality of probes for detecting RNA expression from a plurality of subregions within a second genomic region, wherein the second plurality of probes comprises at least two different probes capable of hybridizing to at least two different RNA molecules expressed from different subregions within the second genomic region, and wherein the second plurality of probes individually comprise a second common barcode sequence that identifies the second genomic region. In some instances, the first plurality of probes comprises at least 5, at least 10, at least 15, or at least 20 different probes capable of hybridizing to different RNA molecules expressed from different subregions within the genomic region. In some instances, the second plurality of probes comprises at least 5, at least 10, at least 15, or at least 20 different probes capable of hybridizing to different RNA molecules expressed from different subregions within the genomic region. In some instances, the different subregions span at least 1, at least 10, at least 20, or at least 50 megabases within the first genomic region or within the second genomic region. In some instances, individual probes of the first plurality of probes and/or the second plurality of probes further comprise a gene-specific barcode sequence. In some instances, individual probes of the first plurality of probes and/or the second plurality of probes do not comprise a gene-specific barcode sequence.

In some aspects, the first plurality of probes comprises circular probes or circularizable probes or probe sets configured to hybridize to each of the different RNA molecules expressed from the different subregions within the first genomic region. In some instances, the second plurality of probes comprises circular probes or circularizable probes or probe sets configured to hybridize to each of the different RNA molecules expressed from the different subregions within the second genomic region.

In some aspects, a system comprises a ligase for circularizing a nucleic acid molecule of the first plurality of probes or the second plurality of probes. In some aspects, a system comprises reagents for performing a ligation reaction. In some embodiments, a ligation reaction is performed to generate a plurality of circular templates for amplification. In some instances, a ligation reaction is performed to generate a plurality of circular templates from the first plurality of probes and/or the second plurality of probes. In some aspects, a system comprises a polymerase for generating a plurality of rolling circle amplification products. In some instances, the system comprises a plurality of detectably labeled probes that binds directly or indirectly to the first common barcode sequence or the second barcode sequence or a complement thereof in hybridized probes of the first plurality of probes or the second plurality of probes, or in products thereof. In some cases, the system comprises one or more reagents for performing sequencing-by-synthesis (SBS), sequencing-by-avidity (SBA) or sequencing-by-binding (SBB) to detect the first common barcode sequence and/or the second common barcode sequence.

Provided herein is a system for a computer system configured to analyze a plurality of probes for detecting RNA expression from a plurality of subregions within a genomic region detected in a biological sample, wherein the biological sample is a cell or tissue sample; and the plurality of probes individually comprise a common barcode sequence that identifies the genomic region; wherein the computer system determines a copy number of the genomic region based on a barcode count corresponding to the genomic region for a cell in the biological sample. In some instances, the computer module is used to detect the common barcode sequence or a complement thereof to detect the plurality of probes in the cell or tissue sample. In some instances, the computer system compares the determined copy number of the genomic region of the cell in the biological sample to an additional determined copy number of an additional genomic region of an additional cell. In some cases, the computer system identifies the cell in the biological sample as a cancer cell or a non-cancer cell based on the determined copy number.

### BRIEF DESCRIPTION OF THE DRAWINGS

The drawings illustrate certain features and advantages of this disclosure. These embodiments are not intended to limit the scope of the appended claims in any manner.
**FIG. 1** illustrates an example method wherein a common Barcode 1 corresponds to Genomic Region 1 and Common Barcode N corresponds to Genomic Region N.
**FIG. 2** is an example workflow of analysis of a biological sample (*e.g.,* a cell or tissue sample) using an opto-fluidic instrument, according to various embodiments.

### DETAILED DESCRIPTION

All publications, comprising patent documents, scientific articles and databases, referred to in this application are incorporated by reference in their entirety for all purposes to the same extent as if each individual publication were individually incorporated by reference. If a definition set forth herein is contrary to or otherwise inconsistent with a definition set forth in the patents, applications, published applications and other publications that are herein incorporated by reference, the definition set forth herein prevails over the definition that is incorporated herein by reference.

The section headings used herein are for organizational purposes only and are not to be construed as limiting the subject matter described.

### I. Overview

Detection of target nucleic acids *in situ* allows for detection of nucleic acid analytes (e.g., RNA transcripts) or non-nucleic acid analytes associated with target nucleic acids via binding of a labeling agent, thereby allowing spatial resolution of target analytes *in situ.* However, in addition to understanding the landscape of RNA and/or protein expression, researchers are also interested in profiling the genomic landscape of tumors as well. Among genomic variants, copy number variants (CNVs) are an important genetic driver of cancer. In some instances, CNVs are genomic events in which the number of copies of a particular gene (or genomic region) varies from individual to individual, or even from cell to cell. The present application provides a method for analyzing copy number of a genomic region in a biological sample that addresses these and other needs. In some aspects, the method provides a solution to the problem of practical limitations on RNA detection *in situ* by using a common barcode region to detect multiple different RNAs expressed from a particular genomic region, wherein the common barcode region corresponds to the particular genomic region.

When a particular target RNA is detected using a nucleic acid barcode corresponding to the particular target RNA, the "barcode count" (the number of that nucleic acid barcode detected) corresponding to the particular target RNA may not be large enough to detect a copy number variation with statistical significance. For example, if a particular target RNA would only have a barcode count of 2-5 with a normal copy number, it may be difficult to determine whether a barcode count ranging from 4-10 represents a copy number variation (e.g., duplication), or random noise within the range of barcode counts sometimes detected for the normal copy number. In some aspects, the present disclosure provides methods of addressing this problem by summing the expression of multiple different RNAs expressed from a genomic region of interest, using a common barcode sequence (a barcode sequence that is common among the multiple different RNAs expressed from the genomic region), wherein the common barcode sequence identifies the genomic region. Without being bound by theory, although detection of an individual RNA may not reliably allow detection of copy number variations, the presently provided methods are designed to provide greater reliability using the barcode counts corresponding to genomic regions rather than individual RNAs.

In some aspects, provided herein is a method comprising: (a) contacting the biological sample with a plurality of probes for detecting RNA expression from a plurality of subregions within a genomic region, wherein the biological sample is a cell or tissue sample, wherein the plurality of probes comprises at least two different probes capable of hybridizing to at least two different RNA molecules expressed from different subregions within the genomic region, wherein the plurality of probes individually comprise a common barcode sequence that identifies the genomic region; (b) detecting the common barcode sequence or a complement thereof in hybridized probes of the plurality of probes or in products thereof, wherein detecting the barcode sequence or the complement thereof provides a barcode count corresponding to the genomic region for a cell in the biological sample; and (c) analyzing the copy number of the genomic region based on the barcode count corresponding to the genomic region for the cell in the biological sample.

In some aspects, provided herein is a method comprising: (a) contacting the biological sample with a plurality of probes for detecting RNA expression from a plurality of subregions within a genomic region, wherein the biological sample is a cell or tissue sample, wherein the plurality of probes comprises at least two different probes capable of hybridizing to at least two different RNA molecules expressed from different subregions within the genomic region, wherein the plurality of probes individually comprise a common barcode sequence that identifies the genomic region; and (b) detecting the common barcode sequence or a complement thereof in hybridized probes of the plurality of probes or in products thereof, wherein detecting the barcode sequence or the complement thereof provides a barcode count corresponding to the genomic region for a cell in the biological sample. In some instances, the copy number of the genomic region is analyzed based on the barcode count corresponding to the genomic region for the cell in the biological sample.

In some aspects, the methods provided herein comprise comparing the barcode count corresponding to a genomic region for one cell (e.g., a potential or suspected cancer cell) to the barcode count corresponding to the genomic region for another cell (e.g., a healthy or control cell). In some aspects, provided herein is a method for detecting a copy number variation in a cancer cell in a biological sample, the method comprising: (a) contacting the biological sample with a plurality of probes for detecting RNA expression from a plurality of subregions within a genomic region, wherein the biological sample is a cell or tissue sample comprising the cancer cell and a non-cancer cell, wherein the plurality of probes comprises at least two different probes capable of hybridizing to at least two different RNA molecules expressed from different subregions within the genomic region, wherein the plurality of probes individually comprise a common barcode sequence that identifies the genomic region; (b) detecting the common barcode sequence or a complement thereof in hybridized probes of the plurality of probes or in products thereof, wherein detecting the barcode sequence or the complement thereof provides a barcode count corresponding to the genomic region for the cancer cell and for the non-cancer cell in the biological sample; and (c) detecting the copy number variation in the cancer cell by comparing the barcode count corresponding to the genomic region for the cancer cell to the barcode count corresponding to the genomic region for the non-cancer cell.

In some aspects, provided herein is a method for detecting a copy number variation in a cancer cell in a biological sample, the method comprising: (a) contacting the biological sample with a plurality of probes for detecting RNA expression from a plurality of subregions within a genomic region, wherein the biological sample is a cell or tissue sample comprising the cancer cell and a non-cancer cell, wherein the plurality of probes comprises at least two different probes capable of hybridizing to at least two different RNA molecules expressed from different subregions within the genomic region, wherein the plurality of probes individually comprise a common barcode sequence that identifies the genomic region; and (b) detecting the common barcode sequence or a complement thereof in hybridized probes of the plurality of probes or in products thereof, wherein detecting the barcode sequence or the complement thereof provides a barcode count corresponding to the genomic region for the cancer cell and for the non-cancer cell in the biological sample. In some embodiments, detecting the copy number variation in the cancer cell comprises comparing the barcode count corresponding to the genomic region for the cancer cell to the barcode count corresponding to the genomic region for the non-cancer cell.

In some embodiments, the methods provided herein further comprise detecting particular target nucleic acids and/or analytes in the biological sample (e.g., using fluorescent *in situ* hybridization, rolling circle amplification-based methods, and/or any other method for analyte detection at one or more locations in a biological sample).

### II. Methods for Inferring Copy Number Variations (CNV) in situ

In order to infer copy number variations with increasing accuracy, it is important to remove the confounding effect of natural fluctuations in gene expression levels, so that the excess or relative gene expression can be directly attributed to copy number variation. In some cases in the context of *in situ* expression, gene expression levels are detected as nucleic acid barcode counts (e.g., detected spots) corresponding to the RNA of the expressed gene. In some aspects, the detected nucleic acid barcode counts are detected within a single cell. In some aspects, the detected nucleic acid barcode counts are detected at different locations within a single cell. The present disclosure involves detecting expression of genes (e.g., multiple RNAs expressed) from different sub-regions of a genomic region of interest using a common barcode sequence. In some embodiments, this approach removes the confounding effect of natural fluctuations in gene expression levels for an individual RNA.

### A. Nucleic Acid Probes

Disclosed herein in some aspects are nucleic acid probes and/or probe sets (e.g., circular probes or circularizable probes or probe sets) that are introduced into a cell or used to otherwise contact a biological sample such as a tissue sample. The probes may comprise any of a variety of entities that can hybridize to a nucleic acid, typically by Watson-Crick base pairing, such as DNA, RNA, LNA, PNA, etc. The nucleic acid probe typically contains a sequence (e.g., hybridization region such as a target recognition sequence) that can directly or indirectly bind to at least a portion of a target nucleic acid. In some embodiments, the nucleic acid probe or probe set is able to bind to a specific target nucleic acid (e.g., an mRNA, or other nucleic acids as discussed herein). In some embodiments, RCA products of the circular probes or circularized probes generated from the circularizable probes or probe sets are detected using a detectable label, and/or by using secondary nucleic acid probes able to bind to the RCA products or sequences thereof. In some embodiments, the probes or probe sets bind to mRNA. In some embodiments, the probes or probe sets bind to DNA.

In some embodiments, the probes or probe sets bind to different mRNA expressed from a genomic region, wherein the probes or probe sets comprise a common barcode sequence corresponding to the genomic region. In some embodiments, a plurality of probes of a probe set bind to different mRNAs expressed from genes located within a single genomic region, wherein the probes or probe sets comprise a common barcode sequence corresponding to the genomic region. In some embodiments, a plurality of probes of a probe set bind to different mRNAs expressed from genes located within a single genomic region, wherein the different genes are within proximity within the genomic region. In some cases, the method comprises inferring a copy number variation for the genomic region in the cell based on the barcode count corresponding to the genomic region. In some embodiments, one or more of the probes or probe sets further individually comprise target-specific barcode sequences that correspond to their respective specific target nucleic acids. In some embodiments, the probes or probe sets are designed based on sequencing results (e.g., single-cell sequencing) and/or reference data. In some embodiments, the probes or probe sets are designed to detect one or more genes associated with CNV identified from sequencing results or other reference data. In some embodiments, additional probes or probe sets are designed based on the one or more genes associated with CNV identified from sequencing results or other reference data and expanding along the length of the genomic region to identify neighboring genes to probe.

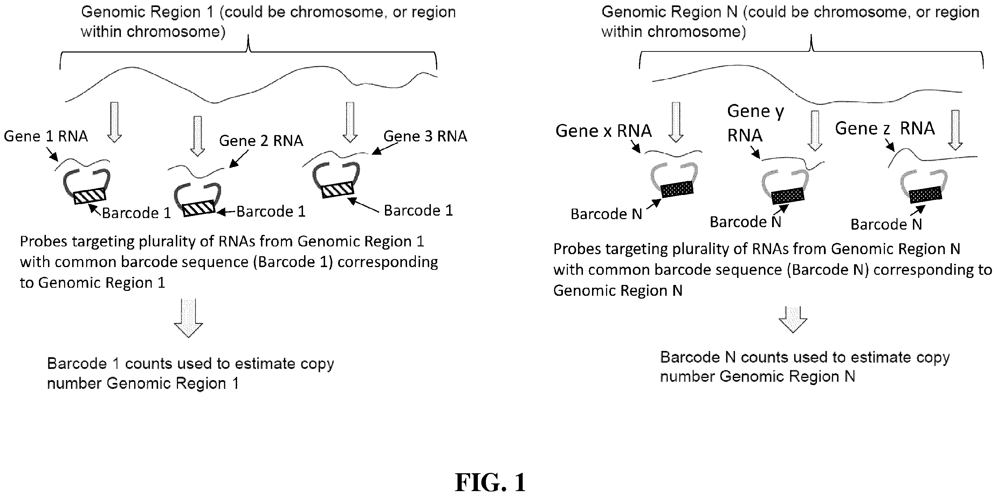

As shown in **FIG. 1****,** in some embodiments, a method provided herein comprises binding a plurality of probes comprising a common barcode sequence (Barcode 1) to a plurality of different RNAs (e.g., Gene 1 RNA, Gene 2 RNA, and Gene 3 RNA) expressed from a genomic region (Genomic Region 1). In some instances, the method comprises detecting the common barcode sequence (Barcode 1) or a complement thereof in bound probes of the plurality of probes or in products thereof, wherein detecting the barcode sequence or the complement thereof provides a barcode count corresponding to the genomic region for a cell in the biological sample; and analyzing the copy number of the genomic region (Genomic Region 1) based on the barcode count corresponding to the genomic region (Barcode 1 counts). In some cases, the probes are circularizable probes (as illustrated in **FIG. 1****),** circular probes, or circularizable probe sets (e.g., two or more nucleic acid molecules that bind directly or indirectly to their respective target sequences and are circularized upon ligation to form a circular probe from the two or more nucleic acid molecules). In some embodiments, the probes are circular probes or circularizable probes or probe sets, and the products thereof are rolling circle amplification products thereof. In some embodiments, the method comprises detecting the complement of the common barcode sequence (Barcode 1) or a complement thereof in rolling circle amplification products of the bound probes of the plurality of probes, wherein detecting the complement of the common barcode sequence (Barcode 1) provides a barcode count corresponding to the genomic region for a cell in the biological sample; and analyzing the copy number of the genomic region (Genomic Region 1) based on the barcode count corresponding to the genomic region (Barcode 1 counts).

As shown in **FIG. 1****,** in some embodiments, a method provided herein further comprises binding a plurality of probes comprising a common barcode sequence (Barcode N) to a plurality of different RNAs (e.g., Gene x RNA, Gene y RNA, and Gene z RNA) expressed from another genomic region (Genomic Region N). In some instances, the method comprises detecting the common barcode sequence (Barcode N) or a complement thereof in bound probes of the plurality of probes or in products thereof, wherein detecting the barcode sequence or the complement thereof provides a barcode count corresponding to the genomic region for a cell in the biological sample; and analyzing the copy number of the genomic region (Genomic Region N) based on the barcode count corresponding to the genomic region (Barcode N counts). In some cases, the probes are circularizable probes (as illustrated in **FIG. 1****),** circular probes, or circularizable probe sets (e.g., two or more nucleic acid molecules that bind directly or indirectly to their respective target sequences and are circularized upon ligation to form a circular probe from the two or more nucleic acid molecules). In some embodiments, the probes are circular probes or circularizable probes or probe sets, and the products thereof are rolling circle amplification products thereof. In some embodiments, the method comprises detecting the complement of the common barcode sequence (Barcode N) or a complement thereof in rolling circle amplification products of the bound probes of the plurality of probes, wherein detecting the complement of the common barcode sequence (Barcode N) provides a barcode count corresponding to the genomic region for a cell in the biological sample; and analyzing the copy number of the genomic region (Genomic Region N) based on the barcode count corresponding to the genomic region (Barcode N counts).

In some embodiments, a first plurality of probes is contacted with the biological sample, wherein the first plurality of probes comprises at least two different probes capable of hybridizing to at least two different RNA molecules expressed from different subregions within a first genomic region, and a barcode sequence corresponding to the first genomic region. In some instances, two probes of the plurality of probes are configured to bind to two different RNA molecules expressed from different subregions within a single genomic region. In some embodiments, each probe of a first plurality of probes comprise the same barcode sequence corresponding to the first genomic region. In some embodiments, two probes of the plurality of probes comprise different target recognition sequences (e.g., hybridization regions) and share the same barcode sequence in the non-target binding region. In some embodiments, the first genomic region is a chromosome. In some embodiments, the first genomic region is at least 1, at least 10, at least 20, or at least 50 contiguous megabases of a chromosome. In some embodiments, the first plurality of probes comprises at least 3, 4, 5, 10, 15, 20, 30, or more different probes capable of hybridizing to different RNA molecules expressed from different subregions within the first genomic region. In some embodiments, the first plurality of probes comprises between about 2 and about 50, between about 2 and about 30, between about 5 and about 30, between about 5 and about 25, or between about 10 and about 30 different probes capable of hybridizing to different RNA molecules expressed from different subregions within the first genomic region. In some embodiments, the different RNA molecules comprise RNA molecules expressed from two or more adjacent genes. In some embodiments, the different RNA molecules comprise RNA molecules expressed from 3, 4, 5, 10, 15, 20, 30 or more adjacent genes. In some embodiments, two or more of the different subregions are separated by at least one gene. In some embodiments, the different RNA molecules expressed from different subregions within the first genomic region comprise one or more housekeeping genes. In some embodiments, the different RNA molecules expressed from different subregions within the first genomic region comprise a plurality of homogenously expressed genes. In some embodiments, the different RNA molecules expressed from different subregions within the first genomic region comprise 2, 3, 4, 5, or more different housekeeping genes. In some embodiments, the different subregions span at least 1, at least 10, at least 20, or at least 50 megabases within the first genomic region. In some cases, the method comprises inferring a copy number variation for the first genomic region in the cell based on the barcode count corresponding to the first genomic region.

In some embodiments, a second plurality of probes is contacted with the biological sample, wherein the second plurality of probes comprises at least two different probes capable of hybridizing to at least two different RNA molecules expressed from different subregions within a second genomic region, and a barcode sequence corresponding to the second genomic region. In some embodiments, the second genomic region is a chromosome. In some embodiments, each probe of a second plurality of probes comprise the same barcode sequence corresponding to the second genomic region. In some embodiments, the second genomic region is at least 1, at least 10, at least 20, or at least 50 contiguous megabases of a chromosome. In some embodiments, the second plurality of probes comprises at least 3, 4, 5, 10, 15, 20, 30, or more different probes capable of hybridizing to different RNA molecules expressed from different subregions within the second genomic region. In some embodiments, the second plurality of probes comprises between about 2 and about 50, between about 2 and about 30, between about 5 and about 30, between about 5 and about 25, or between about 10 and about 30 different probes capable of hybridizing to different RNA molecules expressed from different subregions within the second genomic region. In some embodiments, the different RNA molecules comprise RNA molecules expressed from two or more adjacent genes. In some embodiments, the different RNA molecules comprise RNA molecules expressed from 3, 4, 5, 10, 15, 20, 30 or more adjacent genes. In some embodiments, the different RNA molecules comprise RNA molecules expressed from 5, 10, 15, 20, 30, 50, 100 or more different genes on a single chromosome. In some embodiments, two or more of the different subregions are separated by at least one gene. In some embodiments, the different RNA molecules expressed from different subregions within the second genomic region comprise one or more housekeeping genes. In some embodiments, the different RNA molecules expressed from different subregions within the second genomic region comprise 2, 3, 4, 5, or more different housekeeping genes. In some embodiments, the different subregions span at least 1, at least 10, at least 20, or at least 50 megabases within the second genomic region. In some cases, the method comprises inferring a copy number variation for the second genomic region in the cell based on the barcode count corresponding to the second genomic region.

In some embodiments, N additional pluralities of probes are contacted with the biological sample, wherein each of the N additional pluralities of probes comprises at least two different probes capable of hybridizing to at least two different RNA molecules expressed from different subregions within a particular genomic region, and a barcode sequence corresponding to the particular genomic region. In some embodiments, one or more of the particular genomic regions is/are a chromosome. In some embodiments, one or more of the particular genomic regions is/are at least 1, at least 10, at least 20, or at least 50 contiguous megabases of a chromosome. In some embodiments, one or more of the N additional pluralities of probes comprises at least 3, 4, 5, 10, 15, 20, 30, or more different probes capable of hybridizing to different RNA molecules expressed from different subregions within the corresponding genomic region. In some embodiments, one or more of the N additional pluralities of probes comprises between about 2 and about 50, between about 2 and about 30, between about 5 and about 30, between about 5 and about 25, or between about 10 and about 30 different probes capable of hybridizing to different RNA molecules expressed from different subregions within the corresponding genomic region. In some embodiments, the different RNA molecules comprise RNA molecules expressed from two or more adjacent genes. In some embodiments, the different RNA molecules comprise RNA molecules expressed from 3, 4, 5, 10, 15, 20, 30 or more adjacent genes. In some embodiments, two or more of the different subregions are separated by at least one gene. In some embodiments, the different RNA molecules expressed from different subregions within the genomic region comprise one or more housekeeping genes. In some embodiments, the different RNA molecules expressed from different subregions within the genomic region comprise a plurality of homogenously expressed genes. In some embodiments, the different RNA molecules expressed from different subregions within a given particular genomic region comprise 2, 3, 4, 5, or more different housekeeping genes. In some cases, the method comprises inferring a copy number variation for any one or more of the genomic regions in the cell based on the barcode counts corresponding to the one or more genomic regions.

In some embodiments, the different subregions span at least 1, at least 10, at least 20, or at least 50 megabases within a particular genomic region. In some embodiments, the different subregions span at least 10, at least 20, at least 50, or at least 100 megabases within a particular genomic region. In some embodiments, a genomic region is at least 1, at least 10, at least 20, at least 40, at least 60, at least 80, at least 100, at least 150, or at least 200 megabases of a chromosome. In some embodiments, a genomic region is at least 1, at least 10, at least 20, or at least 50 contiguous megabases of a chromosome. In some embodiments, a genomic region spans at least 10 contiguous megabases of a chromosome. In some embodiments, the size of the genomic region is about 10-200 megabases, 20-200 megabases, 40-200 megabases, 60-200 megabases, 80-200 megabases, 100-200 megabases, 120-200 megabases, 140-200 megabases, 160-200 megabases, 180-200 megabases, 20-200 megabases, 40-200 megabases, 60-200 megabases, 80-200 megabases, 100-200 megabases, 120-200 megabases, 140-200 megabases, 160-200 megabases, 180-200 megabases, 50-200 megabases, 100-200 megabases, 150-200 megabases, 100-200 megabases, 120-160 megabases, 180-200 megabases, 10-100 megabases, 20-100 megabases, 40-100 megabases, 60-100 megabases, 80-100 megabases, 20-100 megabases, 40-100 megabases, 60-100 megabases, 80-100 megabases, 50-100 megabases, 80-100 megabases, 50-150 megabases, 100-150 megabases, about 10-50 megabases, 20-50 megabases, 30-50 megabases, or 40-50 megabases.

In some embodiments, between about 10 and about 3000 genes, between about 10 and about 1500, between about 10 and about 1000, between about 10 and about 500, or between about 10 and about 100 different RNA molecules expressed from the same genomic region are probed. In some embodiments, between about 100 and about 3000 genes, between about 100 and about 1500, between about 100 and about 1000, between about 100 and about 500, or between about 100 and about 250 different RNA molecules expressed from the same genomic region are probed. In some embodiments, the between about 2 and about 50, between about 2 and about 30, between about 5 and about 30, between about 5 and about 25, or between about 10 and about 30 different RNA molecules expressed from the same genomic region are probed. In some embodiments, at least 10, at least 20, at least 50, at least 100, at least 200, at least 400, at least 600, at least 800, or at least 1000 different RNA molecules expressed from the same genomic region are probed.

In some embodiments, the probes or probe sets bind to different mRNA expressed from a genomic region, wherein the genomic region is an entire chromosome, and wherein the probes or probe sets comprise a common barcode sequence corresponding to the chromosome. In some embodiments, the genomic region spans at least 10%, at least 20%, at least 30%, at least 40%, at least 50%, at least 60%, at least 70%, at least 80%, or at least 90% of an entire chromosome. In some embodiments, the probes or probe sets bind to different mRNA expressed from a genomic region, wherein the genomic region is a portion of a single chromosome.

In some embodiments, the genomic region is chromosome 6, chromosome 7, chromosome 8, chromosome 11, chromosome 17, or chromosome 20. In some embodiments, the genomic region is chromosome 5. In some embodiments, the method comprises detecting deletion of chromosome 5. In some embodiments, the genomic region is chromosome 6. In some embodiments, the method comprises detecting gain of chromosome 6. In some embodiments, the genomic region is chromosome 7. In some embodiments, the method comprises detecting gain of chromosome 7. In some embodiments, the genomic region is chromosome 10. In some embodiments, the method comprises detecting loss of chromosome 10. In some instances, the loss of a portion of a chromosome is detected.

In some embodiments, the barcode count for the genomic region is between about 10 and about 400, between about 10 and about 300, between about 10 and about 100, between about 10 and about 50, between about 10 and about 30, between about 10 and about 20, between about 15 and about 300, between about 15 and about 200, between about 15 and about 80, between about 15 and about 50, or between about 20 and about 100. In some embodiments, the barcode count for the genomic region is at least any one of 10, 15, 20, 30, 40, 50, or more.

In some embodiments, the barcode count for first genomic region is between about 10 and about 400, between about 10 and about 300, between about 10 and about 100, between about 10 and about 50, between about 10 and about 30, between about 10 and about 20, between about 15 and about 300, between about 15 and about 200, between about 15 and about 80, between about 15 and about 50, or between about 20 and about 100. In some embodiments, the barcode count for first genomic region is at least any one of 10, 15, 20, 30, 40, 50, or more.

In some embodiments, the barcode count for second genomic region is between about 10 and about 400, between about 10 and about 300, between about 10 and about 100, between about 10 and about 50, between about 10 and about 30, between about 10 and about 20, between about 15 and about 300, between about 15 and about 200, between about 15 and about 80, between about 15 and about 50, or between about 20 and about 100. In some embodiments, the barcode count for second genomic region is at least any one of 10, 15, 20, 30, 40, 50, or more.

In some embodiments, the barcode count for any of the genomic regions herein is between about 10 and about 400, between about 10 and about 300, between about 10 and about 100, between about 10 and about 50, between about 10 and about 30, between about 10 and about 20, between about 15 and about 300, between about 15 and about 200, between about 15 and about 80, between about 15 and about 50, or between about 20 and about 100. In some embodiments, the barcode count for any of the genomic regions is at least any one of 10, 15, 20, 30, 40, 50, or more.

In some aspects, the method comprises comparing the barcode count for the genomic region with a reference barcode count for the genomic region. In some embodiments, the reference barcode count for the genomic region is a barcode count obtained using the same method for detection in a control sample, or a barcode count obtained using the same method for detection in a non-cancer cell in the biological sample. In some instances, a baseline is determined using the reference barcode count for a genomic region comprising a control gene. In some instances, the control gene is a housekeeping gene. In some instances, the baseline is used for comparison with a barcode count for the genomic region in a cell suspected of being associated with high CNV (e.g., a potential or suspected cancer cell).

In some instances, cancer cells and non-cancer cells are detected in the biological sample using histology and/or by detecting expression of one or more cancer biomarkers in the biological sample. In some aspects, the method comprises comparing the barcode count for the genomic region in the cancer cell with the barcode count for the genomic region in a non-cancer cell. In some embodiments, the method comprises comparing the barcode count for the genomic region in the cancer cell with the barcode count for the genomic region in a cell of a healthy control sample.

In some embodiments, the barcode count is higher than the reference barcode count (e.g., the barcode count for the genomic region in a healthy control sample or a non-cancer cell in the biological sample). In some cases, a higher barcode count compared to the reference barcode indicates a copy number variation comprising a duplication, gain, or insertion of a genomic region. In some embodiments, the genomic region is a chromosome, and the copy number variation is a gain of the chromosome. In some embodiments, the genomic region is a chromosomal segment, and the copy number variation is gain of the chromosomal segment. In some embodiments, the barcode count that is higher than the reference barcode count is at least about 1.5, 2, 2.5, 3, or more times the reference barcode count. In some embodiments, the barcode count that is higher than the reference barcode count is between about 1.5 times and about 10 times the reference barcode count.

In some embodiments, the barcode count is lower than the reference barcode count (e.g., the barcode count for the genomic region in a healthy control sample or a non-cancer cell in the biological sample). In some cases, a lower barcode count compared to the reference barcode indicates a copy number variation comprises loss and/or deletion of a genomic region. In some embodiments, the genomic region is a chromosome, and the copy number variation is loss of a chromosome. In some embodiments, the genomic region is a chromosomal segment, and the copy number variation is deletion of the chromosomal segment. In some embodiments, the barcode count that is lower than the reference barcode count is no more than ¼, ½, or ¾ of the reference barcode count. In some embodiments, the barcode count that is lower than the reference barcode count is about half of the reference barcode count.

In some embodiments, the comparing comprises dividing the barcode count for the genomic region in a cancer cell by the barcode count for the genomic region in a reference sample (e.g., non-cancer cell) to determine a fold copy number change. In some embodiments, greater than 1 fold change of the barcode count in the cancer cell relative to the non-cancer cell corresponds to copy number gain (e.g., duplication), and a less than 1 fold change of the barcode count in the cancer cell relative to the non-cancer cell corresponds to copy number loss. In some embodiments, the comparison further comprises performing a statistical calculation to determine a confidence interval.

In some embodiments, individual probes of any one of the pluralities of probes described herein do not comprise gene-specific barcode sequences. For example, in some instances, a probe does not comprise a barcode sequence that corresponds to the target RNA to which the probe binds (directly or indirectly). In some embodiments, one or more individual probes of any of the pluralities of probes described herein comprise gene-specific barcode sequences. In some embodiments, a target RNA to which the individual probe binds (directly or indirectly) is identified by detecting the gene-specific barcode sequence, or a complement thereof (e.g., wherein the individual probe is a circular or circularizable probe comprising the gene-specific barcode sequence, and wherein the method comprises detecting a rolling circle amplification product produced from the circular or circularizable probe, wherein the rolling circle amplification product comprises the complement of the gene-specific barcode sequence.

In some embodiments, the plurality of probes for detecting RNA expression from a plurality of subregions within a genomic region is a plurality of probe sets. In some cases, a probe set for a given target RNA is a set of probes comprising different target-recognition regions complementary to the target RNA, wherein each probe or a combination of the probes together provides the common barcode sequence corresponding to the genomic region. In some embodiments, two or more other RNAs expressed from different sub-regions of the genomic region are similarly targeted with probe sets, wherein each probe set is a set of probes comprising different target-recognition regions complementary to the target RNA, wherein each probe or a combination of the probes together provides the common barcode sequence corresponding to the genomic region. In some cases, the use of multiple probes tiling regions of individual target RNAs provides multiple copies of the common barcode sequence associated with each target RNA, thereby providing signal amplification during one or more detection and/or analysis steps (e.g., hybridization of detectably labeled probes to the common barcode sequence and/or sequencing of the common barcode sequence).

In some embodiments, the plurality of probes for detecting RNA expression from a plurality of subregions within a genomic region is a plurality of circular probes. In some embodiments, each of the RNAs expressed from the plurality of subregions is targeted with one or more circular probes comprising the common barcode sequence. In some embodiments, the method comprises performing rolling circle amplification to generate rolling circle amplification products (RCPs) using the circular probes bound directly or indirectly to target RNAs as templates, wherein the RCPs comprise the complement of the common barcode sequence corresponding to the genomic region. In some embodiments, the barcode count is the count of the complement of the barcode sequence detected from the RCPs. In some instances, a plurality of probes comprises at least two different that hybridize to tiled regions of an individual RNA molecule. In some instances, a plurality of probes comprises at least 10, at least 20, or at least 30 probes that hybridize to tiled regions of an individual RNA molecule. In some aspects, the tiled regions are hybridize to adjacent regions of the RNA molecules. In some aspects, the tiled regions are separated by no more than 1, no more than 2, no more, than 5, no more than 10, no more than 15, or no more than 20 nucleotides of the RNA molecule.

In some embodiments, the plurality of probes for detecting RNA expression from a plurality of subregions within a genomic region is a plurality of circularizable probes or probe sets. In some embodiments, each of the RNAs expressed from the plurality of subregions is targeted with one or more circularizable probes or probe sets comprising the common barcode sequence. In some embodiments, the method comprises ligating the circularizable probes or probe sets to generate circularized probes bound directly or indirectly to the different RNAs, and performing rolling circle amplification to generate rolling circle amplification products (RCPs) using the circularized probes bound directly or indirectly to target RNAs as templates, wherein the RCPs comprise the complement of the common barcode sequence corresponding to the genomic region. In some cases, a circularizable probe or probe set is ligated using one or more ligations as described in Section III.B.(iii). In some embodiments, the common barcode sequence comprises at least two or more common barcode sequences. In some embodiments, the common barcode sequence comprises a single barcode sequence.

In some instances, between any of the probe contacting steps disclosed herein, the method comprises one or more intervening reactions and/or processing steps, such as modifications of a target nucleic acid, modifications of a probe or product thereof (e.g., via hybridization, ligation, extension, amplification, cleavage, digestion, branch migration, primer exchange reaction, click chemistry reaction, crosslinking, attachment of a detectable label, activating photo-reactive moieties, etc.), removal of a probe or product thereof (e.g., cleaving off a portion of a probe and/or unhybridizing the entire probe), signal modifications (e.g., quenching, masking, photo-bleaching, signal enhancement (e.g., via FRET), signal amplification, etc.), signal removal (e.g., cleaving off or permanently inactivating a detectable label), crosslinking, de-crosslinking, and/or signal detection.

The target recognition sequence (e.g., hybridization region) of a probe may be positioned anywhere within the probe. In some cases, the target recognition sequence of a primary probe such as a circularizable probe that binds to a target nucleic acid is 5' or 3' to any barcode sequence in the primary probe. In some embodiments, the target recognition sequence of a secondary probe (which binds to an RCA product of a circular or circularized primary probe) is 5' or 3' to any barcode sequence in the secondary probe. In some embodiments, the target recognition sequence comprises a sequence that is substantially complementary to a portion of a target nucleic acid (a probe target sequence). In some embodiments, the target recognition sequence and the probe target sequence are at least 50%, at least 60%, at least 70%, at least 75%, at least 80%, at least 85%, at least 90%, at least 92%, at least 94%, at least 95%, at least 96%, at least 97%, at least 98%, at least 99%, or 100% complementary.

The target recognition sequence of a primary nucleic acid probe may be designed with reference to a target nucleic acid (e.g., a cellular RNA such as an mRNA) that is present or suspected of being present in a sample. In some embodiments, the target recognition sequence of a primary nucleic acid probe is designed based on single-cell sequencing data. In some embodiments, more than one target recognition sequence is used to identify a particular target RNA. In some embodiments, the more than one target-binding sequence are in the same probe or in different probes. In some embodiments, multiple probes are used, sequentially and/or simultaneously, that bind to (e.g., hybridize to) different regions of the same target RNA. In some embodiments, a single RCA product is associated with a particular target RNA (e.g., by providing a panel of circular probes or circularizable probes or probe sets, wherein each probe or probe set is designed to hybridize to a different target RNA in the biological sample).

In some embodiments, a circular probe is a probe that is pre-circularized prior to hybridization to a target RNA. In some embodiments, a circularizable probe is a probe that is circularized upon hybridization to a target RNA and/or one or more other probes such as a splint. In some embodiments, a circularizable probe set comprises at least a first nucleic acid probe and a second nucleic acid probe that is circularized upon hybridization to a target RNA and another probe such as a splint (e.g., the first and second nucleic acid probes are ligated to each other, optionally using the target RNA and a separate nucleic acid splint to form a circularized probe).

In some embodiments, the method comprises detecting the RCA product by hybridizing one or more linear probes to the RCA product. In some embodiments, a linear probe is one that comprises a target recognition sequence (e.g., a sequence complementary to a barcode sequence or subunit thereof in the RCA product) and a sequence that does not hybridize to a target nucleic acid, such as a 5' overhang, a 3' overhang, and/or a linker or spacer (which may comprise a nucleic acid sequence or a non-nucleic acid moiety). In some embodiments, the sequence (e.g., the 5' overhang, 3' overhang, and/or linker or spacer) is non-hybridizing to the target nucleic acid but may hybridize to one another and/or one or more other probes, such as detectably labeled probes. In some embodiments, a linear probe is one that comprises a target recognition sequence (e.g., a sequence complementary to a barcode sequence or subunit thereof in the RCA product) and an optically detectable label.

In any of the embodiments herein, the circularizable probe or probe set comprises one, two, three, four, or more ribonucleotides. In some embodiments, a circularizable probe or probe set disclosed herein comprises one, two, three, four, or more ribonucleotides in a DNA backbone. In any of the embodiments herein, the one or more ribonucleotides are at and/or near a ligatable 3' end of the circularizable probe or probe set. In some embodiments, a circularizable probe disclosed herein comprises one, two, three, four, or more ribonucleotides in a DNA backbone, wherein the one or more ribonucleotides are at a ligatable 3' end of the circularizable probe (e.g., a ligatable 3' end in a target recognition sequence of the circularizable probe, wherein the ligatable 3' end are ligated to a ligatable 5' end in a target recognition sequence of the circularizable probe to generate a circularized probe). In some embodiments, a 3' terminal nucleotide of the circularizable probe hybridized to the target RNA is a ribonucleotide. In some embodiments, a 3' terminal nucleotide of the circularizable probe set hybridized to the target RNA is a ribonucleotide. In some embodiments, a 3' end and a 5' end of the circularizable probe or probe set are ligated using the target RNA as a template.

In some embodiments, a probe disclosed herein (e.g., circularizable probe or probe set) comprises a 5' flap which may be recognized by a structure-specific cleavage enzyme, e.g., an enzyme capable of recognizing the junction between single-stranded 5' overhang and a DNA duplex, and cleaving the single-stranded overhang. It will be understood that the branched three-strand structure which is the substrate for the structure-specific cleavage enzyme may be formed by 5' end of one probe part and the 3' end of another probe part when both have hybridized to the target nucleic acid molecule, as well as by the 5' and 3' ends of a one-part probe. Enzymes suitable for such cleavage include Flap endonucleases (FENS), which are a class of enzymes having endonucleolytic activity and being capable of catalyzing the hydrolytic cleavage of the phosphodiester bond at the junction of single- and double-stranded DNA. Thus, in some embodiment, cleavage of the additional sequence 5' to the first target-specific binding site is performed by a structure-specific cleavage enzyme, e.g., a Flap endonuclease. Suitable Flap endonucleases are described in Ma et al. 2000. JBC 275, 24693- 24700 and in
US 2020/0224244 (herein incorporated by reference in their entireties) may include *P. furiosus* (Pfu), *A. fulgidus* (Afu), *M. jannaschii* (Mja) or *M. thermoautotrophicum* (Mth). In other embodiments, an enzyme capable of recognizing and degrading a single-stranded oligonucleotide having a free 5' end is used to cleave an additional sequence (5' flap) from a structure as described above. Thus, an enzyme having 5' nuclease activity may be used to cleave a 5' additional sequence. Such 5' nuclease activity may be 5' exonuclease and/or 5' endonuclease activity. A 5' nuclease enzyme is capable of recognizing a free 5' end of a single-stranded oligonucleotide and degrading said single-stranded oligonucleotide. A 5' exonuclease degrades a single-stranded oligonucleotide having a free 5' end by degrading the oligonucleotide into constituent mononucleotides from its 5' end. A 5' endonuclease activity may cleave the 5' flap sequence internally at one or more nucleotides. Further, a 5' nuclease activity may take place by the enzyme traversing the single-stranded oligonucleotide to a region of duplex once it has recognized the free 5' end, and cleaving the single-stranded region into larger constituent nucleotides (e.g., dinucleotides or trinucleotides), or cleaving the entire 5' single- stranded region, e.g., as described in Lyamichev et al. 1999. PNAS 96, 6143-6148 (the content of which is herein incorporated by reference in its entirety) for Taq DNA polymerase and the 5' nuclease thereof. Preferred enzymes having 5' nuclease activity include Exonuclease VIII, or a native or recombinant DNA polymerase enzyme from *Thermus aquaticus* (Taq), *Thermus thermophilus* or *Thermus flavus,* or the nuclease domain therefrom.

Any suitable circularizable probe or probe set may be used to generate the RCA template which is used to generate the RCA product. In some embodiments, a circularizable probe is in the form of a linear molecule having ligatable ends which may be circularized by ligating the ends together directly or indirectly, e.g., to each other, or to the respective ends of an intervening ("gap") oligonucleotide or to an extended 3' end of the circularizable probe. A circularizable probe may also be provided in two or more parts, namely two or more molecules (e.g., oligonucleotides) which may be ligated together to form a circle. When said RCA template is circularizable it is circularized by ligation prior to RCA. Ligation may be templated using a ligation template, and in the case of padlock and molecular inversion probes and such like the target analyte may provide the ligation template, or it may be separately provided. The circularizable RCA template (or template part or portion) will comprise at its respective 3' and 5' ends regions of complementarity to corresponding cognate complementary regions (or binding sites) in the ligation template, which may be adjacent where the ends are directly ligated to each other, or non-adjacent, with an intervening "gap" sequence, where indirect ligation is to take place.

In some embodiments (e.g., wherein the circularizable probe is a padlock probe) the ends of the circularizable probe are brought into proximity to each other by hybridization to adjacent sequences on a target nucleic acid molecule (such as a target analyte), which acts as a ligation template, thus allowing the ends to be ligated together to form a circular nucleic acid molecule, allowing the circularized circularizable probe to act as template for an RCA reaction. In such an example the terminal sequences of the circularizable probe which hybridize to the target nucleic acid molecule will be specific to the target analyte in question, and will be replicated repeatedly in the RCA product. They may therefore act as a marker sequence indicative of that target analyte. Accordingly, it can be seen that the marker sequence in the RCA product may be equivalent to a sequence present in the target analyte itself. Alternatively, a marker sequence (e.g., tag or barcode sequence) may be provided in the non-target complementary parts of the circularizable probe. In still a further embodiment, the marker sequence is present in the gap oligonucleotide which is hybridized between the respective hybridized ends of the circularizable probe, where they are hybridized to non-adjacent sequences in the target molecule. Such gap-filling padlock probes are akin to molecular inversion probes.

In some embodiments, similar circular RCA template molecules are generated using molecular inversion probes. Like padlock probes, these are also typically linear nucleic acid molecules capable of hybridizing to a target nucleic acid molecule (such as a target analyte) and being circularized. The two ends of the molecular inversion probe may hybridize to the target nucleic acid molecule at sites which are proximate but not directly adjacent to each other, resulting in a gap between the two ends. The size of this gap ranges from only a single nucleotide in some embodiments, to larger gaps of 100 to 500 nucleotides, or longer, in other embodiments. Accordingly, it is necessary to supply a polymerase and a source of nucleotides, or an additional gap-filling oligonucleotide, in order to fill the gap between the two ends of the molecular inversion probe, such that it can be circularized.

As with the circularizable probe, the terminal sequences of the molecular inversion probe which hybridize to the target nucleic acid molecule, and the sequence between them, will be specific to the target analyte in question, and will be replicated repeatedly in the RCA product. They may therefore act as a marker sequence indicative of that target analyte. Alternatively, a marker sequence (e.g., tag or barcode sequence) may be provided in the non-target complementary parts of the molecular inversion probe.

In some embodiments, the probes disclosed herein comprise invader probes, e.g., for generating a circular nucleic acid such as a circularized probe. Such probes are of particular utility in the detection of single nucleotide polymorphisms. The detection method of the present disclosure may, therefore, be used in the detection of a single nucleotide polymorphism, or indeed any variant base, in the target nucleic acid sequence. Probes for use in such a method may be designed such that the 3' ligatable end of the probe is complementary to and capable of hybridizing to the nucleotide in the target molecule which is of interest (the variant nucleotide), and the nucleotide at the 3' end of the 5' additional sequence at the 5' end of the probe or at the 5' end of another, different, probe part is complementary to the same said nucleotide, but is prevented from hybridizing thereto by a 3' ligatable end (e.g., it is a displaced nucleotide). Cleavage of the probe to remove the additional sequence provides a 5' ligatable end, which may be ligated to the 3' ligatable end of the probe or probe part if the 3' ligatable end is hybridized correctly to (e.g. is complementary to) the target nucleic acid molecule. Probes designed according to this principle provide a high degree of discrimination between different variants at the position of interest, as only probes in which the 3' ligatable end is complementary to the nucleotide at the position of interest may participate in a ligation reaction. In one embodiment, the probe is provided in a single part, and the 3' and 5' ligatable ends are provided by the same probe. In some embodiments, an invader probe is a padlock probe (an invader padlock or "iLock"), e.g., as described in Krzywkowski et al., Nucleic Acids Research 45, e161, 2017, and US 2020/0224244, which are incorporated herein by reference in their entirety.

In some embodiments, other types of probe which result in circular molecules which is detectable by RCA and which comprise either a target analyte sequence or a complement thereof include selector-type probes described in US 2019/0144940 (herein incorporated by reference in its entirety), which comprise sequences capable of directing the cleavage of a target nucleic acid molecule (e.g. a target analyte) so as to release a fragment comprising a target sequence from the target analyte and sequences capable of templating the circularization and ligation of the fragment. US 2018/0327818, the content of which is herein incorporated by reference in its entirety, describes probes which comprise a 3' sequence capable of hybridizing to a target nucleic acid molecule (e.g. a target analyte) and acting as a primer for the production of a complement of a target sequence within the target nucleic acid molecule (e.g. by target templated extension of the primer), and an internal sequence capable of templating the circularization and ligation of the extended probe comprising the reverse complement of the target sequence within the target analyte and a portion of the probe. In the case of both such probes, target sequences or complements thereof are incorporated into a circularized molecule which acts as the template for the RCA reaction to generate the RCA product, which consequently comprises concatenated repeats of said target sequence. In some embodiments, said target sequence acts as or comprises a marker sequence within the RCA product indicative of the target analyte in question. Alternatively, a marker sequence (e.g., tag or barcode sequence) may be provided in the non-target complementary parts of the probes.

In some embodiments, a nucleic acid probe disclosed herein is pre-assembled from multiple components, e.g., prior to contacting the nucleic acid probe with a target nucleic acid or a sample. In some embodiments, a nucleic acid probe disclosed herein is assembled during and/or after contacting a target nucleic acid or a sample with multiple components. In some embodiments, a nucleic acid probe disclosed herein is assembled *in situ* in a sample. In some embodiments, the multiple components is contacted with a target nucleic acid or a sample in any suitable order and any suitable combination. For instance, a first component and a second component is contacted with a target nucleic acid, to allow binding between the components and/or binding between the first and/or second components with the target nucleic acid. Optionally a reaction involving either or both components and/or the target nucleic acid, between the components, and/or between either one or both components and the target nucleic acid is performed, such as hybridization, ligation, primer extension and/or amplification, chemical or enzymatic cleavage, click chemistry, or any combination thereof. In some embodiments, a third component is added prior to, during, or after the reaction. In some embodiments, a third component is added prior to, during, or after contacting the sample with the first and/or second components. In some embodiments, the first, second, and third components are contacted with the sample in any suitable combination, sequentially or simultaneously. In some embodiments, the nucleic acid probe is assembled *in situ* in a stepwise manner, each step with the addition of one or more components, or in a dynamic process where all components are assembled together. One or more removing steps, e.g., by washing the sample such as under stringent conditions, may be performed at any point during the assembling process to remove or destabilize undesired intermediates and/or components at that point and increase the chance of accurate probe assembly and specific target binding of the assembled probe.

In some embodiments, a nucleic acid probe disclosed herein is pre-assembled from multiple components, e.g., prior to contacting the nucleic acid probe with a target nucleic acid or a sample. In some embodiments, a nucleic acid probe disclosed herein is assembled *in vitro* prior to contacting with the sample. For example, a circular probe disclosed herein is ligated and purified prior to contacting with the sample. In some embodiments, the 3' and 5' ends of a linear nucleic acid molecule are ligated to form a circular probe (e.g., using a nucleic acid splint that hybridizes to sequences at the 3' and 5' ends of a linear nucleic acid molecule). In some embodiments, a common splint is used to ligate a plurality of different linear nucleic acid molecules to generate a plurality of different circular probes for different target RNAs. In some embodiments, different linear nucleic acid molecules hybridize to a corresponding different splint for ligation. In some embodiments, the 3' and 5' ends of a linear nucleic acid molecule is ligated to form a circular probe without the use of a splint. In some embodiments, to generate a plurality of different circular probes for different target RNAs, the different circular probes are generated separately (e.g., in individual reactions) and then purified and pooled with other circular probes targeting different target RNAs to generate a pool of circular probes prior to contacting with the sample.

In some embodiments, the hybridization conditions include salt concentrations of approximately less than 1 M, e.g. less than about 500 mM and or less than about 200 mM. In some embodiments, hybridization is performed in a hybridization buffer that includes a buffered salt solution such as 5% SSPE or any other suitable buffer. Hybridization temperatures can be as low as 5°C, but are typically greater than 22°C, and more typically greater than about 30°C, and typically in excess of 37°C. Hybridizations are often performed under stringent conditions, e.g., conditions under which a sequence will hybridize to its target sequence but will not hybridize to other, non-complementary sequences. Stringent conditions are sequence-dependent and are different in different circumstances. For example, longer fragments may require higher hybridization temperatures for specific hybridization than short fragments. As other factors may affect the stringency of hybridization, including base composition and length of the complementary strands, presence of organic solvents, and the extent of base mismatching, the combination of parameters is more important than the absolute measure of any one parameter alone. Generally stringent conditions are selected to be about 5°C lower than the T*ₘ* for the specific sequence at a defined ionic strength and pH. The melting temperature T*ₘ* can be the temperature at which a population of double-stranded nucleic acid molecules becomes half dissociated into single strands. Any suitable equation for calculating the T*ₘ* of nucleic acids can used. As indicated by standard references, a simple estimate of the T*ₘ* value may be calculated by the equation, T*ₘ* =81.5 + 0.41 (% G + C), when a nucleic acid is in aqueous solution at 1 M NaCl (see *e.g.,* Anderson and Young, Quantitative Filter Hybridization, in Nucleic Acid Hybridization (1985), the content of which is herein incorporated by reference in its entirety). Other references (e.g., Allawi and SantaLucia, Jr., Biochemistry, 36:10581-94 (1997), the content of which is herein incorporated by reference in its entirety) include alternative methods of computation which take structural and environmental, as well as sequence characteristics into account for the calculation of T*ₘ*. In general, the stability of a hybrid is a function of the ion concentration and temperature. Typically, a hybridization reaction is performed under conditions of lower stringency, followed by washes of varying, but higher, stringency.

In some instances, the circular or circularizable probe is hybridized to the target nucleic acid (e.g., target RNA) and ligated to form a circular template for RCA. In some embodiments, the ligation comprises RNA-templated ligation using the target RNA as a template. In some embodiments, the ligation involves chemical ligation. In some embodiments, the ligation involves template dependent ligation. In some embodiments, the ligation involves template independent ligation. In some embodiments, the ligation involves enzymatic ligation. In some embodiments, the enzymatic ligation involves use of a ligase. In some aspects, the ligase used herein comprises an enzyme that is commonly used to join polynucleotides together or to join the ends of a single polynucleotide. An RNA ligase, a DNA ligase, or another variety of ligase can be used to ligate two nucleotide sequences together. Ligases comprise ATP-dependent double-strand polynucleotide ligases, NAD-i-dependent double-strand DNA or RNA ligases and single-strand polynucleotide ligases, for example any of the ligases described in EC 6.5.1.1 (ATP-dependent ligases), EC 6.5.1.2 (NAD+-dependent ligases), EC 6.5.1.3 (RNA ligases). Specific examples of ligases comprise bacterial ligases such as E. coli DNA ligase, Tth DNA ligase, Thermococcus sp. (strain 9° N) DNA ligase (9°N^{™} DNA ligase, New England Biolabs), Taq DNA ligase, Ampligase^{™} (Epicentre Biotechnologies) and phage ligases such as T3 DNA ligase, T4 DNA ligase and T7 DNA ligase and mutants thereof. In some embodiments, the ligase is a T4 RNA ligase or derivative thereof. In some embodiments, the ligase is a T4 RNA ligase 2 (Rnl2) or derivative thereof. In some embodiments, the ligase is a splintR ligase. In some embodiments, the ligase is a Chlorella virus DNA Ligase (PBCV-1 DNA ligase) or derivative thereof. In some embodiments, the ligase is a single stranded DNA ligase. In some embodiments, the ligase is a T4 DNA ligase. In some embodiments, the ligase is a ligase that has an DNA-splinted DNA ligase activity. In some embodiments, the ligase is a ligase that has an RNA-splinted DNA ligase activity. In some embodiments, the ligase is selected from the group consisting of a Chlorella virus DNA ligase (PBCV DNA ligase), a T4 RNA ligase, a T4 DNA ligase, and a single-stranded DNA (ssDNA) ligase. In some embodiments, the DNA ligase is SplintR^{®} ligase (also known as Chlorella virus DNA ligase or PBCV-1 DNA ligase), T4 DNA ligase or T4 RNA ligase 2.

In some embodiments, a circular probe, circularizable probe, or circularizable probe set disclosed herein comprises a barcode sequence or complement thereof (e.g., such that the RCA product produced using the circular probe or circularized probe as a template comprises the barcode sequence). In some embodiments, a barcode includes two or more sub-barcodes that together function as a single barcode. For example, a polynucleotide barcode can include two or more polynucleotide sequences (e.g., sub-barcodes) that are separated by one or more non-barcode sequences. In some embodiments, the one or more barcode(s) also provide a platform for targeting functionalities, such as oligonucleotides, oligonucleotide-antibody conjugates, oligonucleotide-streptavidin conjugates, modified oligonucleotides, affinity purification, detectable moieties, enzymes, enzymes for detection assays or other functionalities, and/or for detection and identification of the polynucleotide. In any of the preceding embodiments, the methods provided herein include analyzing the barcodes by sequential hybridization and detection with a plurality of labelled probes (e.g., detection oligos).

In some embodiments, in a barcode sequencing method, barcode sequences are detected for identification of other molecules including nucleic acid molecules (DNA or RNA) longer than the barcode sequences themselves, as opposed to direct sequencing of the longer nucleic acid molecules. In some embodiments, a N-mer barcode sequence comprises 4^{N} complexity given a sequencing read of N bases, and a much shorter sequencing read may be required for molecular identification compared to non-barcode sequencing methods such as direct sequencing. For example, 1024 molecular species may be identified using a 5-nucleotide barcode sequence (4⁵=1024), whereas 8 nucleotide barcodes can be used to identify up to 65,536 molecular species, a number greater than the total number of distinct genes in the human genome. In some embodiments, the barcode sequences contained in the probes or RCPs are detected, rather than endogenous sequences, which can be an efficient read-out in terms of information per cycle of sequencing. Because the barcode sequences are pre-determined, they can also be designed to feature error detection and correction mechanisms, see, e.g., U.S. Pat. Pub. 20190055594 and U.S. Pat. Pub 20210164039, which are hereby incorporated by reference in their entirety.

In some embodiments, the ligation involves chemical ligation (e.g., click chemistry ligation). In some embodiments, the chemical ligation involves template dependent ligation. In some embodiments, the chemical ligation involves template independent ligation. In some embodiments, the click reaction is a template-independent reaction (*see, e.g.,* Xiong and Seela (2011), J. Org. Chem. 76(14): 5584-5597, incorporated by reference herein in its entirety). In some embodiments, the click reaction is a template-dependent reaction or template-directed reaction. In some embodiments, the template-dependent reaction is sensitive to base pair mismatches such that reaction rate is significantly higher for matched versus unmatched templates. In some embodiments, the click reaction is a nucleophilic addition template-dependent reaction. In some embodiments, the click reaction is a cyclopropane-tetrazine template-dependent reaction.

In some embodiments, the ligation involves enzymatic ligation. In some embodiments, the enzymatic ligation involves use of a ligase. In some aspects, the ligase used herein comprises an enzyme that is commonly used to join polynucleotides together or to join the ends of a single polynucleotide. An RNA ligase, a DNA ligase, or another variety of ligase can be used to ligate two nucleotide sequences together. Ligases comprise ATP-dependent double-strand polynucleotide ligases, NAD-i-dependent double-strand DNA or RNA ligases and single-strand polynucleotide ligases, for example any of the ligases described in EC 6.5.1.1 (ATP-dependent ligases), EC 6.5.1.2 (NAD+-dependent ligases), EC 6.5.1.3 (RNA ligases). Specific examples of ligases comprise bacterial ligases such as E. coli DNA ligase, Tth DNA ligase, Thermococcus sp. (strain 9° N) DNA ligase (9°N^{™} DNA ligase, New England Biolabs), Taq DNA ligase, Ampligase^{™} (Epicentre Biotechnologies) and phage ligases such as T3 DNA ligase, T4 DNA ligase and T7 DNA ligase and mutants thereof. In some embodiments, the ligase is a T4 RNA ligase. In some embodiments, the ligase is a splintR ligase. In some embodiments, the ligase is a single stranded DNA ligase. In some embodiments, the ligase is a T4 DNA ligase. In some embodiments, the ligase is a ligase that has an DNA-splinted DNA ligase activity. In some embodiments, the ligase is a ligase that has an RNA-splinted DNA ligase activity.

In some embodiments, the ligation herein is a direct ligation. In some embodiments, the ligation herein is an indirect ligation. "Direct ligation" means that the ends of the polynucleotides hybridize immediately adjacently to one another to form a substrate for a ligase enzyme resulting in their ligation to each other (intramolecular ligation). Alternatively, "indirect" means that the ends of the polynucleotides hybridize non-adjacently to one another, e.g., separated by one or more intervening nucleotides or "gaps". In some embodiments, said ends are not ligated directly to each other, but instead occurs either via the intermediacy of one or more intervening (so-called "gap" or "gap-filling" (oligo)nucleotides) or by the extension of the 3' end of a probe to "fill" the "gap" corresponding to said intervening nucleotides (intermolecular ligation). In some cases, the gap of one or more nucleotides between the hybridized ends of the polynucleotides is "filled" by one or more "gap" (oligo)nucleotide(s) which are complementary to a splint, padlock probe, or target nucleic acid. The gap may be a gap of 1 to 60 nucleotides or a gap of 1 to 40 nucleotides or a gap of 3 to 40 nucleotides. In specific embodiments, the gap is a gap of about 1, 2, 3, 4, 5, 6, 7, 8, 9 or 10 or more nucleotides, of any integer (or range of integers) of nucleotides in between the indicated values. In some embodiments, the gap between said terminal regions is filled by a gap oligonucleotide or by extending the 3' end of a polynucleotide. In some cases, ligation involves ligating the ends of the probe to at least one gap (oligo)nucleotide, such that the gap (oligo)nucleotide becomes incorporated into the resulting polynucleotide. In some embodiments, the ligation herein is preceded by gap filling. In other embodiments, the ligation herein does not require gap filling.

In some embodiments, ligation of the polynucleotides produces polynucleotides with melting temperature higher than that of unligated polynucleotides. Thus, in some aspects, ligation stabilizes the hybridization complex containing the ligated polynucleotides prior to subsequent steps, comprising amplification and detection.

In some aspects, a high fidelity ligase, such as a thermostable DNA ligase (e.g., a *Taq* DNA ligase), is used. Thermostable DNA ligases are active at elevated temperatures, allowing further discrimination by incubating the ligation at a temperature near the melting temperature (Tₘ) of the DNA strands. This selectively reduces the concentration of annealed mismatched substrates (expected to have a slightly lower Tₘ around the mismatch) over annealed fully base-paired substrates. Thus, high-fidelity ligation can be achieved through a combination of the intrinsic selectivity of the ligase active site and balanced conditions to reduce the incidence of annealed mismatched dsDNA.

In some embodiments, the ligation herein is a proximity ligation of ligating two (or more) nucleic acid sequences that are in proximity with each other, e.g., through enzymatic means (e.g., a ligase). In some embodiments, proximity ligation includes a "gap-filling" step that involves incorporation of one or more nucleic acids by a polymerase, based on the nucleic acid sequence of a template nucleic acid molecule, spanning a distance between the two nucleic acid molecules of interest (see, e.g., U.S. Patent No. 7,264,929, the entire contents of which are incorporated herein by reference). A wide variety of different methods can be used for proximity ligating nucleic acid molecules, including (but not limited to) "sticky-end" and "blunt-end" ligations. Additionally, single-stranded ligation can be used to perform proximity ligation on a single-stranded nucleic acid molecule. Sticky-end proximity ligations involve the hybridization of complementary single-stranded sequences between the two nucleic acid molecules to be joined, prior to the ligation event itself. Blunt-end proximity ligations generally do not include hybridization of complementary regions from each nucleic acid molecule because both nucleic acid molecules lack a single-stranded overhang at the site of ligation.

The target recognition sequences may be of any length, and multiple recognition sequences in the same or different circular probes or circularizable probes or probe sets may be of the same or different lengths. For instance, in some embodiments, the target recognition sequence is at least 20, at least 25, at least 30, at least 35, at least 40, or at least 50 nucleotides in length. In some embodiments, the target recognition sequence is no more than 48, no more than 45, or no more than 40 nucleotides in length. Combinations of any of these are also possible, e.g., in some embodiments, the recognition sequence has a length of between 25 and 40, between 30 and 45, or between 20 and 48 nucleotides, etc. In some embodiments, the target recognition sequence is at least 95%, at least 98%, at least 99%, or at least 100% complementary to the probe target sequence in the target RNA.

In some embodiments, the ligation herein is a direct ligation. In some embodiments, the ligation herein is an indirect ligation. "Direct ligation" means that the ends of the polynucleotides hybridize immediately adjacently to one another to form a substrate for a ligase enzyme resulting in their ligation to each other (intramolecular ligation). Alternatively, "indirect" means that the ends of the polynucleotides hybridize non-adjacently to one another, e.g., separated by one or more intervening nucleotides or "gaps". In some embodiments, said ends are not ligated directly to each other, but instead occurs either via the intermediacy of one or more intervening (so-called "gap" or "gap-filling" (oligo)nucleotides) or by the extension of the 3' end of a probe to "fill" the "gap" corresponding to said intervening nucleotides (intermolecular ligation). In some cases, the gap of one or more nucleotides between the hybridized ends of the polynucleotides is "filled" by one or more "gap" (oligo)nucleotide(s) which are complementary to a splint, a circularizable probe or probe set (e.g., padlock probe), or target nucleic acid. The gap may be a gap of 1 to 60 nucleotides or a gap of 1 to 40 nucleotides or a gap of 3 to 40 nucleotides. In specific embodiments, the gap is a gap of about 1, 2, 3, 4, 5, 6, 7, 8, 9 or 10 or more nucleotides, of any integer (or range of integers) of nucleotides in between the indicated values. In some embodiments, the gap between said terminal regions is filled by a gap oligonucleotide or by extending the 3' end of a polynucleotide. In some cases, ligation involves ligating the ends of the probe to at least one gap (oligo)nucleotide, such that the gap (oligo)nucleotide becomes incorporated into the resulting polynucleotide. In some embodiments, the ligation herein is preceded by gap filling. In other embodiments, the ligation herein does not require gap filling.

In some embodiments, ligation of the polynucleotides produces polynucleotides with melting temperature higher than that of unligated polynucleotides. Thus, in some aspects, ligation stabilizes the hybridization complex containing the ligated polynucleotides prior to subsequent steps, comprising amplification and detection.

In some aspects, a high-fidelity ligase, such as a thermostable DNA ligase (e.g., a *Taq* DNA ligase), is used, for example, for ligating two or more probes to form a circular probe disclosed herein. Thermostable DNA ligases are active at elevated temperatures, allowing further discrimination by incubating the ligation at a temperature near the melting temperature (Tₘ) of the DNA strands. This selectively reduces the concentration of annealed mismatched substrates (expected to have a slightly lower Tₘ around the mismatch) over annealed fully base-paired substrates. Thus, high-fidelity ligation can be achieved through a combination of the intrinsic selectivity of the ligase active site and balanced conditions to reduce the incidence of annealed mismatched dsDNA.

In some embodiments, a ligation herein comprises ligating two (or more) nucleic acid termini that are in proximity with each other, e.g., that are brought into proximity upon hybridization to the target RNA and/or to a separate nucleic acid molecule (e.g., a splint oligonucleotide). In some embodiments, the circularizable probe comprises a 3' end and a 5' end that are brought into proximity upon hybridization to the target RNA (e.g., as shown for the circularizable probe in **FIG. 1****).** In some embodiments, the circularizable probe is a padlock probe. In some embodiments, the 3' end and the 5' end of the circularizable probe do not hybridize to the target RNA (e.g., the target recognition sequence is in an internal region of the circularizable probe), and the 3' end and 5' end optionally hybridize to a separate nucleic acid molecule (e.g., a splint oligonucleotide) to bring the ends in proximity for ligation. In some embodiments, the ligation is with a ligase. In some embodiments, ligation includes a gap-filling step that involves incorporation of one or more nucleic acids by a polymerase, based on the nucleic acid sequence of a template nucleic acid molecule (e.g., a nucleic acid molecule such as a DNA splint).

In any of the preceding embodiments, the rolling circle amplification is performed in a buffer comprising a crowding agent. In some embodiments, the crowding agent is selected from the group consisting of poly(ethylene glycol) (PEG), glycerol, Ficoll ^{®}, and dextran sulfate. In any of the preceding embodiments, the crowding agent is poly(ethylene glycol) (PEG). In any of the preceding embodiments, the PEG is selected from the group consisting of PEG200, PEG8000, and PEG35000. In any of the preceding embodiments, the buffer comprises between about 5% and about 15% PEG, optionally wherein the buffer comprises about 10% PEG. In any of the preceding embodiments, the rolling circle amplification is performed in a buffer comprising PEG (e.g., from about PEG 2K to about PEG 16K). In some embodiments, the PEG is PEG 2K, 3K, 4K, 5K, 6K, 7K, 8K, 9K, 10K, 11K, 12K, 13K, 14K, 15K, or 16K. In some embodiments, the PEG is present at a concentration from about 2% to 25%, from about 4% to about 23%, from about 6% to about 21%, or from about 8% to about 20% (v/v). In some aspects, the crowding agent is used to stabilize the nucleic acid probes (e.g., circular or circularizable probes) and/or amplification product in a location in the biological sample.

The methods for RCA provided herein can be used to detect and/or analyze one or more target RNAs (e.g., nucleic acid analytes). Examples of nucleic acid analytes include RNA analytes such as various types of coding and non-coding RNA. Examples of the different types of RNA analytes include messenger RNA (mRNA), including a nascent RNA, a pre-mRNA, a primary-transcript RNA, and a processed RNA, such as a capped mRNA (e.g., with a 5' 7-methyl guanosine cap), a polyadenylated mRNA (poly-A tail at the 3' end), and a spliced mRNA in which one or more introns have been removed. Also included in the analytes disclosed herein are non-capped mRNA, a non-polyadenylated mRNA, and a non-spliced mRNA. The RNA analyte can be a transcript of another nucleic acid molecule (e.g., DNA or RNA such as viral RNA) present in a tissue sample. Examples of a non-coding RNAs (ncRNA) that is not translated into a protein include transfer RNAs (tRNAs) and ribosomal RNAs (rRNAs), as well as small non-coding RNAs such as microRNA (miRNA), small interfering RNA (siRNA), Piwi-interacting RNA (piRNA), small nucleolar RNA (snoRNA), small nuclear RNA (snRNA), extracellular RNA (exRNA), small Cajal body-specific RNAs (scaRNAs), and the long ncRNAs such as Xist and HOTAIR. The RNA can be small (e.g., less than 200 nucleic acid bases in length) or large (e.g., RNA greater than 200 nucleic acid bases in length). The RNA can be circular RNA. In some embodiments, the RNA comprises one or more secondary structures. In some embodiments, the RNA is single-stranded.

Methods and compositions disclosed herein can be used to analyze any number of target RNAs. For example, the number of target RNAs that are analyzed using the target-primed RCA methods disclosed herein can be at least about 2, at least about 3, at least about 4, at least about 5, at least about 6, at least about 7, at least about 8, at least about 9, at least about 10, at least about 11, at least about 12, at least about 13, at least about 14, at least about 15, at least about 20, at least about 25, at least about 30, at least about 40, at least about 50, at least about 100, at least about 1,000, at least about 10,000 or more different target RNAs present in a region of the biological sample.

In any embodiment described herein, the target RNA (e.g., target RNA analyte such as an mRNA) can comprise a probe target sequence for a circular or circularizable probe or probe set. In some embodiments, the probe target sequence is endogenous to the sample. In some embodiments, the probe target sequence is a single-stranded probe target sequence in the target RNA. In some embodiments, the probe target sequence uniquely identifies the target RNA among the target RNAs present in the biological sample, or among the target RNAs detectably expressed in the biological sample. In some embodiments, the probe target sequence uniquely identifies the gene encoding the target RNA among the detectably expressed genes in the biological sample. In some embodiments, a target RNA or each target RNA comprises a single probe target sequence. In some embodiments, a first target RNA comprises a first probe target sequence, a second target RNA comprises a second probe target sequence, and an Nth target RNA comprises an Nth probe target sequence, wherein the first, second, and Nth probe target sequence are different.

In some embodiments the target RNA(s) is/are attached directly or indirectly to the biological sample or to a matrix embedding the biological sample. In some embodiments, the target RNA(s) is/are crosslinked in the biological sample or in a matrix embedding the biological sample. In some embodiments, the RCP is covalently linked to the cut target RNA or a portion thereof. For example, priming of the RCA by the cut target RNA results in formation of an RCP comprising the cut target RNA or a portion thereof covalently attached to the RCP. In some embodiments, the analytes (e.g., target RNAs), probes and/or amplification products (e.g., RCPs) described herein are anchored to a polymer matrix (e.g., as described in Section III). For example, the polymer matrix is a hydrogel. In some embodiments, cross-linking of the matrix or components to be anchored to the matrix is performed chemically and/or photochemically, or alternatively by any other suitable hydrogel-formation method.

In some embodiments, performing the rolling circle amplification comprises incubating the biological sample with a polymerase for a duration of between about 10 minutes and about 4 hours, between about 10-120, 30-120, 20-90, 60-90, 30-90, 30-60, 60-120, or 60-135 minutes. In some embodiments, performing the RCA comprises incubating the biological sample at a temperature between about 20 °C and about 60 °C. In some embodiments, performing the rolling circle amplification comprises incubating the biological sample with a polymerase for about 30 minutes at about 30-40 °C (e.g., at about 37 °C). In some embodiments, performing the rolling circle amplification comprises incubating the biological sample with a polymerase for about 1 hour at about 30-40 °C (e.g., at about 37 °C). In some embodiments, performing the rolling circle amplification comprises incubating the biological sample with a polymerase for about 2 hours minutes at about 30-40 °C (e.g., at about 37 °C). In some embodiments, performing the rolling circle amplification comprises incubating the biological sample with a polymerase for about 30 minutes at about 40-50 °C (e.g., at about 45 °C). In some embodiments, performing the rolling circle amplification comprises incubating the biological sample with a polymerase for about 1 hour at about 40-50 °C (e.g., at about 45 °C).

In some embodiments, the polymerase is Phi29 DNA polymerase, Phi29-like DNA polymerase, M2 DNA polymerase, B103 DNA polymerase, GA-1 DNA polymerase, phi-PRD1 polymerase, Vent DNA polymerase, Deep Vent DNA polymerase, Vent (exo-) DNA polymerase, KlenTaq DNA polymerase, DNA polymerase I, Klenow fragment of DNA polymerase I, DNA polymerase III, T3 DNA polymerase, T4 DNA polymerase, T5 DNA polymerase, T7 DNA polymerase, Bst polymerase, rBST DNA polymerase, N29 DNA polymerase, TopoTaq DNA polymerase, T7 RNA polymerase, SP6 RNA polymerase, T3 RNA polymerase, or a variant or derivative of any of the foregoing polymerases. In some embodiments, the polymerase is a Phi29 polymerase.

In some embodiments, the RCA is synchronized by synchronizing polymerase activity. In various embodiments, the method comprises contacting the biological sample with the polymerase in a first reaction mixture that reduces or inhibits polymerase activity, and then contacting the sample with a second reaction mixture that allows polymerase activity. For example, in some instances the first reaction mixture comprises Ca²⁺. In some embodiments, the second reaction mixture comprises Mg²⁺. In some embodiments, the synchronization of polymerase activity leads to more homogeneously sized RCPs and/or brighter RCP signal spots. In some embodiments, an increase in RCP homogeneity leads to a reduction in amplification time. Overall, the synchronization of polymerase activity can improve RCP detection during *in situ* analysis of a biological sample.

### B. Cancer Biomarkers

In some embodiments, a method provided herein further comprises identifying one or more specific target nucleic acids, using any of the nucleic acid probe configurations disclosed herein comprising a target nucleic acid-specific barcode sequence or target nucleic acid-specific combination of barcode sequences. In some cases, the target nucleic acid-specific barcode sequence or combination of barcode sequences is a gene-specific barcode sequence or combination of barcode sequences. In some embodiments, the gene-specific barcode sequence or combination of barcode sequences identifies a particular mRNA in the biological sample.

In some embodiments, the method comprises identifying the cancer cell and the non-cancer cell based on RNA expression of one or more cancer biomarker detected *in situ* in the biological sample. In some cases, the RNA expression of the one or more cancer biomarker is detected using any of the nucleic acid probe configurations disclosed herein comprising a gene-specific barcode sequence or gene-specific combination of barcode sequences. In some embodiments, the method comprises detecting the RNA expression of the one or more cancer biomarker by binding one or more detectably labeled probes directly or indirectly to the RNA, to a probe that binds to the RNA, or to an amplification product of a probe that binds to the RNA. In some embodiments, the one or more detectably labeled probes bind directly or indirectly to the gene-specific barcode sequence(s) or complement(s) thereof. In some embodiments, the gene-specific barcode sequence or combination of barcode sequences is analyzed as described in Section II.C herein. In some cases, the method comprises identifying the cancer cell and the non-cancer cell based on protein expression of one or more cancer biomarker detected *in situ* in the biological sample, e.g., using any of the labeling agents described in Section III.B. and any of the detection and analysis methods described in Section II.C. In some instances, the method comprises detecting the protein expression of the one or more cancer biomarker by binding one or more detectably labeled probes directly or indirectly to a labeling agent that binds to the protein, to a probe that binds to the labeling agent, or to an amplification product of a probe that binds to the labeling agent.

Some examples of cancer biomarkers include alpha fetoprotein (AFP), CAI 5-3, CA27-29, CA19-9, CA-125, calcitonin, calretinin, carcinoembryonic antigen, CD34, CD99MIC 2, CD117, chromogranin, chromosomes 3, 7, 17, and 9p21, cytokeratin (various types: TPA, TPS, Cyfra21-1), desmin, epithelial membrane antigen (EMA), factor VIII, CD31 FL1, glial fibrillary acidic protein (GFAP), gross cystic disease fluid protein (GCDFP-15), HMB- 45, human chorionic gonadotropin (hCG), immunoglobulin, inhibin, keratin (various types), lymphocyte marker (various types, MART-1 (Melan-A), myo DI, muscle-specific actin (MSA), neurofilament, neuron-specific enolase (NSE), placental alkaline phosphatase (PLAP), prostate-specific antigen (PSA), PTPRC (CD45), SI 00 protein,smooth muscle actin (SMA), synaptophysin, thymidine kinase, thyroglobulin (Tg), thyroid transcription factor- 1 (TTF-1), tumor M2-PK, and vimentin. In some embodiments, the one or more cancer biomarkers comprise MDM2, EGFR, CCNE1, MYC, ERBB2, ZFHX3, TP53, RNF43, PTEN, PPP2R1A, PIK3R1, KRAS, KMT2D, KMT2B, CTNNB1, CTCF, IGF1R, CDKN2B, BRCA2, RB1, ATM, SMAD4, NCOR1, UTX, and/or ARID1A. In some embodiments, the one or more cancer biomarkers comprise 11q13 (CCND1), 11q14, and/or 17q12 (HER2). In some embodiments, the one or more cancer biomarkers comprise ZNF703. In some embodiments, the one or more cancer biomarkers comprise AIM1. In some embodiments, the one or more cancer biomarkers comprise PPP2R2A, MTAP and/or MAP2K4 (e.g., for detecting deletion of PPP2R2A, MTAP and/or MAP2K4). In some embodiments, the one or more cancer biomarkers comprise one or more biomarkers provided in **Table 1.**

**Table 1: Examples of cancer biomarkers**

| **Lung Cancer:** | **Breast cancer:** | **Glioblastoma:** |
|---|---|---|
| *DUOX1* | *ABCC11* | *ANXA1* |
| *EGFR* | *APOBEC3B* | *B4GALNT1* |
| *FGFBP2* | *AR* | *BCAN* |
| *GKN2* | *CAV1* | *CAV1* |
| *KDR* | *CCND1* | *CHODL* |
| *LGR5* | *CDH1* | *EGFR* |
| *MYC* | *CEACAM6* | *ELOVL2* |
| *NFKB1* | *CENPF* | *HES1* |
| *SLC2A1* | *CXCL12* | *HILPDA* |
| *SLC7A11* | *EGFR* | *IDH1* |
| *SOX2* | *ERBB2* | *IDH2* |
| *SOX9* | *ESR1* | *IGFBP3* |
| | *FOXA1* | *IGFBP5* |
| | *GATA3* | *LOX* |
| | *KIT* | *MGST1* |
| | *KRT7* | *NNAT* |
| | *LDHB* | *PSEN2* |
| | *LRRC15* | *PSENEN* |
| | *MDM2* | *SOX11* |
| | *MKI67* | *SOX2* |
| | *PCLAF* | *SOX4* |
| | *PGR* | *TP53* |
| | *PTGDS* | *TRIL* |
| | *RUNX1* | *TTYH1* |
| | *S100A4* | |
| | *SCD* | |
| | *SFRP1* | |
| | *SNAI1* | |
| | *TACSTD2* | |
| | *TCF4* | |
| | *TOP2A* | |
| | *ZEB1* | |
| | *ZEB2* | |

### C. Detection and Analysis

In some aspects, the provided methods involve analyzing, e.g., detecting or determining, one or more sequences present in the probes or probe sets or products thereof (e.g., rolling circle amplification products thereof). In some embodiments, the detecting is performed at one or more locations in the biological sample. In some embodiments, the detecting is performed at one or more locations within a single cell. In some embodiments, the biological sample is a cell or tissue sample. In some embodiments, the locations are the locations of RNA transcripts in the biological sample. In some embodiments, the locations are the locations at which the probes or probe sets hybridize to the RNA transcripts in the biological sample, and are optionally ligated and amplified by rolling circle amplification.

In some embodiments, detecting the one or more sequences present in the probes or probe sets in the biological sample is performed, and the detected sequences are compared to an expected set of detected sequences. In some embodiments, the expected set of sequences is based on the barcode sequences of the panels of probes or probe sets in the probe mixture and the known expression levels of the RNA transcripts of the first, second, and/or third sets of genes in the first and second cell populations. In some embodiments, the one or more sequences are one or more barcode sequences or complements thereof. In some embodiments, the expected set of detected sequences include sequences expected to be detected at a high expression level (e.g., more than 20 counts of the detected sequence per cell) in one or both of the first and second cell populations. In some embodiments, the expected set of detected sequences include sequences expected to be detected at a medium expression level (e.g., 5-20 counts of the detected sequence per cell) in one or both of the first and second cell populations. In some embodiments, the expected set of detected sequences include sequences expected to be detected at a low expression level (e.g., 1-5 counts of the detected sequence per cell) in one or both of the first and second cell populations.

In some embodiments, the detecting comprises a plurality of repeated cycles of hybridization and removal of probes (e.g., detectably labeled probes, or intermediate probes that bind to detectably labeled probes) to the primary probe or probe set hybridized to the target nucleic acid, or to a rolling circle amplification product generated from the probe or probe set hybridized to the target nucleic acid.

Methods for binding and identifying a target nucleic acid that uses various probes or oligonucleotides have been described in, e.g., US2003/0013091, US2007/0166708, US2010/0015607, US2010/0261026, US2010/0262374, US2010/0112710, US2010/0047924, and US2014/0371088, each of which is incorporated herein by reference in its entirety. In some embodiments, detectably-labeled probes are used for detecting multiple target nucleic acids and be detected in one or more hybridization cycles (e.g., sequential hybridization assays, or sequencing by hybridization).

In some embodiments, the detecting comprises binding an intermediate probe directly or indirectly to the primary probe or probe set, binding a detectably labeled probe directly or indirectly to a detection region of the intermediate probe, and detecting a signal associated with the detectably labeled probe. In some embodiments, the method comprises detecting a rolling circle amplification product (RCP) generated using a circular or circularized primary probe or probe set as a template. In some embodiments, the method comprises detecting a rolling circle amplification product (RCP) generated using a circular or circularized probe or probe that binds to a primary probe or probe set as a template. In some embodiments, detecting the RCP comprises binding an intermediate probe directly or indirectly to the RCP, binding a detectably labeled probe directly or indirectly to a detection region of the intermediate probe, and detecting a signal associated with the detectably labeled probe. In some embodiments, the method comprises performing one or more wash steps to remove unbound and/or nonspecifically bound intermediate probe molecules from the primary probes or the products of the primary probes.

In some embodiments, the detecting comprises: detecting signals associated with detectably labeled probes that are hybridized to barcode regions or complements thereof in the primary probe or probe set or a product thereof (e.g., an RCP); and/or detecting signals associated with detectably labeled probes that are hybridized to intermediate probes which are in turn hybridized to the barcode regions or complements thereof. In some embodiments, the detectably labeled probes are fluorescently labeled.

In some embodiments, the methods comprise detecting the sequence in all or a portion of a primary probe or probe set or an RCP, or detecting a sequence of the primary probe or probe set or RCP, such as one or more barcode sequences present in the primary probe or probe set or RCP. In some embodiments, the sequence of the RCP, or barcode thereof, is indicative of a sequence of the target nucleic acid to which the RCP is hybridized. In some embodiments, the analysis and/or sequence determination comprises detecting a sequence in all or a portion of the nucleic acid concatemer and/or *in situ* hybridization to the RCP. In some embodiments, the detection step involves sequencing by hybridization, sequencing by ligation, sequencing by synthesis, sequencing by binding, and/or fluorescent *in situ* sequencing (FISSEQ), and/or hybridization-based *in situ* sequencing. In some embodiments, the detection step is by sequential fluorescent *in situ* hybridization (e.g., for combinatorial decoding of the barcode sequence or complement thereof).

In some embodiments, the detection or determination comprises hybridizing to the a probe directly or indirectly a detection oligonucleotide labeled with a fluorophore, an isotope, a mass tag, or a combination thereof. In some embodiments, the detection or determination comprises imaging the probe hybridized to the target nucleic acid (e.g., imaging one or more detectably labeled probes hybridized thereto). In some embodiments, the target nucleic acid is an mRNA in a tissue sample, and the detection or determination is performed when the target nucleic acid and/or the amplification product is *in situ* in the tissue sample. In some embodiments, the target nucleic acid is an amplification product (e.g., a rolling circle amplification product).

In some instances, the disclosed methods may comprise the use of a branched DNA (bDNA) amplification approach to amplify signals. In branched DNA (bDNA) amplification, primary and secondary amplifier oligonucleotides, each containing multiple replicate binding sites, are assembled on, e.g., individual smFISH probes to form a branched structure which binds multiple copies of a fluorescently labeled probe (Xia, *et al.* (2019), "Multiplexed Detection of RNA Using MERFISH and Branched DNA Amplification", *Scientific Reports* 9:7721 the content of which is herein incorporated by reference in its entirety). The degree of amplification in bDNA amplification is controlled by the design of the amplification reaction, e.g., the assembled bDNA structures cannot grow indefinitely even in the presence of excess reagents, which may be used to control spot size or limit the variability in brightness from molecule to molecule (Xia, *et al.* (2019), ibid.).

In some instances, the disclosed methods may comprise the use of a hybridization chain reaction (HCR) approach to amplify signals. In a hybridization chain reaction, two fluorescently-labeled metastable hairpin oligonucleotides self-assemble into long fluorescent polymers starting from an initiator sequence present on each probe molecule (Xia, *et al.* (2019), ibid.). The degree of amplification achieved through HCR can be tuned by changing the hybridization or polymerization times, and can be adjusted to achieve highly amplified signals (which may, however, increase the size of the fluorescent spots generated and/or lead to variable degrees of amplification for different copies of the same target molecule).

In some embodiments, provided herein are methods and compositions for analyzing analytes in a sample using concatemer primers and labeling agents. In various embodiments, a primer with domain on its 3' end binds to a catalytic hairpin, and is extended with a new domain by a strand displacing polymerase. For example, a primer with domain 1 on its 3 ends binds to a catalytic hairpin, and is extended with a new domain 1 by a strand displacing polymerase, with repeated cycles generating a concatemer of repeated domain 1 sequences. In various embodiments, the strand displacing polymerase is Bst. In various embodiments, the catalytic hairpin includes a stopper which releases the strand displacing polymerase. In various embodiments, branch migration displaces the extended primer, which can then dissociate. In various embodiments, the primer undergoes repeated cycles to form a concatemer primer.

In various embodiments, a plurality of concatemer primers is contacted with a sample. In various embodiments, an assembly include a plurality of concatemer primers, a plurality of labeled probes, and a sample including nucleic acids. In various embodiments, each the plurality of concatemer primers each includes domain 1, 2, 3, etc. In various embodiments, each the plurality of labeled probes each include domain 1', 2', 3', etc., with each corresponding domain 1', 2', 3' being complementary to domain 1, 2, 3, etc., respectively. In various embodiments, the assembly includes the plurality of concatemer primers, which are capable of hybridizing to target nucleic acid sequences in the sample. Described herein is a method using the aforementioned assembly, including contacting the sample including target nucleic acids with the plurality of concatemer primers, then contacting the sample and plurality of concatemer primers with the plurality of labeled probes, thereby labeling the target nucleic acid sequences with a plurality of labeled probes. See e.g., Kishi et al., SABER enables amplified and multiplexes imaging of RNA and DNA in cells and tissues. Nat. Methods. (2019), Saka et al., Immuno-SABER enables highly multiplexed and amplified protein imaging in tissues. Nat. Biotechnol. (2019), and U.S. Pat. No. 11,981,956, each of which is fully incorporated by reference herein.

In some aspects, the provided methods comprise imaging a detectably labeled probe bound directly or indirectly to the primary probe or probe set or product thereof and detecting the detectable label. In some embodiments, the detectably labeled probe comprises a detectable label that can be measured and quantitated. The label or detectable label can comprise a directly or indirectly detectable moiety, e.g., any fluorophores, radioactive isotopes, fluorescers, chemiluminescers, enzymes, enzyme substrates, enzyme cofactors, enzyme inhibitors, chromophores, dyes, metal ions, metal sols, ligands (e.g., biotin or haptens) and the like.

A fluorophore can comprise a substance or a portion thereof that is capable of exhibiting fluorescence in the detectable range. Particular examples of labels that may be used in accordance with the provided embodiments comprise, but are not limited to phycoerythrin, Alexa Flour^{™} dyes, fluorescein, YPet, CyPet, Cascade Blue^{®}, allophycocyanin, Cy3^{™}, Cy5^{™}, Cy7^{™}, rhodamine, dansyl, umbelliferone, Texas RedO, luminol, acradimum esters, biotin, green fluorescent protein (GFP), enhanced green fluorescent protein (EGFP), yellow fluorescent protein (YFP), enhanced yellow fluorescent protein (EYFP), blue fluorescent protein (BFP), red fluorescent protein (RFP), firefly luciferase, Renilla luciferase, NADPH, beta-galactosidase, horseradish peroxidase, glucose oxidase, alkaline phosphatase, chloramphenical acetyl transferase, and urease.

Fluorescence detection in tissue samples can often be hindered by the presence of strong background fluorescence. Background fluorescence can include autofluorescence (that can arise from a variety of sources, including aldehyde fixation, extracellular matrix components, red blood cells, lipofuscin, and the like), as opposed to the desired immunofluorescence from the fluorescently labeled antibodies or probes. Tissue autofluorescence can lead to difficulties in distinguishing the signals due to fluorescent antibodies or probes from the general background. In some embodiments, a method disclosed herein utilizes one or more agents to reduce tissue autofluorescence, for example, Autofluorescence Eliminator (Sigma/EMD Millipore), TrueBlack Lipofuscin Autofluorescence Quencher (Biotium), MaxBlock Autofluorescence Reducing Reagent Kit (MaxVision Biosciences), and/or a very intense black dye (e.g., Sudan Black, or comparable dark chromophore).

Examples of detectable labels comprise but are not limited to various radioactive moieties, enzymes, prosthetic groups, fluorescent markers, luminescent markers, bioluminescent markers, metal particles, protein-protein binding pairs and protein-antibody binding pairs. Examples of fluorescent proteins comprise, but are not limited to, yellow fluorescent protein (YFP), green fluorescence protein (GFP), cyan fluorescence protein (CFP), umbelliferone, fluorescein, fluorescein isothiocyanate, rhodamine, dichlorotriazinylamine fluorescein, dansyl chloride and phycoerythrin.

Examples of bioluminescent markers comprise, but are not limited to, luciferase (e.g., bacterial, firefly and click beetle), luciferin, aequorin and the like. Examples of enzyme systems having visually detectable signals comprise, but are not limited to, galactosidases, glucorimidases, phosphatases, peroxidases and cholinesterases. Identifiable markers also comprise radioactive compounds such as ¹²⁵I, ³⁵S, ¹⁴C, or ³H. Identifiable markers are commercially available from a variety of sources.

Examples of fluorescent labels and nucleotides and/or polynucleotides conjugated to such fluorescent labels comprise those described in, for example, Hoagland, Handbook of Fluorescent Probes and Research Chemicals, Ninth Edition (Molecular Probes, Inc., Eugene, 2002); Keller and Manak, DNA Probes, 2nd Edition (Stockton Press, New York, 1993); Eckstein, editor, Oligonucleotides and Analogues: A Practical Approach (IRL Press, Oxford, 1991); and Wetmur, Critical Reviews in Biochemistry and Molecular Biology, 26:227-259 (1991), all of which are herein incorporated by reference in their entireties. In some embodiments, exemplary techniques and methods methodologies applicable to the provided embodiments comprise those described in, for example, US 4,757,141, US 5,151,507 and US 5,091,519, all of which are herein incorporated by reference in their entireties. In some embodiments, one or more fluorescent dyes are used as labels for labeled target sequences, for example, as described in US 5,188,934 (4,7-dichlorofluorescein dyes); US 5,366,860 (spectrally resolvable rhodamine dyes); US 5,847,162 (4,7- dichlororhodamine dyes); US 4,318,846 (ether-substituted fluorescein dyes); US 5,800,996 (energy transfer dyes); US 5,066,580 (xanthine dyes); and US 5,688,648 (energy transfer dyes), all of which are herein incorporated by reference in their entireties. Labeling can also be carried out with quantum dots, as described in US 6,322,901, US 6,576,291, US 6,423,551, US 6,251,303, US 6,319,426, US 6,426,513, US 6,444,143, US 5,990,479, US 6,207,392, US 2002/0045045 and US 2003/0017264, all of which are herein incorporated by reference in their entireties. In some embodiments, a fluorescent label comprises a signaling moiety that conveys information through the fluorescent absorption and/or emission properties of one or more molecules. Exemplary fluorescent properties comprise fluorescence intensity, fluorescence lifetime, emission spectrum characteristics and energy transfer.

Examples of commercially available fluorescent nucleotide analogues readily incorporated into nucleotide and/or polynucleotide sequences comprise, but are not limited to, Cy3-dCTP, Cy3-dUTP, Cy5-dCTP, Cy5-dUTP (Amersham Biosciences, Piscataway, N.J.), fluorescein- 12-dUTP, tetramethylrhodamine-6-dUTP, TEXAS RED^{™}-5-dUTP, CASCADE BLUE^{™}-7-dUTP, BODIPY TMFL-14-dUTP, BODIPY TMR-14-dUTP, BODIPY TMTR-14-dUTP, RHOD AMINE GREEN^{™}-5-dUTP, OREGON GREENR^{™} 488-5-dUTP, TEXAS RED^{™}-l2-dUTP, BODIPY^{™} 630/650-14-dUTP, BODIPY^{™} 650/665-14-dUTP, ALEXA FLUOR^{™} 488-5-dUTP, ALEXA FLUOR^{™} 532-5-dUTP, ALEXA FLUOR^{™} 568-5-dUTP, ALEXA FLUOR^{™} 594-5-dUTP, ALEXA FLUOR^{™} 546-14-dUTP, fluorescein- 12-UTP, tetramethylrhodamine-6-UTP, TEXAS RED^{™}-5- UTP, mCherry, CASCADE BLUE^{™}-7-UTP, BODIPY^{™} FL-14-UTP, BODIPY TMR- 14-UTP, BODIPY^{™} TR-14-UTP, RHOD AMINE GREEN^{™}-5-UTP, ALEXA FLUOR^{™} 488-5-UTP, and ALEXA FLUOR^{™} 546-14-UTP (Molecular Probes, Inc. Eugene, Oreg.). Methods are known for custom synthesis of nucleotides having other fluorophores (See, Henegariu et al. (2000) Nature Biotechnol. 18:345).

Other fluorophores available for post-synthetic attachment comprise, but are not limited to, ALEXA FLUOR^{™} 350, ALEXA FLUOR^{™} 532, ALEXA FLUOR^{™} 546, ALEXA FLUOR^{™} 568, ALEXA FLUOR^{™} 594, ALEXA FLUOR^{™} 647, BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY 630/650, BODIPY 650/665, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethyl rhodamine, Texas Red (available from Molecular Probes, Inc., Eugene, Oreg.), Cy^{™}2, Cy^{™}3.5, Cy^{™}5.5, and Cy^{™}7 (Amersham Biosciences, Piscataway, N.J.). FRET tandem fluorophores may also be used, comprising, but not limited to, PerCP-Cy^{™}5.5, PE-Cy^{™}5, PE-Cy^{™}5.5, PE-Cy^{™}7, PE-Texas Red^{™}, APC-Cy^{™}7, PE-Alexa^{™} dyes (610, 647, 680), and APC-Alexa^{™} dyes.

In some cases, metallic silver or gold particles are used to enhance signal from fluorescently labeled nucleotide and/or polynucleotide sequences (Lakowicz et al. (2003) Bio Techniques 34:62, the content of which is herein incorporated by reference in its entirety).

Biotin, or a derivative thereof, may also be used as a label on a nucleotide and/or a polynucleotide sequence, and subsequently bound by a detectably labeled avidin/streptavidin derivative (e.g., phycoerythrin-conjugated streptavidin), or a detectably labeled anti-biotin antibody. Digoxigenin may be incorporated as a label and subsequently bound by a detectably labeled anti-digoxigenin antibody (e.g., fluoresceinated anti-digoxigenin). An aminoallyl-dUTP residue may be incorporated into a polynucleotide sequence and subsequently coupled to an N-hydroxy succinimide (NHS) derivatized fluorescent dye. In general, any member of a conjugate pair may be incorporated into a detection polynucleotide provided that a detectably labeled conjugate partner can be bound to permit detection.

Other suitable labels for a polynucleotide sequence may comprise fluorescein (FAM), digoxigenin, dinitrophenol (DNP), dansyl, biotin, bromodeoxyuridine (BrdU), hexahistidine (6xHis), and phosphor-amino acids (e.g., P-tyr, P-ser, P-thr). In some embodiments the following hapten/antibody pairs are used for detection, in which each of the antibodies is derivatized with a detectable label: biotin/a-biotin, digoxigenin/a- digoxigenin, dinitrophenol (DNP)/a-DNP, 5-Carboxyfluorescein (FAM)/a-FAM.

In some embodiments, a nucleotide and/or a oligonucleotide sequence is indirectly labeled, especially with a hapten that is then bound by a capture agent, e.g., as disclosed in US 5,344,757, US 5,702,888, US 5,354,657, US 5,198,537 and US 4,849,336, and US 5,073,562, all of which are herein incorporated by reference in their entireties. Many different hapten-capture agent pairs are available for use. Exemplary haptens comprise, but are not limited to, biotin, des-biotin and other derivatives, dinitrophenol, dansyl, fluorescein, Cy5^{™}, and digoxigenin. For biotin, a capture agent may be avidin, streptavidin, or antibodies. Antibodies may be used as capture agents for the other haptens (many dye-antibody pairs being commercially available, e.g., Molecular Probes, Eugene, Oreg.).

In some aspects, the detecting involves using detection methods such as flow cytometry; sequencing; probe binding and electrochemical detection; pH alteration; catalysis induced by enzymes bound to DNA tags; quantum entanglement; Raman spectroscopy; terahertz wave technology; and/or scanning electron microscopy. In some aspects, the flow cytometry is mass cytometry or fluorescence-activated flow cytometry. In some aspects, the detecting comprises performing microscopy, scanning mass spectrometry or other imaging techniques described herein. In such aspects, the detecting comprises determining a signal, e.g., a fluorescent signal.

In some aspects, the detection (comprising imaging) is carried out using any of a number of different types of microscopy, e.g., confocal microscopy, two-photon microscopy, light-field microscopy, intact tissue expansion microscopy, and/or CLARITY^{™}-optimized light sheet microscopy (COLM).

In some embodiments, fluorescence microscopy is used for detection and imaging of the detection probe. In some aspects, a fluorescence microscope is an optical microscope that uses fluorescence and phosphorescence instead of, or in addition to, reflection and absorption to study properties of organic or inorganic substances. In fluorescence microscopy, a sample is illuminated with light of a wavelength which excites fluorescence in the sample. The fluoresced light, which is usually at a longer wavelength than the illumination, is then imaged through a microscope objective. Two filters may be used in this technique; an illumination (or excitation) filter which ensures the illumination is near monochromatic and at the correct wavelength, and a second emission (or barrier) filter which ensures none of the excitation light source reaches the detector. Alternatively, these functions may both be accomplished by a single dichroic filter. The fluorescence microscope can be any microscope that uses fluorescence to generate an image, whether it is a more simple set up like an epifluorescence microscope, or a more complicated design such as a confocal microscope, which uses optical sectioning to get better resolution of the fluorescent image.

In some embodiments, confocal microscopy is used for detection and imaging of the detection probe. Confocal microscopy uses point illumination and a pinhole in an optically conjugate plane in front of the detector to eliminate out-of-focus signal. As only light produced by fluorescence very close to the focal plane can be detected, the image's optical resolution, particularly in the sample depth direction, is much better than that of wide-field microscopes. However, as much of the light from sample fluorescence is blocked at the pinhole, this increased resolution is at the cost of decreased signal intensity - so long exposures are often required. As only one point in the sample is illuminated at a time, 2D or 3D imaging requires scanning over a regular raster (e.g., a rectangular pattern of parallel scanning lines) in the specimen. The achievable thickness of the focal plane is defined mostly by the wavelength of the used light divided by the numerical aperture of the objective lens, but also by the optical properties of the specimen. The thin optical sectioning possible makes these types of microscopes particularly good at 3D imaging and surface profiling of samples. CLARITY^{™}-optimized light sheet microscopy (COLM) provides an alternative microscopy for fast 3D imaging of large clarified samples. COLM interrogates large immunostained tissues, permits increased speed of acquisition and results in a higher quality of generated data.

Other types of microscopy that can be employed comprise bright field microscopy, oblique illumination microscopy, dark field microscopy, phase contrast, differential interference contrast (DIC) microscopy, interference reflection microscopy (also known as reflected interference contrast, or RIC), single plane illumination microscopy (SPIM), super-resolution microscopy, laser microscopy, electron microscopy (EM), Transmission electron microscopy (TEM), Scanning electron microscopy (SEM), reflection electron microscopy (REM), Scanning transmission electron microscopy (STEM) and low- voltage electron microscopy (LVEM), scanning probe microscopy (SPM), atomic force microscopy (ATM), ballistic electron emission microscopy (BEEM), chemical force microscopy (CFM), conductive atomic force microscopy (C- AFM), electrochemical scanning tunneling microscope (ECSTM), electrostatic force microscopy (EFM), fluidic force microscope (FluidFM), force modulation microscopy (FMM), feature-oriented scanning probe microscopy (FOSPM), kelvin probe force microscopy (KPFM), magnetic force microscopy (MFM), magnetic resonance force microscopy (MRFM), near-field scanning optical microscopy (NSOM) (or SNOM, scanning near-field optical microscopy, SNOM, Piezoresponse Force Microscopy (PFM), PSTM, photon scanning tunneling microscopy (PSTM), PTMS, photothermal microspectroscopy/ microscopy (PTMS), SCM, scanning capacitance microscopy (SCM), SECM, scanning electrochemical microscopy (SECM), SGM, scanning gate microscopy (SGM), SHPM, scanning Hall probe microscopy (SHPM), SICM, scanning ion-conductance microscopy (SICM), SPSM spin polarized scanning tunneling microscopy (SPSM), SSRM, scanning spreading resistance microscopy (SSRM), SThM, scanning thermal microscopy (SThM), STM, scanning tunneling microscopy (STM), STP, scanning tunneling potentiometry (STP), SVM, scanning voltage microscopy (SVM), and synchrotron x-ray scanning tunneling microscopy (SXSTM), and intact tissue expansion microscopy (exM).

In some embodiments, the assay comprises *in situ* sequencing. *In situ* sequencing typically involves incorporation of a labeled nucleotide (e.g., fluorescently labeled mononucleotides or dinucleotides) in a sequential, template-dependent manner or hybridization of a labeled primer (e.g., a labeled random hexamer) to a nucleic acid template such that the identities (e.g., nucleotide sequence) of the incorporated nucleotides or labeled primer extension products can be determined, and consequently, the nucleotide sequence of the corresponding template nucleic acid. Aspects of *in situ* sequencing are described, for example, in Mitra et al., (2003) Anal. Biochem. 320, 55-65, and Lee et al., (2014) Science, 343(6177), 1360-1363, each of which are herein incorporated by reference in their entireties. In addition, examples of methods and systems for performing *in situ* sequencing are described in US 2016/0024555, US 2019/0194709, and in US 10,138,509, US 10,494,662 and US 10,179,932, all of which are herein incorporated by reference in their entireties. Exemplary techniques for in situ sequencing or in in situ sequence detection comprise, but are not limited to, STARmap (described for example in Wang et al., (2018) Science, 361(6499) 5691, the content of which is herein incorporated by reference in its entirety), MERFISH (described for example in Moffitt, (2016) Methods in Enzymology, 572, 1-49, the content of which is herein incorporated by reference in its entirety), hybridization-based *in situ* sequencing (HybISS) (described for example in Gyllborg et al., Nucleic Acids Res (2020) 48(19):e112, the content of which is herein incorporated by reference in its entirety), and FISSEQ (described for example in US 2019/0032121, the content is herein incorporated by reference in its entirety).

In some embodiments, analyzing, e.g., detecting or determining, one or more sequences present in the biological sample is performed using a base-by-base sequencing method, e.g., sequencing-by-synthesis (SBS), sequencing-by-avidity (SBA) or sequencing-by-binding (SBB). In some embodiments, the biological sample is contacted with a sequencing primer and base-by-base sequencing using a cyclic series of nucleotide incorporation or binding, respectively, thereby generating extension products of the sequencing primer is performed followed by removing, cleaving, or blocking the extension products of the sequencing primer.

Generally in sequencing-by-synthesis methods, a first population of detectably labeled nucleotides (e.g., dNTPs) are introduced to contact a template nucleotide (e.g., a barcode sequence in the RCP) hybridized to a sequencing primer, and a first detectably labeled nucleotide (e.g., A, T, C, or G nucleotide) is incorporated by a polymerase to extend the sequencing primer in the 5' to 3' direction using a complementary nucleotide (a first nucleotide residue) in the template nucleotide as template. A signal from the first detectably labeled nucleotide can then be detected. The first population of nucleotides may be continuously introduced, but in order for a second detectably labeled nucleotide to incorporate into the extended sequencing primer, nucleotides in the first population of nucleotides that have not incorporated into a sequencing primer are generally removed (e.g., by washing), and a second population of detectably labeled nucleotides are introduced into the reaction. Then, a second detectably labeled nucleotide (e.g., A, T, C, or G nucleotide) is incorporated by the same or a different polymerase to extend the already extended sequencing primer in the 5' to 3' direction using a complementary nucleotide (a second nucleotide residue) in the template nucleotide as template. Thus, in some embodiments, cycles of introducing and removing detectably labeled nucleotides are performed.

In some embodiments, the base-by-base sequencing comprises using a polymerase that is fluorescently labeled. In some embodiments, the base-by-base sequencing comprises using a polymerase-nucleotide conjugate comprising a fluorescently labeled polymerase linked to a nucleotide moiety that is not fluorescently labeled. In some embodiments, the base-by-base sequencing comprises using a multivalent polymer-nucleotide conjugate comprising a polymer core, multiple nucleotide moieties, and one or more fluorescent labels.

In some embodiments, sequencing is performed by sequencing-by-synthesis (SBS). In some embodiments, a sequencing primer is complementary to sequences at or near the one or more barcode(s). In such embodiments, sequencing-by-synthesis comprises reverse transcription and/or amplification in order to generate a template sequence from which a primer sequence can bind. Example SBS methods comprise those described for example, but not limited to, US 2007/0166705, US 2006/0188901, US 7,057,026, US 2006/0240439, US 2006/0281109, US 2011/0059865, US 2005/0100900, US 9,217,178, US 2009/0118128, US 2012/0270305, US 2013/0260372, and US 2013/0079232, all of which are herein incorporated by reference in their entireties.

In some embodiments, sequencing is performed by sequencing-by-binding (SBB). Various aspects of SBB are described in U.S. Pat. No. 10,655,176 B2, the content of which is herein incorporated by reference in its entirety. In some embodiments, SBB comprises performing repetitive cycles of detecting a stabilized complex that forms at each position along the template nucleic acid to be sequenced (e.g. a ternary complex that includes the primed template nucleic acid, a polymerase, and a cognate nucleotide for the position), under conditions that prevent covalent incorporation of the cognate nucleotide into the primer, and then extending the primer to allow detection of the next position along the template nucleic acid. In the sequencing-by-binding approach, detection of the nucleotide at each position of the template occurs prior to extension of the primer to the next position. Generally, the methodology is used to distinguish the four different nucleotide types that can be present at positions along a nucleic acid template by uniquely labelling each type of ternary complex (e.g., different types of ternary complexes differing in the type of nucleotide it contains) or by separately delivering the reagents needed to form each type of ternary complex. In some instances, the labeling may comprise fluorescence labeling of, e.g., the cognate nucleotide or the polymerase that participate in the ternary complex.

In some embodiments, sequencing is performed by sequencing-by-avidity (SBA). Some aspects of SBA approaches are described in U.S. Pat. No. 10,768,173 B2, the content of which is herein incorporated by reference in its entirety. In some embodiments, SBA comprises detecting a multivalent binding complex formed between a fluorescently-labeled polymer-nucleotide conjugate, and a one or more primed target nucleic acid sequences (e.g., barcode sequences). Fluorescence imaging is used to detect the bound complex and thereby determine the identity of the N+1 nucleotide in the target nucleic acid sequence (where the primer extension strand is N nucleotides in length). Following the imaging step, the multivalent binding complex is disrupted and washed away, the correct blocked nucleotide is incorporated into the primer extension strand, and the sequencing cycle is repeated.

In some embodiments, detection of the barcode sequences is performed by sequential hybridization of probes to the barcode sequences or complements thereof and detecting complexes formed by the probes and barcode sequences or complements thereof. In some cases, each barcode sequence or complement thereof is assigned a sequence of signal codes that identifies the barcode sequence or complement thereof (e.g., a temporal signal signature or code that identifies the analyte), and detecting the barcode sequences or complements thereof comprises decoding the barcode sequences of complements thereof by detecting the corresponding sequences of signal codes detected from sequential hybridization, detection, and removal of sequential pools of intermediate probes and the universal pool of detectably labeled probes. In some cases, the sequences of signal codes are fluorophore sequences assigned to the corresponding barcode sequences or complements thereof. In some embodiments, the detectably labeled probes are fluorescently labeled. In some embodiments, the barcode sequence or complement thereof is performed by sequential probe hybridization as described in US 2021/0340618, the content of which is herein incorporated by reference in its entirety.

In any of the embodiments herein, the detecting step comprises contacting the biological sample with one or more detectably labeled probes that directly or indirectly hybridize to the barcode sequences or complements thereof (e.g., in amplification products generated using the probes or probe sets), and dehybridizing the one or more detectably labeled probes. In any of the embodiments herein, the contacting and dehybridizing steps are repeated with the one or more detectably labeled probes and/or one or more other detectably labeled probes that directly or indirectly hybridize to the barcode sequences or complements thereof. In some aspects, the method comprises sequential hybridization of detectably labeled probes to create a spatiotemporal signal signature or code that identifies the analyte.

In any of the embodiments herein, the detecting step comprises contacting the biological sample with one or more first detectably labeled probes that directly hybridize to the plurality of probes or probe sets. In some instances, the detecting step comprises contacting the biological sample with one or more first detectably labeled probes that indirectly hybridize to the plurality of probes or probe sets. In any of the embodiments herein, the detecting step comprises contacting the biological sample with one or more first detectably labeled probes that directly or indirectly hybridize to the plurality of probes or probe sets.

In any of the embodiments herein, the detecting step comprises contacting the biological sample with one or more intermediate probes that directly or indirectly hybridize to the barcode sequences or complements thereof (e.g., of the plurality of probes or probe sets or rolling circle amplification product generated using the plurality of probes or probe sets), wherein the one or more intermediate probes are detectable using one or more detectably labeled probes. In any of the embodiments herein, the detecting step further comprises dehybridizing the one or more intermediate probes and/or the one or more detectably labeled probes from the barcode sequences or complements thereof (e.g., of the plurality of probes or probe sets or rolling circle amplification product generated using the plurality of probes or probe sets). In any of the embodiments herein, the contacting and dehybridizing steps is repeated with the one or more intermediate probes, the one or more detectably labeled probes, one or more other intermediate probes, and/or one or more other detectably labeled probes. In some cases, the repeated contacting, detection and dehybridizing steps allows detection of barcode sequences or complements thereof and identification of the corresponding sequences of signal codes (e.g., fluorophore sequences assigned to the corresponding barcode sequences or complements thereof).

In some embodiments, sequencing is performed using single molecule sequencing by ligation. Such techniques utilize DNA ligase to incorporate oligonucleotides and identify the incorporation of such oligonucleotides. The oligonucleotides typically have different labels that are correlated with the identity of a particular nucleotide in a sequence to which the oligonucleotides hybridize. Aspects and features involved in sequencing by ligation are described, for example, in Shendure et al. Science (2005), 309: 1728-1732, and in US 5,599,675; US 5,750,341; US 6,969,488; US 6,172,218; US and 6,306,597, all of which are herein incorporated by reference in their entireties.

In some embodiments, nucleic acid hybridization is used for sequencing. These methods utilize labeled nucleic acid decoder probes that are complementary to at least a portion of a barcode sequence. In some embodiments, multiplex decoding is performed with pools of many different probes with distinguishable labels. Non-limiting examples of nucleic acid hybridization sequencing are described for example in US 8,460,865, and in Gunderson et al., Genome Research 14:870-877 (2004), all of which are herein incorporated by reference in their entireties.

In some embodiments, real-time monitoring of DNA polymerase activity is used during sequencing. For example, nucleotide incorporations can be detected through fluorescence resonance energy transfer (FRET), as described for example in Levene et al., Science (2003), 299, 682-686, Lundquist et al., Opt. Lett. (2008), 33, 1026-1028, and Korlach et al., Proc. Natl. Acad. Sci. USA (2008), 105, 1176-1181, all of which are herein incorporated by reference in their entireties.

In some embodiments, the analysis and/or sequence determination involves washing to remove unbound polynucleotides, thereafter revealing a fluorescent product for imaging.

### III. Samples, Analytes, and Target Sequences

### A. Samples

A sample disclosed herein can be or derived from any biological sample. In some embodiments, the biological sample for copy number variation analysis is a sample suspected of having CNV. In some embodiments, the biological sample comprises both cancer cells and non-cancer cells. In some embodiments, a test sample suspected of having CNV and a normal sample is used in the method for interring CNV (e.g., as described in Section II). In some embodiments, the sample has been subjected to RNA-sequencing (e.g., single cell RNA sequencing). Methods and compositions disclosed herein may be used for analyzing a biological sample, which may be obtained from a subject using any of a variety of techniques including, but not limited to, biopsy, surgery, and laser capture microscopy (LCM), and generally includes cells and/or other biological material from the subject. In addition to the subjects described above, in some embodiments, a biological sample is obtained from a prokaryote such as a bacterium, an archaea, a virus, or a viroid. A biological sample can also be obtained from non-mammalian organisms (e.g., a plant, an insect, an arachnid, a nematode, a fungus, or an amphibian). A biological sample can also be obtained from a eukaryote, such as a tissue sample, a patient derived organoid (PDO) or patient derived xenograft (PDX). A biological sample from an organism may comprise one or more other organisms or components therefrom. For example, a mammalian tissue section may comprise a prion, a viroid, a virus, a bacterium, a fungus, or components from other organisms, in addition to mammalian cells and non-cellular tissue components. Subjects from which biological samples can be obtained can be healthy or asymptomatic individuals, individuals that have or are suspected of having a disease (e.g., a patient with a disease such as cancer) or a pre-disposition to a disease, and/or individuals in need of therapy or suspected of needing therapy.

The biological sample can include any number of macromolecules, for example, cellular macromolecules and organelles (e.g., mitochondria and nuclei). The biological sample can include nucleic acids (such as DNA or RNA), proteins/polypeptides, carbohydrates, and/or lipids. In some embodiments, the biological sample is obtained as a tissue sample, such as a tissue section, biopsy, a core biopsy, needle aspirate, or fine needle aspirate. In some embodiments, the biological sample comprises a cell pellet or a section of a cell pellet. The sample can be a fluid sample, such as a blood sample, urine sample, or saliva sample. In some embodiments, the sample is a skin sample, a colon sample, a cheek swab, a histology sample, a histopathology sample, a plasma or serum sample, a tumor sample, living cells, cultured cells, a clinical sample such as, for example, whole blood or blood-derived products, blood cells, or cultured tissues or cells, including cell suspensions. In some embodiments, the biological sample comprises cells which are deposited on a surface.

In some embodiments, the biological samples are derived from a homogeneous culture or population of the subjects or organisms mentioned herein or alternatively from a collection of several different organisms. Biological samples can include one or more diseased cells. A diseased cell can have altered metabolic properties, gene expression, protein expression, and/or morphologic features. Examples of diseases include inflammatory disorders, metabolic disorders, nervous system disorders, and cancer. In some embodiments, the cancer cells are derived from solid tumors, hematological malignancies, cell lines, or obtained as circulating tumor cells. Biological samples can also include fetal cells and immune cells.

In some embodiments, a substrate herein comprises any support that is insoluble in aqueous liquid and which allows for positioning of biological samples, analytes, features, and/or reagents (e.g., probes) on the support. In some embodiments, a biological sample is attached to a substrate. In some embodiments, the attachment of the biological sample is irreversible or reversible, depending upon the nature of the sample and subsequent steps in the analytical method. In certain embodiments, the sample is attached to the substrate reversibly by applying a suitable polymer coating to the substrate, and contacting the sample to the polymer coating. In some embodiments, the sample is then be detached from the substrate, e.g., using an organic solvent that at least partially dissolves the polymer coating. Hydrogels are examples of polymers that are suitable for this purpose. In some embodiments, the substrate is coated or functionalized with one or more substances to facilitate attachment of the sample to the substrate. Suitable substances that can be used to coat or functionalize the substrate include, but are not limited to, lectins, poly-lysine, antibodies, and polysaccharides.

A variety of steps can be performed to prepare or process a biological sample for and/or during an assay. Except where indicated otherwise, the preparative or processing steps described below can generally be combined in any manner and in any order to appropriately prepare or process a particular sample for and/or analysis.

### (i) Preparation

In some embodiments, the biological sample is harvested from a subject (e.g., via surgical biopsy, whole subject sectioning) or grown in vitro on a growth substrate or culture dish as a population of cells, and prepared for analysis as a tissue slice or tissue section. Grown samples may be sufficiently thin for analysis without further processing steps. Alternatively, grown samples, and samples obtained via biopsy or sectioning, can be prepared as thin tissue sections using a mechanical cutting apparatus such as a vibrating blade microtome. As another alternative, in some embodiments, a thin tissue section is prepared by applying a touch imprint of a biological sample to a suitable substrate material.

In some embodiments, the thickness of the tissue section is a fraction of (e.g., less than 0.9, 0.8, 0.7, 0.6, 0.5, 0.4, 0.3, 0.2, or 0.1) the maximum cross-sectional dimension of a cell. However, tissue sections having a thickness that is larger than the maximum cross-section cell dimension can also be used. For example, cryostat sections can be used, which can be, e.g., 10-20 µm thick. More generally, the thickness of a tissue section typically depends on the method used to prepare the section and the physical characteristics of the tissue, and therefore sections having a wide variety of different thicknesses can be prepared and used. For example, the thickness of the tissue section can be at least 0.1, 0.2, 0.3, 0.4, 0.5, 0.7, 1.0, 1.5, 2, 3, 4, 5, 6, 7, 8, 9, 10, 12, 13, 14, 15, 20, 30, 40, or 50 µm. Thicker sections can also be used if desired or convenient, e.g., at least 70, 80, 90, or 100 µm or more. Typically, the thickness of a tissue section is between 1-100 µm, 1-50 µm, 1-30 µm, 1-25 µm, 1-20 µm, 1-15 µm, 1-10 µm, 2-8 µm, 3-7 µm, or 4-6 µm, but as mentioned above, sections with thicknesses larger or smaller than these ranges can also be analysed.

Multiple sections can also be obtained from a single biological sample. For example, multiple tissue sections are obtained from a surgical biopsy sample by performing serial sectioning of the biopsy sample using a sectioning blade. Spatial information among the serial sections can be preserved in this manner, and the sections can be analysed successively to obtain three-dimensional information about the biological sample.

In some embodiments, the biological sample (e.g., a tissue section as described above) is prepared by deep freezing at a temperature suitable to maintain or preserve the integrity (e.g., the physical characteristics) of the tissue structure. In some embodiments, the frozen tissue sample is sectioned, e.g., thinly sliced, onto a substrate surface using any number of suitable methods. For example, a tissue sample is prepared using a chilled microtome (e.g., a cryostat) set at a temperature suitable to maintain both the structural integrity of the tissue sample and the chemical properties of the nucleic acids in the sample. Such a temperature can be, e.g., less than -15°C, less than -20°C, or less than -25°C.

In some embodiments, the biological sample is prepared using formalin-fixation and paraffin-embedding (FFPE), which are established methods. In some embodiments, cell suspensions and other non-tissue samples are prepared using formalin-fixation and paraffin-embedding. Following fixation of the sample and embedding in a paraffin or resin block, the sample can be sectioned as described above. Prior to analysis, in some embodiments, the paraffin-embedding material is removed from the tissue section (e.g., deparaffinization) by incubating the tissue section in an appropriate solvent (e.g., xylene) followed by a rinse (e.g., 99.5% ethanol for 2 minutes, 96% ethanol for 2 minutes, and 70% ethanol for 2 minutes). In some embodiments, the biological sample (e.g., FFPE sample) is permeable after deparaffinization. In some embodiments, processing of the biological sample, such as dewaxing, allows the biological sample to become permeabilized.

As an alternative to formalin fixation described above, a biological sample can be fixed in any of a variety of other fixatives to preserve the biological structure of the sample prior to analysis. For example, a sample can be fixed via immersion in ethanol, methanol, acetone, paraformaldehyde (PFA)-Triton, and combinations thereof.

In some embodiments, the methods provided herein comprises one or more post-fixing (also referred to as postfixation) steps. In some embodiments, one or more post-fixing step is performed after contacting a sample with a polynucleotide disclosed herein, e.g., one or more probes such as a circular or padlock probe. In some embodiments, one or more post-fixing step is performed after a hybridization complex comprising a probe and a target is formed in a sample. In some embodiments, one or more post-fixing step is performed prior to a ligation reaction disclosed herein.

In some embodiments, a method disclosed herein comprises de-crosslinking the reversibly cross-linked biological sample. The de-crosslinking does not need to be complete. In some embodiments, only a portion of crosslinked molecules in the reversibly cross-linked biological sample are de-crosslinked and allowed to migrate.

In some embodiments, a biological sample is permeabilized to facilitate transfer of species (such as probes) into the sample. If a sample is not permeabilized sufficiently, the transfer of species (such as probes) into the sample may be too low to enable adequate analysis. Conversely, if the tissue sample is too permeable, the relative spatial relationship of the analytes within the tissue sample can be lost. Hence, a balance between permeabilizing the tissue sample enough to obtain good signal intensity while still maintaining the spatial resolution of the analyte distribution in the sample is desirable.

In general, a biological sample can be permeabilized by exposing the sample to one or more permeabilizing agents. Suitable agents for this purpose include, but are not limited to, organic solvents (e.g., acetone, ethanol, and methanol), cross-linking agents (e.g., paraformaldehyde), detergents (e.g., saponin, Triton X-100^{™} or Tween-20^{™}), and enzymes (e.g., trypsin, proteases). In some embodiments, the biological sample is incubated with a cellular permeabilizing agent to facilitate permeabilization of the sample. Additional methods for sample permeabilization are described, for example, in Jamur et al., Method Mol. Biol. 588:63-66, 2010, the entire contents of which are incorporated herein by reference. Any suitable method for sample permeabilization can generally be used in connection with the samples described herein.

In some embodiments, the biological sample is permeabilized by any suitable methods. For example, one or more lysis reagents can be added to the sample. Examples of suitable lysis agents include, but are not limited to, bioactive reagents such as lysis enzymes that are used for lysis of different cell types, e.g., gram positive or negative bacteria, plants, yeast, mammalian, such as lysozymes, achromopeptidase, lysostaphin, labiase, kitalase, lyticase, and a variety of other commercially available lysis enzymes. Other lysis agents can additionally or alternatively be added to the biological sample to facilitate permeabilization. For example, surfactant-based lysis solutions can be used to lyse sample cells. Lysis solutions can include ionic surfactants such as, for example, sarcosyl and sodium dodecyl sulfate (SDS). More generally, chemical lysis agents can include, without limitation, organic solvents, chelating agents, detergents, surfactants, and chaotropic agents.

Additional reagents can be added to a biological sample to perform various functions prior to analysis of the sample. In some embodiments, DNase and RNase inactivating agents or inhibitors such as proteinase K, and/or chelating agents such as EDTA, are added to the sample. For example, a method disclosed herein may comprise a step for increasing accessibility of a nucleic acid for binding, e.g., a denaturation step to open up DNA in a cell for hybridization by a probe. For example, proteinase K treatment may be used to free up DNA with proteins bound thereto.

### (ii) Embedding

In some embodiments, the biological sample is embedded in a matrix (e.g., a hydrogel matrix). Embedding the sample in this manner typically involves contacting the biological sample with a hydrogel such that the biological sample becomes surrounded by the hydrogel. For example, the sample is embedded by contacting the sample with a suitable polymer material, and activating the polymer material to form a hydrogel. In some embodiments, the hydrogel is formed such that the hydrogel is internalized within the biological sample. Biological samples can include analytes (e.g., protein, RNA, and/or DNA) embedded in a 3D matrix. In some embodiments, amplicons (e.g., rolling circle amplification products) derived from or associated with analytes (e.g., protein, RNA, and/or DNA) are embedded in a 3D matrix. In some embodiments, a 3D matrix may comprise a network of natural molecules and/or synthetic molecules that are chemically and/or enzymatically linked, e.g., by crosslinking. In some embodiments, a 3D matrix may comprise a synthetic polymer. In some embodiments, a 3D matrix comprises a hydrogel.

In some aspects, a biological sample is embedded in any of a variety of other embedding materials to provide structural substrate to the sample prior to sectioning and other handling steps. In some cases, the embedding material is removed e.g., prior to analysis of tissue sections obtained from the sample. Suitable embedding materials include, but are not limited to, waxes, resins (e.g., methacrylate resins), epoxies, and agar.

In some embodiments, the biological sample is embedded in a matrix (e.g., a hydrogel matrix). Embedding the sample in this manner typically involves contacting the biological sample with a hydrogel such that the biological sample becomes surrounded by the hydrogel. For example, the sample is embedded by contacting the sample with a suitable polymer material, and activating the polymer material to form a hydrogel. In some embodiments, the hydrogel is formed such that the hydrogel is internalized within the biological sample.

In some embodiments, the biological sample is immobilized in the hydrogel via cross-linking of the polymer material that forms the hydrogel. Cross-linking can be performed chemically and/or photochemically, or alternatively by any other suitable hydrogel-formation method.

In some embodiments, the biological sample is reversibly cross-linked prior to or during an *in situ* assay. In some aspects, the analytes, polynucleotides and/or amplification product (e.g., amplicon) of an analyte or a probe bound thereto are anchored to a polymer matrix. For example, the polymer matrix comprises a hydrogel. In some embodiments, one or more of the polynucleotide probe(s) and/or amplification product (e.g., amplicon) thereof are modified to contain functional groups that can be used as an anchoring site to attach the polynucleotide probes and/or amplification product to a polymer matrix. In some embodiments, a modified probe comprising oligo dT is used to bind to mRNA molecules of interest, followed by reversible or irreversible crosslinking of the mRNA molecules.

In some embodiments, the biological sample is immobilized in a hydrogel via cross-linking of the polymer material that forms the hydrogel. Cross-linking can be performed chemically and/or photochemically, or alternatively by any other suitable hydrogel-formation method. A hydrogel may include a macromolecular polymer gel including a network. Within the network, some polymer chains can optionally be cross-linked, although cross-linking does not always occur.

In some embodiments, a hydrogel includes hydrogel subunits, such as, but not limited to, acrylamide, bis-acrylamide, polyacrylamide and derivatives thereof, poly(ethylene glycol) and derivatives thereof (e.g. PEG-acrylate (PEG-DA), PEG-RGD), gelatin-methacryloyl (GelMA), methacrylated hyaluronic acid (MeHA), polyaliphatic polyurethanes, polyether polyurethanes, polyester polyurethanes, polyethylene copolymers, polyamides, polyvinyl alcohols, polypropylene glycol, polytetramethylene oxide, polyvinyl pyrrolidone, polyacrylamide, poly(hydroxyethyl acrylate), and poly(hydroxyethyl methacrylate), collagen, hyaluronic acid, chitosan, dextran, agarose, gelatin, alginate, protein polymers, methylcellulose, and the like, and combinations thereof.

In some embodiments, a hydrogel includes a hybrid material, e.g., the hydrogel material includes elements of both synthetic and natural polymers. Examples of suitable hydrogels are described, for example, in U.S. Patent Nos. 6,391,937, 9,512,422, and 9,889,422, and in U.S. Patent Application Publication Nos. 2017/0253918, 2018/0052081 and 2010/0055733, the entire contents of each of which are incorporated herein by reference.

The composition and application of the hydrogel-matrix to a biological sample typically depends on the nature and preparation of the biological sample (e.g., sectioned, non-sectioned, type of fixation). As one example, where the biological sample is a tissue section, the hydrogel-matrix can include a monomer solution and an ammonium persulfate (APS) initiator/tetramethylethylenediamine (TEMED) accelerator solution. As another example, where the biological sample consists of cells (e.g., cultured cells or cells disassociated from a tissue sample), the cells can be incubated with the monomer solution and APS/TEMED solutions. For cells, hydrogel-matrix gels are formed in compartments, including but not limited to devices used to culture, maintain, or transport the cells. For example, hydrogel-matrices can be formed with monomer solution plus APS/TEMED added to the compartment to a depth ranging from about 0.1 µm to about 2 mm.

Additional methods and aspects of hydrogel embedding of biological samples are described for example in Chen et al., Science 347(6221):543-548, 2015, the entire contents of which are incorporated herein by reference.

In some embodiments, the hydrogel forms the substrate. In some embodiments, the substrate includes a hydrogel and one or more second materials. In some embodiments, the hydrogel is placed on top of one or more second materials. For example, the hydrogel are pre-formed and then placed on top of, underneath, or in any other configuration with one or more second materials. In some embodiments, hydrogel formation occurs after contacting one or more second materials during formation of the substrate. Hydrogel formation can also occur within a structure (e.g., wells, ridges, projections, and/or markings) located on a substrate.

In some embodiments, hydrogel formation on a substrate occurs before, contemporaneously with, or after probes are provided to the sample. For example, hydrogel formation can be performed on the substrate already containing the probes.

In some embodiments, hydrogel formation occurs within a biological sample. In some embodiments, a biological sample (e.g., tissue section) is embedded in a hydrogel. In some embodiments, hydrogel subunits are infused into the biological sample, and polymerization of the hydrogel is initiated by an external or internal stimulus.

In embodiments in which a hydrogel is formed within a biological sample, functionalization chemistry can be used. In some embodiments, functionalization chemistry includes hydrogel-tissue chemistry (HTC). Any hydrogel-tissue backbone (e.g., synthetic or native) suitable for HTC can be used for anchoring biological macromolecules and modulating functionalization. Non-limiting examples of methods using HTC backbone variants include CLARITY, PACT, ExM, SWITCH and ePACT. In some embodiments, hydrogel formation within a biological sample is permanent. For example, biological macromolecules can permanently adhere to the hydrogel allowing multiple rounds of interrogation. In some embodiments, hydrogel formation within a biological sample is reversible. In some embodiments, HTC reagents are added to the hydrogel before, contemporaneously with, and/or after polymerization. In some embodiments, a cell labeling agent is added to the hydrogel before, contemporaneously with, and/or after polymerization. In some embodiments, a cell-penetrating agent is added to the hydrogel before, contemporaneously with, and/or after polymerization.

In some embodiments, additional reagents are added to the hydrogel subunits before, contemporaneously with, and/or after polymerization. For example, additional reagents can include but are not limited to oligonucleotides (e.g., probes), endonucleases to fragment DNA, fragmentation buffer for DNA, DNA polymerase enzymes, dNTPs used to amplify the nucleic acid and to attach the barcode to the amplified fragments. Other enzymes can be used, including without limitation, RNA polymerase, ligase, proteinase K, and DNAse. Additional reagents can also include reverse transcriptase enzymes, including enzymes with terminal transferase activity, primers, and oligonucleotides. In some embodiments, optical labels are added to the hydrogel subunits before, contemporaneously with, and/or after polymerization.

Hydrogels embedded within biological samples can be cleared using any suitable method. For example, electrophoretic tissue clearing methods can be used to remove biological macromolecules from the hydrogel-embedded sample. In some embodiments, a hydrogel-embedded sample is stored before or after clearing of hydrogel, in a medium (e.g., a mounting medium, methylcellulose, or other semi-solid mediums).

In some embodiments, a biological sample embedded in a matrix (e.g., a hydrogel) is isometrically expanded. Isometric expansion methods that can be used include hydration, a preparative step in expansion microscopy, as described in, e.g., Chen et al., Science 347(6221):543-548, 2015 and U.S. Pat. 10,059,990, which are herein incorporated by reference in their entireties. Isometric expansion of the sample can increase the spatial resolution of the subsequent analysis of the sample. The increased resolution in spatial profiling can be determined by comparison of an isometrically expanded sample with a sample that has not been isometrically expanded. In some embodiments, a biological sample is isometrically expanded to a size at least 2x, 2.1x, 2.2x, 2.3x, 2.4x, 2.5x, 2.6x, 2.7x, 2.8x, 2.9x, 3x, 3.1x, 3.2x, 3.3x, 3.4x, 3.5x, 3.6x, 3.7x, 3.8x, 3.9x, 4x, 4.1x, 4.2x, 4.3x, 4.4x, 4.5x, 4.6x, 4.7x, 4.8x, or 4.9x its non-expanded size. In some embodiments, the sample is isometrically expanded to at least 2x and less than 20x of its non-expanded size.

### (iii) Staining and Immunohistochemistry (IHC)

To facilitate visualization, biological samples can be stained using a wide variety of stains and staining techniques. In some embodiments, for example, a sample is stained using any number of stains and/or immunohistochemical reagents. In some embodiments, one or more staining steps are performed to prepare or process a biological sample for an assay described herein or are performed during and/or after an assay. In some embodiments, the sample is contacted with one or more nucleic acid stains, membrane stains (e.g., cellular or nuclear membrane), cytological stains, or combinations thereof. In some examples, the stain is specific to proteins, phospholipids, DNA (e.g., dsDNA, ssDNA), RNA, an organelle or compartment of the cell. The sample may be contacted with one or more labeled antibodies (e.g., a primary antibody specific for the analyte of interest and a labeled secondary antibody specific for the primary antibody). In some embodiments, cells in the sample are segmented using one or more images taken of the stained sample.

In some embodiments, the stain is performed using a lipophilic dye. In some examples, the staining is performed with a lipophilic carbocyanine or aminostyryl dye, or analogs thereof (e.g, DiI, DiO, DiR, DiD). Other cell membrane stains may include FM and RH dyes or immunohistochemical reagents specific for cell membrane proteins. In some examples, the stain may include but is not limited to, acridine orange, acid fuchsin, Bismarck brown, carmine, coomassie blue, cresyl violet, DAPI, eosin, ethidium bromide, acid fuchsine, haematoxylin, Hoechst stains, iodine, methyl green, methylene blue, neutral red, Nile blue, Nile red, osmium tetroxide, ruthenium red, propidium iodide, rhodamine (e.g., rhodamine B), or safranine, or derivatives thereof. In some embodiments, the sample is stained with haematoxylin and eosin (H&E).

In some embodiments, the sample is stained using hematoxylin and eosin (H&E) staining techniques, using Papanicolaou staining techniques, Masson's trichrome staining techniques, silver staining techniques, Sudan staining techniques, and/or using Periodic Acid Schiff (PAS) staining techniques. PAS staining is typically performed after formalin or acetone fixation. In some embodiments, the sample is stained using Romanowsky stain, including Wright's stain, Jenner's stain, Can-Grunwald stain, Leishman stain, and Giemsa stain.

In some embodiments, biological samples are destained. Any suitable methods of destaining or discoloring a biological sample may be utilized and generally depend on the nature of the stain(s) applied to the sample. For example, in some embodiments, one or more immunofluorescent stains are applied to the sample via antibody coupling. Such stains can be removed using techniques such as cleavage of disulfide linkages via treatment with a reducing agent and detergent washing, chaotropic salt treatment, treatment with antigen retrieval solution, and treatment with an acidic glycine buffer. Methods for multiplexed staining and destaining are described, for example, in Bolognesi et al., J. Histochem. Cytochem. 2017; 65(8): 431-444, Lin et al., Nat Commun. 2015; 6:8390, Pirici et al., J. Histochem. Cytochem. 2009; 57:567-75, and Glass et al., J. Histochem. Cytochem. 2009; 57:899-905, the entire contents of each of which are incorporated herein by reference.

### B. Analytes

A biological sample may comprise one or a plurality of analytes of interest. Methods for performing multiplexed assays to analyze two or more different analytes in a single biological sample are provided. The methods and compositions disclosed herein can be used to detect and analyze a wide variety of different analytes. In some aspects, an analyte can include any biological substance, structure, moiety, or component to be analyzed. In some aspects, a target disclosed herein may similarly include any analyte of interest. In some examples, a target or analyte is directly or indirectly detected.

In some embodiments, analytes are derived from a specific type of cell and/or a specific sub-cellular region. In some embodiments, analytes are derived from cytosol, from cell nuclei, from mitochondria, from microsomes, and more generally, from any other compartment, organelle, or portion of a cell. Permeabilizing agents that specifically target certain cell compartments and organelles can be used to selectively release analytes from cells for analysis, and/or allow access of one or more reagents (e.g., probes for analyte detection) to the analytes in the cell or cell compartment or organelle.

The analyte may include any biomolecule or chemical compound, including a macromolecule such as a protein or peptide, a lipid or a nucleic acid molecule, or a small molecule, including organic or inorganic molecules. The analyte may be a cell or a microorganism, including a virus, or a fragment or product thereof. An analyte can be any substance or entity for which a specific binding partner (e.g. an affinity binding partner) can be developed. Such a specific binding partner may be a nucleic acid probe (for a nucleic acid analyte) and may lead directly to the generation of a RCA template (e.g. a padlock or other circularizable probe). Alternatively, the specific binding partner may be coupled to a nucleic acid, which may be detected using an RCA strategy, e.g. in an assay which uses or generates a circular nucleic acid molecule which can be the RCA template.

Analytes of particular interest may include nucleic acid molecules, such as DNA (e.g. genomic DNA, mitochondrial DNA, plastid DNA, viral DNA, etc.) and RNA (e.g. mRNA, microRNA, rRNA, snRNA, viral RNA, etc.), and synthetic and/or modified nucleic acid molecules, (e.g. including nucleic acid domains comprising or consisting of synthetic or modified nucleotides such as LNA, PNA, morpholino, etc.), proteinaceous molecules such as peptides, polypeptides, proteins or prions or any molecule which includes a protein or polypeptide component, etc., or fragments thereof, or a lipid or carbohydrate molecule, or any molecule which comprise a lipid or carbohydrate component. The analyte may be a single molecule or a complex that contains two or more molecular subunits, e.g. including but not limited to protein-DNA complexes, which may or may not be covalently bound to one another, and which may be the same or different. Thus in addition to cells or microorganisms, such a complex analyte may also be a protein complex or protein interaction. Such a complex or interaction may thus be a homo- or hetero-multimer. Aggregates of molecules, e.g. proteins may also be target analytes, for example aggregates of the same protein or different proteins. The analyte may also be a complex between proteins or peptides and nucleic acid molecules such as DNA or RNA, e.g. interactions between proteins and nucleic acids, e.g. regulatory factors, such as transcription factors, and DNA or RNA.

### (i) Endogenous Analytes

In some embodiments, an analyte herein is endogenous to a biological sample and can include nucleic acid analytes and non-nucleic acid analytes. Methods and compositions disclosed herein can be used to analyze nucleic acid analytes (e.g., using a nucleic acid probe or probe set that directly or indirectly hybridizes to a nucleic acid analyte) and/or non-nucleic acid analytes (e.g., using a labeling agent that comprises a reporter oligonucleotide and binds directly or indirectly to a non-nucleic acid analyte) in any suitable combination.

Examples of non-nucleic acid analytes include, but are not limited to, lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral coat proteins, extracellular and intracellular proteins, antibodies, and antigen binding fragments. In some embodiments, the analyte is inside a cell or on a cell surface, such as a transmembrane analyte or one that is attached to the cell membrane. In some embodiments, the analyte comprises an organelle (e.g., nuclei or mitochondria). In some embodiments, the analyte is an extracellular analyte, such as a secreted analyte. Exemplary analytes include, but are not limited to, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, an extracellular matrix protein, a posttranslational modification (e.g., phosphorylation, glycosylation, ubiquitination, nitrosylation, methylation, acetylation or lipidation) state of a cell surface protein, a gap junction, and an adherens junction.

Examples of nucleic acid analytes include DNA analytes such as single-stranded DNA (ssDNA), double-stranded DNA (dsDNA), genomic DNA, methylated DNA, specific methylated DNA sequences, fragmented DNA, mitochondrial DNA, *in situ* synthesized PCR products, and RNA/DNA hybrids. In some embodiments, the DNA analyte is a transcript of another nucleic acid molecule (e.g., DNA or RNA such as mRNA) present in a tissue sample.

Examples of nucleic acid analytes also include RNA analytes such as various types of coding and non-coding RNA. Examples of the different types of RNA analytes include messenger RNA (mRNA), including a nascent RNA, a pre-mRNA, a primary-transcript RNA, and a processed RNA, such as a capped mRNA (e.g., with a 5' 7-methyl guanosine cap), a polyadenylated mRNA (poly-A tail at the 3' end), and a spliced mRNA in which one or more introns have been removed. Also included in the analytes disclosed herein are non-capped mRNA, a non-polyadenylated mRNA, and a non-spliced mRNA. The RNA analyte can be a transcript of another nucleic acid molecule (e.g., DNA or RNA such as viral RNA) present in a tissue sample. Examples of a non-coding RNAs (ncRNA) that is not translated into a protein include transfer RNAs (tRNAs) and ribosomal RNAs (rRNAs), as well as small non-coding RNAs such as microRNA (miRNA), small interfering RNA (siRNA), Piwi-interacting RNA (piRNA), small nucleolar RNA (snoRNA), small nuclear RNA (snRNA), extracellular RNA (exRNA), small Cajal body-specific RNAs (scaRNAs), and the long ncRNAs such as Xist and HOTAIR. The RNA can be small (e.g., less than 200 nucleic acid bases in length) or large (e.g., RNA greater than 200 nucleic acid bases in length). Examples of small RNAs include 5.8S ribosomal RNA (rRNA), 5S rRNA, tRNA, miRNA, siRNA, snoRNAs, piRNA, tRNA-derived small RNA (tsRNA), and small rDNA-derived RNA (srRNA). The RNA can be double-stranded RNA or single-stranded RNA. The RNA can be circular RNA. The RNA can be a bacterial rRNA (e.g., 16s rRNA or 23s rRNA).

In some embodiments described herein, an analyte is a denatured nucleic acid, wherein the resulting denatured nucleic acid is single-stranded. The nucleic acid may be denatured, for example, optionally using formamide, heat, or both formamide and heat. In some embodiments, the nucleic acid is not denatured for use in a method disclosed herein.

Methods and compositions disclosed herein can be used to analyze any number of analytes. For example, the number of analytes that are analyzed can be at least about 2, at least about 3, at least about 4, at least about 5, at least about 6, at least about 7, at least about 8, at least about 9, at least about 10, at least about 11, at least about 12, at least about 13, at least about 14, at least about 15, at least about 20, at least about 25, at least about 30, at least about 40, at least about 50, at least about 100, at least about 1,000, at least about 10,000, at least about 100,000 or more different analytes present in a region of the sample.

### (ii) Labeling Agents

In some embodiments, provided herein are methods and compositions for analyzing endogenous analytes (e.g., RNA, ssDNA, cell surface or intracellular proteins, and/or metabolites) in a sample using one or more labeling agents. In some embodiments, an analyte labeling agent includes an agent that interacts with an analyte (e.g., an endogenous analyte in a sample). In some embodiments, the labeling agents comprise a reporter oligonucleotide that is indicative of the analyte or portion thereof interacting with the labeling agent. For example, the reporter oligonucleotide may comprise a barcode sequence that permits identification of the labeling agent. In some cases, the sample contacted by the labeling agent is further contacted with a probe (e.g., a single-stranded probe sequence), that hybridizes to a reporter oligonucleotide of the labeling agent, in order to identify the analyte associated with the labeling agent. In some embodiments, the analyte labeling agent comprises an analyte binding moiety and a labeling agent barcode domain comprising one or more barcode sequences, e.g., a barcode sequence that corresponds to the analyte binding moiety and/or the analyte. An analyte binding moiety barcode includes to a barcode that is associated with or otherwise identifies the analyte binding moiety. In some embodiments, by identifying an analyte binding moiety by identifying its associated analyte binding moiety barcode, the analyte to which the analyte binding moiety binds is also identified. An analyte binding moiety barcode can be a nucleic acid sequence of a given length and/or sequence that is associated with the analyte binding moiety. An analyte binding moiety barcode can generally include any of the variety of aspects of barcodes described herein.

In some embodiments, the method comprises one or more post-fixing (also referred to as post-fixation) steps after contacting the sample with one or more labeling agents.

In the methods and systems described herein, one or more labeling agents capable of binding to or otherwise coupling to one or more features may be used to characterize analytes, cells and/or cell features. In some instances, cell features include cell surface features. Analytes may include, but are not limited to, a protein, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, a gap junction, an adherens junction, or any combination thereof. In some instances, cell features may include intracellular analytes, such as proteins, protein modifications (e.g., phosphorylation status or other post-translational modifications), nuclear proteins, nuclear membrane proteins, or any combination thereof.

In some embodiments, an analyte binding moiety includes any molecule or moiety capable of binding to an analyte (e.g., a biological analyte, e.g., a macromolecular constituent). A labeling agent may include, but is not limited to, a protein, a peptide, an antibody (or an epitope binding fragment thereof), a lipophilic moiety (such as cholesterol), a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a darpin, and a protein scaffold, or any combination thereof. The labeling agents can include (e.g., are attached to) a reporter oligonucleotide that is indicative of the cell surface feature to which the binding group binds. For example, the reporter oligonucleotide may comprise a barcode sequence that permits identification of the labeling agent. For example, a labeling agent that is specific to one type of cell feature (e.g., a first cell surface feature) may have coupled thereto a first reporter oligonucleotide, while a labeling agent that is specific to a different cell feature (e.g., a second cell surface feature) may have a different reporter oligonucleotide coupled thereto. For a description of exemplary labeling agents, reporter oligonucleotides, and methods of use, see, e.g., U.S. Pat. 10,550,429; U.S. Pat. Pub. 20190177800; and U.S. Pat. Pub. 20190367969, which are each incorporated by reference herein in their entirety.

In some embodiments, an analyte binding moiety includes one or more antibodies or epitope-binding fragments thereof. The antibodies or epitope-binding fragments including the analyte binding moiety can specifically bind to a target analyte. In some embodiments, the analyte is a protein (e.g., a protein on a surface of the biological sample (e.g., a cell) or an intracellular protein). In some embodiments, a plurality of analyte labeling agents comprising a plurality of analyte binding moieties bind a plurality of analytes present in a biological sample. In some embodiments, the plurality of analytes includes a single species of analyte (e.g., a single species of polypeptide). In some embodiments in which the plurality of analytes includes a single species of analyte, the analyte binding moieties of the plurality of analyte labeling agents are the same. In some embodiments in which the plurality of analytes includes a single species of analyte, the analyte binding moieties of the plurality of analyte labeling agents are the different (e.g., members of the plurality of analyte labeling agents can have two or more species of analyte binding moieties, wherein each of the two or more species of analyte binding moieties binds a single species of analyte, e.g., at different binding sites). In some embodiments, the plurality of analytes includes multiple different species of analyte (e.g., multiple different species of polypeptides).

In other instances, e.g., to facilitate sample multiplexing, a labeling agent that is specific to a particular cell feature may have a first plurality of the labeling agent (e.g., an antibody or lipophilic moiety) coupled to a first reporter oligonucleotide and a second plurality of the labeling agent coupled to a second reporter oligonucleotide.

In some aspects, these reporter oligonucleotides may comprise nucleic acid barcode sequences that permit identification of the labeling agent which the reporter oligonucleotide is coupled to. The selection of oligonucleotides as the reporter may provide advantages of being able to generate significant diversity in terms of sequence, while also being readily attachable to most biomolecules, e.g., antibodies, etc., as well as being readily detected, e.g., using the *in situ* detection techniques described herein.

Attachment (coupling) of the reporter oligonucleotides to the labeling agents may be achieved through any of a variety of direct or indirect, covalent or non-covalent associations or attachments. For example, oligonucleotides may be covalently attached to a portion of a labeling agent (such a protein, e.g., an antibody or antibody fragment) using chemical conjugation techniques (e.g., Lightning-Link^{®} antibody labeling kits available from Innova Biosciences), as well as other non-covalent attachment mechanisms, e.g., using biotinylated antibodies and oligonucleotides (or beads that include one or more biotinylated linker, coupled to oligonucleotides) with an avidin or streptavidin linker. Antibody and oligonucleotide biotinylation techniques are available. See, e.g., Fang, et al., "Fluoride-Cleavable Biotinylation Phosphoramidite for 5'-end-Labelling and Affinity Purification of Synthetic Oligonucleotides," Nucleic Acids Res. Jan. 15, 2003; 31(2):708-715, which is entirely incorporated herein by reference for all purposes. Likewise, protein and peptide biotinylation techniques have been developed and are readily available. See, e.g., U.S. Pat. No. 6,265,552, which is entirely incorporated herein by reference for all purposes. Furthermore, click reaction chemistry may be used to couple reporter oligonucleotides to labeling agents. Commercially available kits, such as those from Thunderlink and Abcam, and techniques common in the art may be used to couple reporter oligonucleotides to labeling agents as appropriate. In another example, a labeling agent is indirectly (e.g., via hybridization) coupled to a reporter oligonucleotide comprising a barcode sequence that identifies the label agent. For instance, the labeling agent may be directly coupled (e.g., covalently bound) to a hybridization oligonucleotide that comprises a sequence that hybridizes with a sequence of the reporter oligonucleotide. Hybridization of the hybridization oligonucleotide to the reporter oligonucleotide couples the labeling agent to the reporter oligonucleotide. In some embodiments, the reporter oligonucleotides are releasable from the labeling agent, such as upon application of a stimulus. For example, the reporter oligonucleotide may be attached to the labeling agent through a labile bond (e.g., chemically labile, photolabile, thermally labile, etc.) as generally described for releasing molecules from supports elsewhere herein.

In some cases, the labeling agent can comprise a reporter oligonucleotide and a label. In some embodiments, a label comprises a fluorophore, a radioisotope, a molecule capable of a colorimetric reaction, a magnetic particle, or any other suitable molecule or compound capable of detection. In some embodiments, the label is conjugated to a labeling agent (or reporter oligonucleotide) either directly or indirectly (e.g., the label is conjugated to a molecule that can bind to the labeling agent or reporter oligonucleotide). In some cases, a label is conjugated to a first oligonucleotide that is complementary (e.g., hybridizes) to a sequence of the reporter oligonucleotide.

In some embodiments, multiple different species of analytes (e.g., polypeptides) from the biological sample is subsequently associated with the one or more physical properties of the biological sample. For example, the multiple different species of analytes can be associated with locations of the analytes in the biological sample. Such information (e.g., proteomic information when the analyte binding moiety(ies) recognizes a polypeptide(s)) can be used in association with other spatial information (e.g., genetic information from the biological sample, such as DNA sequence information, transcriptome information (e.g., sequences of transcripts), or both). For example, a cell surface protein of a cell can be associated with one or more physical properties of the cell (e.g., a shape, size, activity, or a type of the cell). The one or more physical properties can be characterized by imaging the cell. The cell can be bound by an analyte labeling agent comprising an analyte binding moiety that binds to the cell surface protein and an analyte binding moiety barcode that identifies that analyte binding moiety. In some embodiments, results of protein analysis in a sample (e.g., a tissue sample or a cell) are associated with DNA and/or RNA analysis in the sample.

### (iii) Generation of Products

In some embodiments, provided herein are methods and compositions for analyzing one or more products of an endogenous analyte and/or a labeling agent in a biological sample. In some embodiments, an endogenous analyte (e.g., a viral or cellular DNA or RNA) or a product (e.g., a hybridization product, a ligation product, an extension product (e.g., by a DNA or RNA polymerase), a replication product, a transcription/reverse transcription product, and/or an amplification product such as a rolling circle amplification (RCA) product) thereof is analyzed. In some embodiments, a labeling agent that directly or indirectly binds to an analyte in the biological sample is analyzed. In some embodiments, a product (e.g., a hybridization product, a ligation product, an extension product (e.g., by a DNA or RNA polymerase), a replication product, a transcription/reverse transcription product, and/or an amplification product such as a rolling circle amplification (RCA) product) of a labeling agent that directly or indirectly binds to an analyte in the biological sample is analyzed.

### (a) Hybridization

In some embodiments, a hybridization product comprising the pairing of substantially complementary or complementary nucleic acid sequences within two different molecules is analyzed. For example, hybridization of an endogenous analyte or the labeling agent (e.g., reporter oligonucleotide attached thereto) with another endogenous molecule or another labeling agent or a probe can be analyzed. Pairing can be achieved by any process in which a nucleic acid sequence joins with a substantially or fully complementary sequence through base pairing to form a hybridization complex. For purposes of hybridization, two nucleic acid sequences are "substantially complementary" if at least 60% (e.g., at least 70%, at least 80%, or at least 90%) of their individual bases are complementary to one another.

Various probes and probe sets can be hybridized to an endogenous analyte and/or a labeling agent and each probe may comprise one or more barcode sequences. Exemplary barcoded probes or probe sets may be based on a padlock probe, a gapped padlock probe, a SNAIL (Splint Nucleotide Assisted Intramolecular Ligation) probe set, a PLAYR (Proximity Ligation Assay for RNA) probe set, a PLISH (Proximity Ligation *in situ* Hybridization) probe set, and RNA-templated ligation probes. The specific probe or probe set design can vary.

### (b) Ligation

In some embodiments, a ligation product of an endogenous analyte and/or a labeling agent is analyzed. In some embodiments, the ligation product is formed between two or more endogenous analytes. In some embodiments, the ligation product is formed between two or more labeling agents. In some embodiments, the ligation product is an intramolecular ligation of an endogenous analyte. In some embodiments, the ligation product is an intramolecular ligation product or an intermolecular ligation product, for example, the ligation product is generated by the circularization of a circularizable probe or probe set upon hybridization to a target sequence. In some embodiments, the target sequence is comprised in an endogenous analyte (e.g., nucleic acid such as a genomic DNA or mRNA) or a product thereof (e.g., cDNA from a cellular mRNA transcript), or in a labeling agent (e.g., the reporter oligonucleotide) or a product thereof.

In some embodiments, provided herein is a probe or probe set capable of DNA-templated ligation, such as from a cDNA molecule. See, e.g., U.S. Pat. 8,551,710, which is hereby incorporated by reference in its entirety. In some embodiments, provided herein is a probe or probe set capable of RNA-templated ligation. See, e.g., U.S. Pat. Pub. 2020/0224244 which is hereby incorporated by reference in its entirety. In some embodiments, the probe set is a SNAIL probe set. See, e.g., U.S. Pat. Pub. 20190055594, which is hereby incorporated by reference in its entirety. In some embodiments, provided herein is a multiplexed proximity ligation assay. See, e.g., U.S. Pat. Pub. 20140194311 which is hereby incorporated by reference in its entirety. In some embodiments, provided herein is a probe or probe set capable of proximity ligation, for instance a proximity ligation assay for RNA (e.g., PLAYR) probe set. See, e.g., U.S. Pat. Pub. 20160108458, which is hereby incorporated by reference in its entirety. In some embodiments, a circular probe is indirectly hybridized to the target nucleic acid. In some embodiments, the circular construct is formed from a probe set capable of proximity ligation, for instance a proximity ligation *in situ* hybridization (PLISH) probe set. See, e.g., U.S. Pat. Pub. 2020/0224243 which is hereby incorporated by reference in its entirety.

In some embodiments, the ligation involves chemical ligation (e.g., click chemistry ligation). In some embodiments, the chemical ligation involves template dependent ligation. In some embodiments, the chemical ligation involves template independent ligation. In some embodiments, the click reaction is a template-independent reaction (*see, e.g.,* Xiong and Seela (2011), J. Org. Chem. 76(14): 5584-5597, incorporated by reference herein in its entirety). In some embodiments, the click reaction is a template-dependent reaction or template-directed reaction. In some embodiments, the template-dependent reaction is sensitive to base pair mismatches such that reaction rate is significantly higher for matched versus unmatched templates. In some embodiments, the click reaction is a nucleophilic addition template-dependent reaction. In some embodiments, the click reaction is a cyclopropane-tetrazine template-dependent reaction.

In some embodiments, the ligation involves enzymatic ligation. In some embodiments, the enzymatic ligation involves use of a ligase. In some aspects, the ligase used herein comprises an enzyme that is commonly used to join polynucleotides together or to join the ends of a single polynucleotide. An RNA ligase, a DNA ligase, or another variety of ligase can be used to ligate two nucleotide sequences together. Ligases comprise ATP-dependent double-strand polynucleotide ligases, NAD-i-dependent double-strand DNA or RNA ligases and single-strand polynucleotide ligases, for example any of the ligases described in EC 6.5.1.1 (ATP-dependent ligases), EC 6.5.1.2 (NAD+-dependent ligases), EC 6.5.1.3 (RNA ligases). Specific examples of ligases comprise bacterial ligases such as E. coli DNA ligase, Tth DNA ligase, Thermococcus sp. (strain 9° N) DNA ligase (9°N^{™} DNA ligase, New England Biolabs), Taq DNA ligase, Ampligase^{™} (Epicentre Biotechnologies) and phage ligases such as T3 DNA ligase, T4 DNA ligase and T7 DNA ligase and mutants thereof. In some embodiments, the ligase is a T4 RNA ligase. In some embodiments, the ligase is a splintR ligase. In some embodiments, the ligase is a single stranded DNA ligase. In some embodiments, the ligase is a T4 DNA ligase. In some embodiments, the ligase is a ligase that has an DNA-splinted DNA ligase activity. In some embodiments, the ligase is a ligase that has an RNA-splinted DNA ligase activity.

In some embodiments, the ligation herein is a direct ligation. In some embodiments, the ligation herein is an indirect ligation. "Direct ligation" means that the ends of the polynucleotides hybridize immediately adjacently to one another to form a substrate for a ligase enzyme resulting in their ligation to each other (intramolecular ligation). Alternatively, "indirect" means that the ends of the polynucleotides hybridize non-adjacently to one another, e.g., separated by one or more intervening nucleotides or "gaps". In some embodiments, said ends are not ligated directly to each other, but instead occurs either via the intermediacy of one or more intervening (so-called "gap" or "gap-filling" (oligo)nucleotides) or by the extension of the 3' end of a probe to "fill" the "gap" corresponding to said intervening nucleotides (intermolecular ligation). In some cases, the gap of one or more nucleotides between the hybridized ends of the polynucleotides is "filled" by one or more "gap" (oligo)nucleotide(s) which are complementary to a splint, padlock probe, or target nucleic acid. The gap may be a gap of 1 to 60 nucleotides or a gap of 1 to 40 nucleotides or a gap of 3 to 40 nucleotides. In specific embodiments, the gap is a gap of about 1, 2, 3, 4, 5, 6, 7, 8, 9 or 10 or more nucleotides, of any integer (or range of integers) of nucleotides in between the indicated values. In some embodiments, the gap between said terminal regions is filled by a gap oligonucleotide or by extending the 3' end of a polynucleotide. In some cases, ligation involves ligating the ends of the probe to at least one gap (oligo)nucleotide, such that the gap (oligo)nucleotide becomes incorporated into the resulting polynucleotide. In some embodiments, the ligation herein is preceded by gap filling. In other embodiments, the ligation herein does not require gap filling.

In some embodiments, ligation of the polynucleotides produces polynucleotides with melting temperature higher than that of unligated polynucleotides. Thus, in some aspects, ligation stabilizes the hybridization complex containing the ligated polynucleotides prior to subsequent steps, comprising amplification and detection.

In some aspects, a high fidelity ligase, such as a thermostable DNA ligase (e.g., a *Taq* DNA ligase), is used. Thermostable DNA ligases are active at elevated temperatures, allowing further discrimination by incubating the ligation at a temperature near the melting temperature (Tₘ) of the DNA strands. This selectively reduces the concentration of annealed mismatched substrates (expected to have a slightly lower Tₘ around the mismatch) over annealed fully base-paired substrates. Thus, high-fidelity ligation can be achieved through a combination of the intrinsic selectivity of the ligase active site and balanced conditions to reduce the incidence of annealed mismatched dsDNA.

In some embodiments, the ligation herein is a proximity ligation of ligating two (or more) nucleic acid sequences that are in proximity with each other, e.g., through enzymatic means (e.g., a ligase). In some embodiments, proximity ligation can include a "gap-filling" step that involves incorporation of one or more nucleic acids by a polymerase, based on the nucleic acid sequence of a template nucleic acid molecule, spanning a distance between the two nucleic acid molecules of interest (see, e.g., U.S. Patent No. 7,264,929, the entire contents of which are incorporated herein by reference). A wide variety of different methods can be used for proximity ligating nucleic acid molecules, including (but not limited to) "sticky-end" and "blunt-end" ligations. Additionally, single-stranded ligation can be used to perform proximity ligation on a single-stranded nucleic acid molecule. Sticky-end proximity ligations involve the hybridization of complementary single-stranded sequences between the two nucleic acid molecules to be joined, prior to the ligation event itself. Blunt-end proximity ligations generally do not include hybridization of complementary regions from each nucleic acid molecule because both nucleic acid molecules lack a single-stranded overhang at the site of ligation.

### (c) Primer Extension and Amplification

In some embodiments, a primer extension product of an analyte, a labeling agent, a probe or probe set bound to the analyte (e.g., a circularizable probe bound to genomic DNA, mRNA, or cDNA), or a probe or probe set bound to the labeling agent (e.g., a circularizable probe bound to one or more reporter oligonucleotides from the same or different labeling agents) can be analyzed.

In some embodiments, a primer is a single-stranded nucleic acid sequence having a 3' end that is used as a substrate for a nucleic acid polymerase in a nucleic acid extension reaction. RNA primers are formed of RNA nucleotides, and are used in RNA synthesis, while DNA primers are formed of DNA nucleotides and used in DNA synthesis. Primers can also include both RNA nucleotides and DNA nucleotides (e.g., in a random or designed pattern). Primers can also include other natural or synthetic nucleotides described herein that can have additional functionality. In some examples, DNA primers are used to prime RNA synthesis and vice versa (e.g., RNA primers can be used to prime DNA synthesis). Primers can vary in length. For example, primers can be about 6 bases to about 120 bases. For example, primers can include up to about 25 bases. In some embodiments, a primer is a primer binding sequence. In some embodiments, a primer extension reaction is a method where two nucleic acid sequences become linked (e.g., hybridized) by an overlap of their respective terminal complementary nucleic acid sequences (e.g., 3' termini). Such linking can be followed by nucleic acid extension (e.g., an enzymatic extension) of one, or both termini using the other nucleic acid sequence as a template for extension. Enzymatic extension can be performed by an enzyme including, but not limited to, a polymerase and/or a reverse transcriptase.

In some embodiments, a product of an endogenous analyte and/or a labeling agent is an amplification product of one or more polynucleotides, for instance, a circular probe or circularizable probe or probe set. In some embodiments, the amplifying is achieved by performing rolling circle amplification (RCA). In some embodiments, an endogenous nucleic acid or fragment thereof hybridized to the circular probe or circularized probe is used to prime amplification. In some embodiments, a primer that hybridizes to the circular probe or circularized probe is added and used as such for amplification. In some embodiments, the RCA comprises a linear RCA, a branched RCA, a dendritic RCA, or any combination thereof.

In some embodiments, amplification of a circular probe or circularizable probe or probe set is primed by the target RNA. The target RNA can optionally be immobilized in the biological sample. In some embodiments, the target RNA is cleaved by an enzyme (e.g., RNase H). In some embodiments, the target RNA is cleaved at a position downstream of the target sequences bound to the circular probe or circularizable probe or probe set. In some aspects, the methods disclosed herein allow targeting of RNase H activity to a particular region in a target RNA that is adjacent to or overlapping with a target sequence for a probe or probe set. For example, a nucleic acid oligonucleotide is designed to hybridize to a complementary oligonucleotide hybridization region in the target RNA. In some embodiments, a nucleic acid oligonucleotide is used to provide a DNA-RNA duplex for RNase H cleavage of the target RNA in the DNA-RNA duplex. In some embodiments, the oligonucleotide binds to the target RNA at a position that overlaps with the target sequence of the probe or probe set by about 1 to about 20 nucleotides or by about 8 to about 10 nucleotides. The cleaved target RNA itself can then be used to prime RCA of the circular probe generated from a circularizable probe or probe set (e.g., target-primed RCA). In some cases, a plurality of nucleic acid oligonucleotides is used to perform target-primed RCA for a plurality of different target RNAs.

In any of the embodiments herein, the biological sample is contacted with the RNase H (and optionally with the nucleic acid oligonucleotide) before or during formation of the circularized gap-filled first probe or probe set. In some embodiments, the biological sample is contacted with the oligonucleotide and with the RNase H simultaneously or sequentially (in either order) before contacting the sample with the probe or probe set. In any of the embodiments herein, the biological sample is contacted with the RNase H (and optionally with the nucleic acid oligonucleotide) after formation of the circularized probe or probe set. In any of the embodiments herein, the RNase H comprises an RNase H1 and/or an RNAse H2. In some embodiments, RNase inactivating agents or inhibitors are added to the sample after cleaving the target RNA.

In some embodiments, the amplification is performed at a temperature between or between about 20°C and about 60°C. In some embodiments, the amplification is performed at a temperature between or between about 30°C and about 40°C. In some aspects, the amplification step, such as the rolling circle amplification (RCA) is performed at a temperature between at or about 25°C and at or about 50°C, such as at or about 25°C, 27°C, 29°C, 31°C, 33°C, 35°C, 37°C, 39°C, 41°C, 43°C, 45°C, 47°C, or 49°C.

In some embodiments, upon addition of a DNA polymerase in the presence of appropriate dNTP precursors and other cofactors, a primer is elongated to produce multiple copies of the circular template. This amplification step can utilize isothermal amplification or non-isothermal amplification. In some embodiments, after the formation of the hybridization complex and association of the amplification probe, the hybridization complex is rolling-circle amplified to generate a cDNA nanoball (e.g., amplicon) containing multiple copies of the cDNA. Techniques for rolling circle amplification (RCA) include linear RCA, a branched RCA, a dendritic RCA, or any combination thereof. (See, e.g., Baner et al, Nucleic Acids Research, 26:5073-5078, 1998; Lizardi et al, Nature Genetics 19:226, 1998; Mohsen et al., Acc Chem Res. 2016 November 15; 49(11): 2540-2550; Schweitzer et al. Proc. Natl Acad. Sci. USA 97:101 13-1 19, 2000; Faruqi et al, BMC Genomics 2:4, 2000; Nallur et al, Nucl. Acids Res. 29:el 18, 2001; Dean et al. Genome Res. 1 1 :l095- 1099, 2001; Schweitzer et al, Nature Biotech. 20:359-365, 2002; U.S. Patent Nos. 6,054,274, 6,291,187, 6,323,009, 6,344,329 and 6,368,801, all of which are herein incorporated by reference in their entireties). Exemplary polymerases for use in RCA comprise DNA polymerase such phi29 (φ29) polymerase, Klenow fragment, *Bacillus stearothermophilus* DNA polymerase (BST), T4 DNA polymerase, T7 DNA polymerase, or DNA polymerase I. In some aspects, DNA polymerases that have been engineered or mutated to have desirable characteristics can be employed. In some embodiments, the polymerase is phi29 DNA polymerase.

In some aspects, during the amplification step, modified nucleotides are added to the reaction to incorporate the modified nucleotides in the amplification product (e.g., nanoball). Exemplary of the modified nucleotides comprise amine-modified nucleotides. In some aspects of the methods, for example, for anchoring or cross-linking of the generated amplification product (e.g., nanoball) to a scaffold, to cellular structures and/or to other amplification products (e.g., other nanoballs). In some aspects, the amplification products comprises a modified nucleotide, such as an amine-modified nucleotide. In some embodiments, the amine-modified nucleotide comprises an acrylic acid N- hydroxysuccinimide moiety modification. Examples of other amine-modified nucleotides comprise, but are not limited to, a 5- Aminoallyl-dUTP moiety modification, a 5-Propargylamino-dCTP moiety modification, a N6-6-Aminohexyl-dATP moiety modification, or a 7-Deaza-7-Propargylamino-dATP moiety modification.

In some aspects, the polynucleotides and/or amplification product (e.g., amplicon) are anchored to a polymer matrix. For example, the polymer matrix comprises a hydrogel. In some embodiments, one or more of the polynucleotide probe(s) are modified to contain functional groups that can be used as an anchoring site to attach the polynucleotide probes and/or amplification product to a polymer matrix. Exemplary modification and polymer matrix that can be employed in accordance with the provided embodiments comprise those described in, for example, U.S. Pat. No. 10,138,509; U.S. Pat. No. 10,266,888; US 2016/0024555; US 2018/025183; and US 2017/0219465, which are herein incorporated by reference in their entireties. In some examples, the scaffold also contains modifications or functional groups that can react with or incorporate the modifications or functional groups of the probe set or amplification product. In some examples, the scaffold can comprise oligonucleotides, polymers or chemical groups, to provide a matrix and/or support structures.

The amplification products may be immobilized within the matrix generally at the location of the nucleic acid being amplified, thereby creating a localized colony of amplicons. The amplification products may be immobilized within the matrix by steric factors. The amplification products may also be immobilized within the matrix by covalent or noncovalent bonding. In this manner, the amplification products may be considered to be attached to the matrix. By being immobilized to the matrix, such as by covalent bonding or cross-linking, the size and spatial relationship of the original amplicons is maintained. By being immobilized to the matrix, such as by covalent bonding or cross-linking, the amplification products are resistant to movement or unraveling under mechanical stress.

In some aspects, the amplification products are copolymerized and/or covalently attached to the surrounding matrix thereby preserving their spatial relationship and any information inherent thereto. For example, if the amplification products are those generated from DNA or RNA within a cell embedded in the matrix, the amplification products can also be functionalized to form covalent attachment to the matrix preserving their spatial information within the cell thereby providing a subcellular localization distribution pattern. In some embodiments, the provided methods involve embedding the one or more polynucleotide probe sets and/or the amplification products in the presence of hydrogel subunits to form one or more hydrogel-embedded amplification products. In some embodiments, the hydrogel-tissue chemistry described comprises covalently attaching nucleic acids to in situ synthesized hydrogel for tissue clearing, enzyme diffusion, and multiple-cycle sequencing while an existing hydrogel-tissue chemistry method cannot. In some embodiments, to enable amplification product embedding in the tissue-hydrogel setting, amine-modified nucleotides are comprised in the amplification step (e.g., RCA), functionalized with an acrylamide moiety using acrylic acid N-hydroxysuccinimide esters, and copolymerized with acrylamide monomers to form a hydrogel.

In some embodiments, the RCA template comprises the target analyte, or a part thereof, where the target analyte is a nucleic acid, or it is provided or generated as a proxy, or a marker, for the analyte. In some embodiments, different analytes are detected *in situ* in one or more cells using a RCA-based detection system, e.g., where the signal is provided by generating an RCA product from a circular RCA template which is provided or generated in the assay, and the RCA product is detected to detect the corresponding analyte. The RCA product may thus be regarded as a reporter which is detected to detect the target analyte. However, the RCA template may also be regarded as a reporter for the target analyte; the RCA product is generated based on the RCA template, and comprises complementary copies of the RCA template. The RCA template determines the signal which is detected, and is thus indicative of the target analyte. As will be described in more detail below, the RCA template may be a probe, or a part or component of a probe, or may be generated from a probe, or it may be a component of a detection assay (e.g., a reagent in a detection assay), which is used as a reporter for the assay, or a part of a reporter, or signal-generation system. The RCA template used to generate the RCP may thus be a circular (e.g. circularized) reporter nucleic acid molecule, namely from any RCA-based detection assay which uses or generates a circular nucleic acid molecule as a reporter for the assay. Since the RCA template generates the RCP reporter, it may be viewed as part of the reporter system for the assay.

In some embodiments, a product herein includes a molecule or a complex generated in a series of reactions, e.g., hybridization, ligation, extension, replication, transcription/reverse transcription, and/or amplification (e.g., rolling circle amplification), in any suitable combination.

### C. Barcode Sequences

In some embodiments, a genomic region herein is associated with one or more barcode(s) that identify the genomic region. In some embodiments, a genomic region herein is associated with at least two, three, four, five, six, seven, eight, nine, ten, or more barcodes that identify the genomic region. In some embodiments, a genomic region herein is associated with a single barcode that identifies the genomic region. In some embodiments, the genomic region associated with the one or more barcode(s) by binding of a plurality of probes that comprise the one or more barcode(s) to a plurality of RNAs expressed from different subregions of the genomic region. The one or more barcode(s) corresponding to the genomic region are associated with the plurality of RNAs expressed from the different subregions of the genomic region by binding of probes comprising the one or more barcodes to the plurality of RNAs. In some instances, a barcode is associated with a genomic region by binding probes comprising the barcode directly or indirectly to a plurality of different RNAs expressed from the genomic region. In some aspects, a barcode comprises about 4, 5, 6, 7, 8, 9, 10, 11, 12, 13, 14, 15, 16, 17, 18, 19, 20, 21, 22, 23, 24, 25, 26, 27, 28, 29, 30, or more than 30 nucleotides.

In some embodiments, an analyte described herein is associated with one or more barcode(s), e.g., at least two, three, four, five, six, seven, eight, nine, ten, or more barcodes. Barcodes can spatially-resolve molecular components found in biological samples, for example, within a cell or a tissue sample. In some embodiments, a barcode is attached to an analyte or to another moiety or structure in a reversible or irreversible manner. In some instances, a barcode is associated with an analyte or target nucleic acid sequence by binding a probe comprising the barcode directly or indirectly to the analyte or target nucleic acid sequence. In some aspects, a barcode comprises about 4, 5, 6, 7, 8, 9, 10, 11, 12, 13, 14, 15, 16, 17, 18, 19, 20, 21, 22, 23, 24, 25, 26, 27, 28, 29, 30, or more than 30 nucleotides.

In some embodiments, a barcode includes two or more sub-barcodes that together function as a single barcode. For example, a polynucleotide barcode can include two or more polynucleotide sequences (e.g., sub-barcodes) that are separated by one or more non-barcode sequences. In some embodiments, the one or more barcodes also provide a platform for targeting functionalities, such as oligonucleotides, oligonucleotide-antibody conjugates, oligonucleotide-streptavidin conjugates, modified oligonucleotides, affinity purification, detectable moieties, enzymes, enzymes for detection assays or other functionalities, and/or for detection and identification of the polynucleotide. In any of the preceding embodiments, the methods provided herein include analyzing the barcodes by sequential hybridization and detection with a plurality of labelled probes (e.g., detection oligos).

In some embodiments, in a barcode sequencing method, barcode sequences are detected for identification of other molecules including nucleic acid molecules (DNA or RNA) longer than the barcode sequences themselves, as opposed to direct sequencing of the longer nucleic acid molecules. In some embodiments, a N-mer barcode sequence comprises 4^{N} complexity given a sequencing read of N bases, and a much shorter sequencing read may be required for molecular identification compared to non-barcode sequencing methods such as direct sequencing. For example, 1024 molecular species may be identified using a 5-nucleotide barcode sequence (4⁵=1024), whereas 8 nucleotide barcodes can be used to identify up to 65,536 molecular species, a number greater than the total number of distinct genes in the human genome. In some embodiments, the barcode sequences contained in the probes or RCPs are detected, rather than endogenous sequences, which can be an efficient read-out in terms of information per cycle of sequencing. Because the barcode sequences are pre-determined, they can also be designed to feature error detection and correction mechanisms, see, e.g., U.S. Pat. Pub. 20190055594 and U.S. Pat. Pub 20210164039, which are hereby incorporated by reference in their entirety.

### IV. Compositions and Kits

In some aspects, provided herein are compositions comprising any of the probes or probe sets described herein. Also provided herein are systems or kits for analyzing an analyte in a biological sample according to any of the methods described herein. In some embodiments, provided herein is a system or kit comprising a plurality of probes for detecting RNA expression from a plurality of subregions within a genomic region, wherein the plurality of probes comprises at least two different probes capable of hybridizing to at least two different RNA molecules expressed from different subregions within the genomic region, wherein the plurality of probes individually comprise a common barcode sequence that identifies the genomic region. In some embodiments, the plurality of probes is a plurality of circularizable probes or probe sets, wherein the circularizable probes or probe sets comprise nucleic acid hybridization regions complementary to the RNA molecules expressed from different subregions within the genomic region such that the circularizable probes or probe sets are circularized by ligation upon hybridization to the RNA molecules. In some instances, the ligation is with gap-filling. In some instances, the ligation is without gap-filling. In some cases, the kit comprises two, three, four, or more different pluralities of probes comprising barcodes corresponding to different genomic regions.

The various components of the system or kit may be present in separate containers or certain compatible components may be pre-combined into a single container. In some embodiments, the systems or kits further contain instructions for using the components of the kit to practice the provided methods.

In some embodiments, the systems or kits contain reagents and/or consumables required for performing one or more steps of the provided methods. In some embodiments, the systems or kits contain reagents for fixing, embedding, and/or permeabilizing the biological sample. In some embodiments, the systems or kits contain reagents, such as enzymes and buffers for ligation and/or amplification, such as ligases and/or polymerases. In some aspects, the system or kit comprises any of the reagents described herein, e.g., wash buffer and ligation buffer. In some embodiments, the systems or kits contain reagents for detection and/or sequencing, such as barcode detection probes or detectable labels. In some embodiments, the systems or kits optionally contain other components, for example nucleic acid primers,

Provided herein is a system comprising a first plurality of probes for detecting RNA expression from a plurality of subregions within a first genomic region, wherein the first plurality of probes comprises at least two different probes capable of hybridizing to at least two different RNA molecules expressed from different subregions within the first genomic region, and wherein the first plurality of probes individually comprise a first common barcode sequence that identifies the first genomic region; and a second plurality of probes for detecting RNA expression from a plurality of subregions within a second genomic region, wherein the second plurality of probes comprises at least two different probes capable of hybridizing to at least two different RNA molecules expressed from different subregions within the second genomic region, and wherein the second plurality of probes individually comprise a second common barcode sequence that identifies the second genomic region. In some instances, the first plurality of probes comprises at least 5, at least 10, at least 15, or at least 20 different probes capable of hybridizing to different RNA molecules expressed from different subregions within the genomic region. In some instances, the second plurality of probes comprises at least 5, at least 10, at least 15, or at least 20 different probes capable of hybridizing to different RNA molecules expressed from different subregions within the genomic region. In some instances, the different subregions span at least 1, at least 10, at least 20, or at least 50 megabases within the first genomic region or within the second genomic region. In some instances, individual probes of the first plurality of probes and/or the second plurality of probes further comprise a gene-specific barcode sequence. In some instances, individual probes of the first plurality of probes and/or the second plurality of probes do not comprise a gene-specific barcode sequence.

In some aspects, the first plurality of probes comprises circular probes or circularizable probes or probe sets configured to hybridize to each of the different RNA molecules expressed from the different subregions within the first genomic region. In some instances, the second plurality of probes comprises circular probes or circularizable probes or probe sets configured to hybridize to each of the different RNA molecules expressed from the different subregions within the second genomic region.

In some aspects, a system comprises a ligase for circularizing a nucleic acid molecule of the plurality of probes (e.g., as described in Section II.A). In some aspects, a system comprises reagents for performing a ligation reaction. In some aspects, a system comprises reagents for performing an amplification reaction (e.g., RCA reaction). In some aspects, a system comprises a polymerase for generating a plurality of rolling circle amplification products. In some instances, the system comprises a plurality of detectably labeled probes that binds directly or indirectly to the first common barcode sequence or the second barcode sequence or a complement thereof in hybridized probes of the first plurality of probes or the second plurality of probes, or in products thereof. In some cases, the system comprises one or more reagents for performing sequencing-by-synthesis (SBS), sequencing-by-avidity (SBA) or sequencing-by-binding (SBB) to detect the first common barcode sequence and/or the second common barcode sequence.

### V. Opto-Fluidic Instruments for Analysis of Biological Samples

Provided herein is an instrument having integrated optics and fluidics modules (an "opto-fluidic instrument" or "opto-fluidic system") for detecting target molecules (*e.g*., nucleic acids, proteins, antibodies, *etc.*) in biological samples (*e.g.,* one or more cells or a tissue sample) as described herein. In an opto-fluidic instrument, the fluidics module is configured to deliver one or more reagents (*e.g*., detectably labeled probes) to the biological sample and/or remove spent reagents therefrom. Additionally, the optics module is configured to illuminate the biological sample with light having one or more spectral emission curves (over a range of wavelengths) and subsequently capture one or more images of emitted light signals from the biological sample during one or more probing cycles (e.g., as described in Section II.C). In various embodiments, the captured images are processed in real time and/or at a later time to determine the presence of the one or more target molecules in the biological sample, as well as three-dimensional position information associated with each detected target molecule. Additionally, the opto-fluidics instrument includes a sample module configured to receive (and, optionally, secure) one or more biological samples. In some instances, the sample module includes an X-Y stage configured to move the biological sample along an X-Y plane (*e.g.,* perpendicular to an objective lens of the optics module).

In various embodiments, the opto-fluidic instrument is configured to analyze one or more target molecules in their naturally occurring place (*e.g., in situ*) within the biological sample. For example, an opto-fluidic instrument may be an *in-situ* analysis system used to analyze a biological sample and detect target molecules including but not limited to DNA, RNA, proteins, antibodies, and/or the like.

It is to be noted that, although the above discussion relates to an opto-fluidic instrument that can be used for *in situ* target molecule detection via probe hybridization, the discussion herein equally applies to any opto-fluidic instrument that employs any imaging or target molecule detection technique. That is, for example, an opto-fluidic instrument may include a fluidics module that includes fluids needed for establishing the experimental conditions required for the probing of target molecules in the sample. Further, such an opto-fluidic instrument may also include a sample module configured to receive the sample, and an optics module including an imaging system for illuminating (*e.g*., exciting one or more fluorescent probes within the sample) and/or imaging light signals received from the probed sample. The *in-situ* analysis system may also include other ancillary modules configured to facilitate the operation of the opto-fluidic instrument, such as, but not limited to, cooling systems, motion calibration systems, etc.

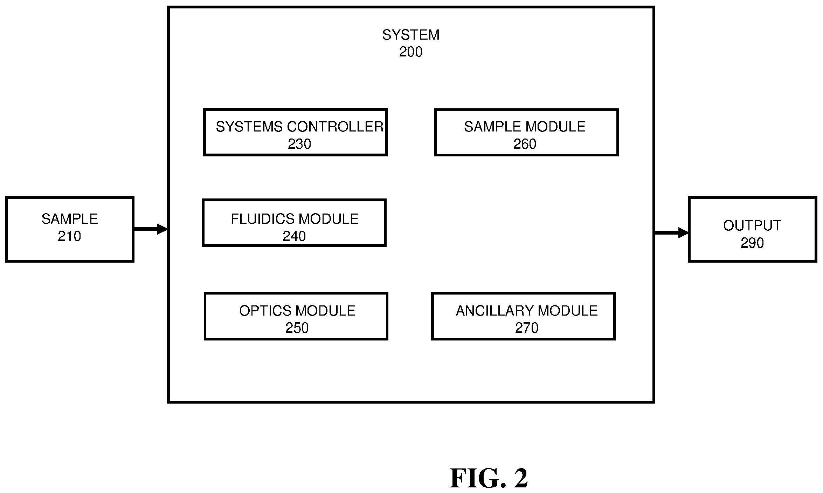

FIG. 2 shows an example workflow of analysis of a biological sample 210 (*e.g.,* cell or tissue sample) using an opto-fluidic instrument 220, according to various embodiments. In various embodiments, the sample 210 is a biological sample (e.g., a tissue) that includes molecules such as DNA, RNA, proteins, antibodies, etc. For example, the sample 210 can be a sectioned tissue that is treated to access the RNA thereof for labeling with probes described herein. Ligation of the probes may generate a circular probe which can be enzymatically amplified and bound with detectably labeled probes, which can create bright signal that is convenient to image and has a high signal-to-noise ratio.

In various embodiments, the sample 210 is placed in the opto-fluidic instrument 220 for analysis and detection of the molecules in the sample 210. In various embodiments, the opto-fluidic instrument 220 is a system configured to facilitate the experimental conditions conducive for the detection of the target molecules. For example, the opto-fluidic instrument 220 can include a fluidics module 240, an optics module 250, a sample module 260, and an ancillary module 270, and these modules may be operated by a system controller 230 to create the experimental conditions for the probing of the molecules in the sample 210 by selected probes (e.g., circularizable DNA probes), as well as to facilitate the imaging of the probed sample (e.g., by an imaging system of the optics module 250). In various embodiments, the various modules of the opto-fluidic instrument 220 are separate components in communication with each other, or at least some of them are integrated together.

In various embodiments, the sample module 260 is configured to receive the sample 210 into the opto-fluidic instrument 220. For instance, the sample module 260 may include a sample interface module (SIM) that is configured to receive a sample device (e.g., cassette) onto which the sample 210 can be deposited. That is, the sample 210 is placed in the opto-fluidic instrument 220 by depositing the sample 210 (e.g., the sectioned tissue) on a sample device that is then inserted into the SIM of the sample module 260. In some instances, the sample module 260 may also include an X-Y stage onto which the SIM is mounted. The X-Y stage may be configured to move the SIM mounted thereon (e.g., and as such the sample device containing the sample 210 inserted therein) in perpendicular directions along the two-dimensional (2D) plane of the opto-fluidic instrument 220. Additional discussion related the SIM can be found in US Provisional Application No.: 63/348,879, filed June 3, 2022, titled "Methods, Systems, and Devices for Sample Interface," which is incorporated herein by reference in its entirety.

The experimental conditions that are conducive for the detection of the molecules in the sample 210 may depend on the target molecule detection technique that is employed by the opto-fluidic instrument 220. For example, in various embodiments, the opto-fluidic instrument 220 is a system that is configured to detect molecules in the sample 210 via hybridization of probes. In such cases, the experimental conditions can include molecule hybridization conditions that result in the intensity of hybridization of the target molecule (e.g., nucleic acid) to a probe (e.g., oligonucleotide) being significantly higher when the probe sequence is complementary to the target molecule than when there is a single-base mismatch. The hybridization conditions include the preparation of the sample 210 using reagents such as washing/stripping reagents, hybridizing reagents, etc., and such reagents may be provided by the fluidics module 240.

In various embodiments, the fluidics module 240 includes one or more components that may be used for storing the reagents, as well as for transporting said reagents to and from the sample device containing the sample 210. For example, the fluidics module 240 may include reservoirs configured to store the reagents, as well as a waste container configured for collecting the reagents (e.g., and other waste) after use by the opto-fluidic instrument 220 to analyze and detect the molecules of the sample 210. Further, the fluidics module 240 may also include pumps, tubes, pipettes, etc., that are configured to facilitate the transport of the reagent to the sample device (e.g., and as such the sample 210). For instance, the fluidics module 240 may include pumps ("reagent pumps") that are configured to pump washing/stripping reagents to the sample device for use in washing/stripping the sample 210 (e.g., as well as other washing functions such as washing an objective lens of the imaging system of the optics module 250).

In various embodiments, the ancillary module 270 is a cooling system of the opto-fluidic instrument 220, and the cooling system may include a network of coolant-carrying tubes that are configured to transport coolants to various modules of the opto-fluidic instrument 220 for regulating the temperatures thereof. In such cases, the fluidics module 240 may include coolant reservoirs for storing the coolants and pumps (e.g., "coolant pumps") for generating a pressure differential, thereby forcing the coolants to flow from the reservoirs to the various modules of the opto-fluidic instrument 220 via the coolant-carrying tubes. In some instances, the fluidics module 240 includes returning coolant reservoirs that may be configured to receive and store returning coolants, e.g., heated coolants flowing back into the returning coolant reservoirs after absorbing heat discharged by the various modules of the opto-fluidic instrument 220. In such cases, the fluidics module 240 may also include cooling fans that are configured to force air (e.g., cool and/or ambient air) into the returning coolant reservoirs to cool the heated coolants stored therein. In some instance, the fluidics module 240 may also include cooling fans that are configured to force air directly into a component of the opto-fluidic instrument 220 so as to cool said component. For example, the fluidics module 240 may include cooling fans that are configured to direct cool or ambient air into the system controller 230 to cool the same.

As discussed above, the opto-fluidic instrument 220 may include an optics module 250 which include the various optical components of the opto-fluidic instrument 220, such as but not limited to a camera, an illumination module (e.g., LEDs), an objective lens, and/or the like. The optics module 250 may include a fluorescence imaging system that is configured to image the fluorescence emitted by the probes (e.g., oligonucleotides) in the sample 210 after the probes are excited by light from the illumination module of the optics module 250.

In some instances, the optics module 250 also includes an optical frame onto which the camera, the illumination module, and/or the X-Y stage of the sample module 260 may be mounted.

In various embodiments, the system controller 230 is configured to control the operations of the opto-fluidic instrument 220 (e.g., and the operations of one or more modules thereof). In some instances, the system controller 230 may take various forms, including a processor, a single computer (or computer system), or multiple computers in communication with each other. In various embodiments, the system controller 230 is communicatively coupled with data storage, set of input devices, display system, or a combination thereof. In some cases, some or all of these components are considered to be part of or otherwise integrated with the system controller 230, are separate components in communication with each other, or are integrated together. In other examples, the system controller 230 is in communication with a cloud computing platform.

In various embodiments, the opto-fluidic instrument 220 analyzes the sample 210 and may generate the output 290 that includes indications of the presence of the target molecules in the sample 210. For instance, with respect to the example embodiment discussed above where the opto-fluidic instrument 220 employs a hybridization technique for detecting molecules, the opto-fluidic instrument 220 may cause the sample 210 to undergo successive rounds of detectably labeled probe hybridization (e.g., using two or more sets of fluorescent probes, where each set of fluorescent probes is excited by a different color channel) and be imaged to detect target molecules in the probed sample 210. In such cases, the output 290 may include optical signatures (e.g., a codeword) specific to each gene, which allow the identification of the target molecules.

Provided herein is a computer system configured to analyze a plurality of probes for detecting RNA expression from a plurality of subregions within a genomic region detected in a biological sample, wherein the biological sample is a cell or tissue sample; and the plurality of probes individually comprise a common barcode sequence that identifies the genomic region; wherein the computer system determines a copy number of the genomic region based on a barcode count corresponding to the genomic region for a cell in the biological sample. In some instances, the computer module is used to detect the common barcode sequence or a complement thereof to detect the plurality of probes in the cell or tissue sample. In some instances, the computer system compares the determined copy number of the genomic region of the cell in the biological sample to an additional determined copy number of an additional genomic region of an additional cell. In some cases, the computer system identifies the cell in the biological sample as a cancer cell or a non-cancer cell based on the determined copy number.

### VI. Terminology

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

The terms "polynucleotide" and "nucleic acid molecule", used interchangeably herein, refer to polymeric forms of nucleotides of any length, either ribonucleotides or deoxyribonucleotides. Thus, this term comprises, but is not limited to, single-, double-, or multi-stranded DNA or RNA, genomic DNA, cDNA, DNA-RNA hybrids, or a polymer comprising purine and pyrimidine bases or other natural, chemically or biochemically modified, non-natural, or derivatized nucleotide bases. The backbone of the polynucleotide can comprise sugars and phosphate groups (as may typically be found in RNA or DNA), or modified or substituted sugar or phosphate groups.

A "primer" used herein, in some embodiments, is an oligonucleotide, either natural or synthetic, that is capable, upon forming a duplex with a polynucleotide template, of acting as a point of initiation of nucleic acid synthesis and being extended from its 3' end along the template so that an extended duplex is formed. The sequence of nucleotides added during the extension process is determined by the sequence of the template polynucleotide. Primers usually are extended by a DNA polymerase.

In some instances, "ligation" refers to the formation of a covalent bond or linkage between the termini of two or more nucleic acids, e.g., oligonucleotides and/or polynucleotides, in a template-driven reaction. The nature of the bond or linkage may vary widely and the ligation, in some embodiments, is carried out enzymatically or chemically. As used herein, ligations are usually carried out enzymatically to form a phosphodiester linkage between a 5' carbon terminal nucleotide of one oligonucleotide with a 3' carbon of another nucleotide.

The term "about" as used herein refers to the usual error range for the respective value readily known to the skilled person in this technical field. Reference to "about" a value or parameter herein comprises (and describes) embodiments that are directed to that value or parameter per se.

As used herein, the singular forms "a," "an," and "the" comprise plural referents unless the context clearly dictates otherwise. For example, "a" or "an" means "at least one" or "one or more."

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be comprised in the smaller ranges, and are also encompassed within the claimed subject matter, subject to any specifically excluded limit in the stated range. Where the stated range comprises one or both of the limits, ranges excluding either or both of those comprised limits are also comprised in the claimed subject matter. This applies regardless of the breadth of the range.

Use of ordinal terms such as "first", "second", "third", etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements. Similarly, use of a), b), etc., or i), ii), etc. does not by itself connote any priority, precedence, or order of steps in the claims. Similarly, the use of these terms in the specification does not by itself connote any required priority, precedence, or order.

### VII. Exemplary Embodiments

Among the provided embodiments are:
Embodiment 1. A method for analyzing copy number of a genomic region in a biological sample, the method comprising:
   (a) contacting the biological sample with a plurality of probes for detecting RNA expression from a plurality of subregions within a genomic region,
      wherein the biological sample is a cell or tissue sample,
      wherein the plurality of probes comprises at least two different probes capable of hybridizing to at least two different RNA molecules expressed from different subregions within the genomic region,
      wherein the plurality of probes individually comprise a common barcode sequence that identifies the genomic region;
   (b) detecting the common barcode sequence or a complement thereof in hybridized probes of the plurality of probes or in products thereof, wherein detecting the barcode sequence or the complement thereof provides a barcode count corresponding to the genomic region for a cell in the biological sample; and
   (c) analyzing the copy number of the genomic region based on the barcode count corresponding to the genomic region for the cell in the biological sample.
Embodiment 2. The method of embodiment 1, wherein (c) comprises inferring a copy number variation for the genomic region in the cell based on the barcode count corresponding to the genomic region.
Embodiment 3. The method of embodiment 1 or 2, wherein the cell in (b) is a cancer cell in the biological sample.
Embodiment 4. The method of embodiment 3, wherein the biological sample further comprises a non-cancer cell, and wherein detecting the common barcode sequence or a complement thereof in hybridized probes of the plurality of probes or in products thereof provides a barcode count corresponding to the genomic region for the non-cancer cell in the biological sample.
Embodiment 5. The method of embodiment 4,wherein the method comprises identifying the cancer cell and the non-cancer cell based on RNA or protein expression of one or more cancer biomarker detected *in situ* in the biological sample.
Embodiment 6. The method of embodiment 4 or embodiment 5, wherein (c) comprises comparing the barcode count for the genomic region in the cancer cell with the barcode count for the genomic region in the non-cancer cell.
Embodiment 7. The method of any of embodiments 3-5, wherein (c) comprises comparing the barcode count for the genomic region in the cancer cell with the barcode count for the genomic region in a cell of a healthy control sample.
Embodiment 8. The method of any of embodiments 2-7, wherein the copy number variation is gain or loss of a chromosome.
Embodiment 9. The method of any of embodiments 2-7, wherein the copy number variation is gain or deletion of a chromosomal segment.
Embodiment 10. The method of any of embodiments 1-9, wherein the different subregions of the contiguous genomic region are separated by at least one gene.
Embodiment 11. The method of any of embodiments 1-10, wherein the different subregions are housekeeping genes within the genomic region.
Embodiment 12. The method of any of embodiments 1-11, wherein the barcode count for the genomic region is at least 10, at least 20, or at least 50.
Embodiment 13. The method of any of embodiments 1-12, wherein the plurality of probes comprises at least 5, at least 10, at least 15, or at least 20 different probes capable of hybridizing to different RNA molecules expressed from different subregions within the genomic region.
Embodiment 14. The method of any of embodiments 1-13, wherein the different subregions span at least 1, at least 10, at least 20, or at least 50 megabases within the genomic region.
Embodiment 15. The method of any of embodiments 1-14, wherein the barcode count for the genomic region is between 10 and 400.
Embodiment 16. The method of any of embodiments 1-8 and 10-15, wherein the copy number variation is gain of chromosome 7 or loss of chromosome 10.
Embodiment 17. The method of any of embodiments 1-16, wherein individual probes of the plurality of probes further comprise a gene-specific barcode sequence.
Embodiment 18. The method of any of embodiments 1-16, wherein the plurality of probes do not comprise a gene-specific barcode sequence.
Embodiment 19. The method of any of embodiments 1-18, wherein the genomic region is a first genomic region and the plurality of probes is a first plurality of probes, and wherein (a) further comprises contacting the biological sample with a second plurality of probes for detecting RNA expression from a plurality of subregions within a second genomic region,
   wherein the second plurality of probes comprises at least two different probes capable of
   hybridizing to different RNA molecules expressed from different subregions within the second genomic region, and
   wherein the second plurality of probes individually comprise a second common barcode sequence that identifies the second genomic region.
Embodiment 20. The method of embodiment 19, wherein the method comprises analyzing the copy number of the second genomic region based on the barcode count corresponding to the second genomic region for the cell in the biological sample.
Embodiment 21. The method of embodiment 19 or 20, wherein the second genomic region is on a different chromosome than the first genomic region.
Embodiment 22. A method for detecting a copy number variation in a cancer cell in a biological sample, the method comprising:
   (a) contacting the biological sample with a plurality of probes for detecting RNA expression from a plurality of subregions within a genomic region,
      wherein the biological sample is a cell or tissue sample comprising the cancer cell and a non-cancer cell,
      wherein the plurality of probes comprises at least two different probes capable of hybridizing to at least two different RNA molecules expressed from different subregions within the genomic region,
      wherein the plurality of probes individually comprise a common barcode sequence that identifies the genomic region;
   (b) detecting the common barcode sequence or a complement thereof in hybridized probes of the plurality of probes or in products thereof, wherein detecting the barcode sequence or the complement thereof provides a barcode count corresponding to the genomic region for the cancer cell and for the non-cancer cell in the biological sample; and
   (c) detecting the copy number variation in the cancer cell by comparing the barcode count corresponding to the genomic region for the cancer cell to the barcode count corresponding to the genomic region for the non-cancer cell.
Embodiment 23. The method of any of embodiments 1-22, wherein the plurality of probes comprises circular probes or circularizable probes or probe sets capable of hybridizing to each of the different RNA molecules expressed from the different subregions within the genomic region.
Embodiment 24. The method of embodiment 23, wherein the method comprises performing rolling circle amplification to generate rolling circle amplification products of the hybridized circular probes or of circularized probes generated from the hybridized circularizable probes or probe sets in the biological sample.
Embodiment 25. The method of embodiment 24, wherein (b) comprises detecting the complement of the common barcode sequence in the rolling circle amplification products.
Embodiment 26. The method of any of embodiments 1-22, wherein the plurality of probes comprises at least two different probe sets, wherein each probe set is capable of hybridizing to a different RNA molecule.
Embodiment 27. The method of embodiment 26, wherein each probe set comprises at least 10, at least 20, or at least 30 probes that hybridize to tiled regions of an individual RNA molecule.
Embodiment 28. The method of any of embodiments 5-27, wherein the method comprises identifying the cancer cell and the non-cancer cell based on RNA expression of one or more cancer biomarker detected *in situ* in the biological sample.
Embodiment 29. The method of embodiment 28, wherein the method comprises detecting the RNA expression of the one or more cancer biomarker by binding one or more detectably labeled probes directly or indirectly to the RNA, to a probe that binds to the RNA, or to an amplification product of a probe that binds to the RNA.
Embodiment 30. The method of any of embodiments 5-27, wherein the method comprises identifying the cancer cell and the non-cancer cell based on protein expression of one or more cancer biomarker detected *in situ* in the biological sample.
Embodiment 31. The method of embodiment 30, wherein the method comprises detecting the protein expression of the one or more cancer biomarker by binding one or more detectably labeled probes directly or indirectly to a labeling agent that binds to the protein, to a probe that binds to the labeling agent, or to an amplification product of a probe that binds to the labeling agent.
Embodiment 32. The method of embodiment 31, wherein the labeling agent comprises a binding moiety that binds to the protein, wherein the binding moiety is an antibody or antigen-binding portion thereof.
Embodiment 33. The method of any one of embodiments 1-32, wherein the biological sample is a tissue section.
Embodiment 34. The method of any one of embodiments 1-33, wherein the biological sample is a formalin-fixed, paraffin-embedded (FFPE) sample or a fresh frozen tissue sample. Embodiment 35. The method of any one of embodiments 1-34, wherein the biological sample is fixed and/or permeabilized.
Embodiment 36. The method of any one of embodiments 1-35, wherein the biological sample is crosslinked and/or embedded in a matrix, optionally wherein the matrix comprises a hydrogel.
Embodiment 37. The method of any one of embodiments 1-36, wherein the biological sample is cleared.
Embodiment 38. A kit, comprising:
   a plurality of probes for detecting RNA expression from a plurality of subregions within a genomic region,
   wherein the plurality of probes comprises at least two different probes capable of hybridizing to at least two different RNA molecules expressed from different subregions within the genomic region,
   wherein the plurality of probes individually comprise a common barcode sequence that identifies the genomic region.
Embodiment 39. The kit of embodiment 38, wherein the plurality of probes is a plurality of circularizable probes or probe sets, wherein the circularizable probes or probe sets comprise nucleic acid hybridization regions complementary to the RNA molecules expressed from different subregions within the genomic region such that the circularizable probes or probe sets can be circularized by ligation upon hybridization to the RNA molecules.
Embodiment 40. The kit of embodiment 39, wherein the ligation is ligation preceded by gap-filling.
Embodiment 41. The kit of embodiment 39, wherein the ligation is not preceded by gap-filling.
Embodiment 42. A method, comprising:
   (a) contacting the biological sample with a plurality of probes for detecting RNA expression from a plurality of subregions within a genomic region,
      wherein the biological sample is a cell or tissue sample,
      wherein the plurality of probes comprises at least two different probes capable of hybridizing to at least two different RNA molecules expressed from different subregions within the genomic region,
      wherein the plurality of probes individually comprise a common barcode sequence that identifies the genomic region; and
   (b) detecting the common barcode sequence or a complement thereof in hybridized probes of the plurality of probes or in products thereof, wherein detecting the barcode sequence or the complement thereof provides a barcode count corresponding to the genomic region for a cell in the biological sample.
Embodiment 43. The method of embodiment 42, wherein the method comprises inferring a copy number variation for the genomic region in the cell based on the barcode count corresponding to the genomic region.
Embodiment 44. The method of embodiment 42 or embodiment 43, wherein the cell in (b) is a cancer cell in the biological sample.
Embodiment 45. The method of embodiment 44, wherein the biological sample further comprises a non-cancer cell, and wherein detecting the common barcode sequence or a complement thereof in hybridized probes of the plurality of probes or in products thereof provides a barcode count corresponding to the genomic region for the non-cancer cell in the biological sample.
Embodiment 46. The method of embodiment 45, wherein the method comprises identifying the cancer cell and the non-cancer cell based on RNA or protein expression of one or more cancer biomarker detected *in situ* in the biological sample.
Embodiment 47. The method of embodiment 45 or embodiment 46, wherein the method comprises comparing the barcode count for the genomic region in the cancer cell with the barcode count for the genomic region in the non-cancer cell.
Embodiment 48. The method of any of embodiments 44-46, wherein the method comprises comparing the barcode count for the genomic region in the cancer cell with the barcode count for the genomic region in a cell of a healthy control sample.
Embodiment 49. The method of any of embodiments 43-48, wherein the copy number variation is gain or loss of a chromosome.
Embodiment 50. The method of any of embodiments 43-49, wherein the copy number variation is gain or deletion of a chromosomal segment.
Embodiment 51. The method of any of embodiments 42-50, wherein the different subregions of the contiguous genomic region are separated by at least one gene.
Embodiment 52. The method of any of embodiments 42-51, wherein the different subregions are housekeeping genes within the genomic region.
Embodiment 53. The method of any of embodiments 42-52, wherein the barcode count for the genomic region is at least 10, at least 20, or at least 50.
Embodiment 54. The method of any of embodiments 42-53, wherein the plurality of probes comprises at least 5, at least 10, at least 15, or at least 20 different probes capable of hybridizing to different RNA molecules expressed from different subregions within the genomic region.
Embodiment 55. The method of any of embodiments 42-54, wherein the different subregions span at least 1, at least 10, at least 20, or at least 50 megabases within the genomic region.
Embodiment 56. The method of any of embodiments 42-55, wherein the barcode count for the genomic region is between 10 and 400.
Embodiment 57. The method of any of embodiments 42-49 and 51-56, wherein the copy number variation is gain of chromosome 7 or loss of chromosome 10.
Embodiment 58. The method of any of embodiments 42-57, wherein individual probes of the plurality of probes further comprise a gene-specific barcode sequence.
Embodiment 59. The method of any of embodiments 42-57, wherein the plurality of probes do not comprise a gene-specific barcode sequence.
Embodiment 60. The method of any of embodiments 42-59, wherein the genomic region is a first genomic region and the plurality of probes is a first plurality of probes, and wherein (a) further comprises contacting the biological sample with a second plurality of probes for detecting RNA expression from a plurality of subregions within a second genomic region,
   wherein the second plurality of probes comprises at least two different probes capable of hybridizing to different RNA molecules expressed from different subregions within the second genomic region, and
   wherein the second plurality of probes individually comprise a second common barcode sequence that identifies the second genomic region.
Embodiment 61. The method of embodiment 60, wherein the method comprises analyzing the copy number of the second genomic region based on the barcode count corresponding to the second genomic region for the cell in the biological sample.
Embodiment 62. The method of embodiment 60 or 61, wherein the second genomic region is on a different chromosome than the first genomic region.
Embodiment 63. A method for detecting a copy number variation in a cancer cell in a biological sample, the method comprising:
   (a) contacting the biological sample with a plurality of probes for detecting RNA expression from a plurality of subregions within a genomic region,
      wherein the biological sample is a cell or tissue sample comprising the cancer cell and a non-cancer cell,
      wherein the plurality of probes comprises at least two different probes capable of hybridizing to at least two different RNA molecules expressed from different subregions within the genomic region,
      wherein the plurality of probes individually comprise a common barcode sequence that identifies the genomic region;
   (b) detecting the common barcode sequence or a complement thereof in hybridized probes of the plurality of probes or in products thereof, wherein detecting the barcode sequence or the complement thereof provides a barcode count corresponding to the genomic region for the cancer cell and for the non-cancer cell in the biological sample; and
   (c) detecting the copy number variation in the cancer cell by comparing the barcode count corresponding to the genomic region for the cancer cell to the barcode count corresponding to the genomic region for the non-cancer cell.
Embodiment 64. The method of any of embodiments 42-63, wherein the plurality of probes comprises circular probes or circularizable probes or probe sets capable of hybridizing to each of the different RNA molecules expressed from the different subregions within the genomic region.
Embodiment 65. The method of embodiment 64, wherein the method comprises performing rolling circle amplification to generate rolling circle amplification products of the hybridized circular probes or of circularized probes generated from the hybridized circularizable probes or probe sets in the biological sample.
Embodiment 66. The method of embodiment 65, wherein (b) comprises detecting the complement of the common barcode sequence in the rolling circle amplification products.
Embodiment 67. The method of any of embodiments 42-63, wherein the plurality of probes comprises at least two different probe sets, wherein each probe set is capable of hybridizing to a different RNA molecule.
Embodiment 68. The method of embodiment 67, wherein each probe set comprises at least 10, at least 20, or at least 30 probes that hybridize to tiled regions of an individual RNA molecule.
Embodiment 69. The method of any of embodiments 46-68, wherein the method comprises identifying the cancer cell and the non-cancer cell based on RNA expression of one or more cancer biomarker detected *in situ* in the biological sample.
Embodiment 70. The method of embodiment 69, wherein the method comprises detecting the RNA expression of the one or more cancer biomarker by binding one or more detectably labeled probes directly or indirectly to the RNA, to a probe that binds to the RNA, or to an amplification product of a probe that binds to the RNA.
Embodiment 71. The method of any of embodiments 46-68, wherein the method comprises identifying the cancer cell and the non-cancer cell based on protein expression of one or more cancer biomarker detected *in situ* in the biological sample.
Embodiment 72. The method of embodiment 71, wherein the method comprises detecting the protein expression of the one or more cancer biomarker by binding one or more detectably labeled probes directly or indirectly to a labeling agent that binds to the protein, to a probe that binds to the labeling agent, or to an amplification product of a probe that binds to the labeling agent.
Embodiment 73. The method of embodiment 72, wherein the labeling agent comprises a binding moiety that binds to the protein, wherein the binding moiety is an antibody or antigen-binding portion thereof.
Embodiment 74. The method of any one of embodiments 42-73, wherein the biological sample is a tissue section.
Embodiment 75. The method of any one of embodiments 42-74, wherein the biological sample is a formalin-fixed, paraffin-embedded (FFPE) sample or a fresh frozen tissue sample. Embodiment 76. The method of any one of embodiments 42-75, wherein the biological sample is fixed and/or permeabilized.
Embodiment 77. The method of any one of embodiments 42-76, wherein the biological sample is crosslinked and/or embedded in a matrix, optionally wherein the matrix comprises a hydrogel.
Embodiment 78. The method of any one of embodiments 42-76, wherein the biological sample is cleared.
Embodiment 79. A kit, comprising:
   a plurality of probes for detecting RNA expression from a plurality of subregions within a genomic region,
   wherein the plurality of probes comprises at least two different probes capable of hybridizing to at least two different RNA molecules expressed from different subregions within the genomic region,
   wherein the plurality of probes individually comprise a common barcode sequence that identifies the genomic region.
Embodiment 80. The kit of embodiment 79, wherein the plurality of probes is a plurality of circularizable probes or probe sets, wherein the circularizable probes or probe sets comprise nucleic acid hybridization regions complementary to the RNA molecules expressed from different subregions within the genomic region such that the circularizable probes or probe sets can be circularized by ligation upon hybridization to the RNA molecules.
Embodiment 81. The kit of embodiment 80, wherein the ligation is ligation preceded by gap-filling.
Embodiment 82. The kit of embodiment 80, wherein the ligation is not preceded by gap-filling.
Embodiment 83. A system, comprising:
   a first plurality of probes for detecting RNA expression from a plurality of subregions within a first genomic region, wherein the first plurality of probes comprises at least two different probes capable of hybridizing to at least two different RNA molecules expressed from different subregions within the first genomic region, and wherein the first plurality of probes individually comprise a first common barcode sequence that identifies the first genomic region; and
   a second plurality of probes for detecting RNA expression from a plurality of subregions within a second genomic region, wherein the second plurality of probes comprises at least two different probes capable of hybridizing to at least two different RNA molecules expressed from different subregions within the second genomic region, and wherein the second plurality of probes individually comprise a second common barcode sequence that identifies the second genomic region.
Embodiment 84. The system of embodiment 83, wherein the first plurality of probes comprises at least 5, at least 10, at least 15, or at least 20 different probes capable of hybridizing to different RNA molecules expressed from different subregions within the genomic region.
Embodiment 85. The system of embodiment 83 or embodiment 84, wherein the second plurality of probes comprises at least 5, at least 10, at least 15, or at least 20 different probes capable of hybridizing to different RNA molecules expressed from different subregions within the genomic region.
Embodiment 86. The system of any one of embodiments 83-85, wherein the different subregions span at least 1, at least 10, at least 20, or at least 50 megabases within the first genomic region or within the second genomic region.
Embodiment 87. The system of any one of embodiments 83-86, wherein individual probes of the first plurality of probes and/or the second plurality of probes further comprise a gene-specific barcode sequence.
Embodiment 88. The system of any one of embodiments 83-86, wherein individual probes of the first plurality of probes and/or the second plurality of probes do not comprise a gene-specific barcode sequence.
Embodiment 89. The system of any one of embodiments 83-88, wherein the first plurality of probes comprises circular probes or circularizable probes or probe sets configured to hybridize to each of the different RNA molecules expressed from the different subregions within the first genomic region.
Embodiment 90. The system of any one of embodiments 83-89, wherein the second plurality of probes comprises circular probes or circularizable probes or probe sets configured to hybridize to each of the different RNA molecules expressed from the different subregions within the second genomic region.
Embodiment 91. The system of embodiment 89 or embodiment 90, further comprising further comprising a ligase for circularizing a nucleic acid molecule of the first plurality of probes or the second plurality of probes.
Embodiment 92. The system of any one of embodiment 89-91, further comprising a polymerase for generating a plurality of rolling circle amplification products.
Embodiment 93. The system of any one of embodiment 83-92, further comprising a plurality of detectably labeled probes that binds directly or indirectly to the first common barcode sequence or the second barcode sequence or a complement thereof in hybridized probes of the first plurality of probes or the second plurality of probes, or in products thereof.
Embodiment 94. The system of any one of embodiment 83-92, further comprising one or more reagents for performing sequencing-by-synthesis (SBS), sequencing-by-avidity (SBA) or sequencing-by-binding (SBB) to detect the first common barcode sequence and/or the second common barcode sequence.
Embodiment 95. A system, comprising:
   a computer system configured to analyze a plurality of probes for detecting RNA expression from a plurality of subregions within a genomic region detected in a biological sample,
   wherein the biological sample is a cell or tissue sample; and
   wherein the plurality of probes individually comprise a common barcode sequence that identifies the genomic region;
   wherein the computer system determines a copy number of the genomic region based on a barcode count corresponding to the genomic region for a cell in the biological sample. Embodiment 96. The system of embodiment 95, wherein the computer module is used to detect the common barcode sequence or a complement thereof to detect the plurality of probes in the cell or tissue sample.
Embodiment 97. The system of embodiment 96, wherein the computer system compares the determined copy number of the genomic region of the cell in the biological sample to an additional determined copy number of an additional genomic region of an additional cell.
Embodiment 98. The system of any one of embodiments 95-97, wherein the computer system identifies the cell in the biological sample as a cancer cell or a non-cancer cell based on the determined copy number.

### EXAMPLES

The following examples are included for illustrative purposes only and are not intended to limit the scope of the present disclosure.

### Example 1: Inferring Copy Number Variations based on RNA expression in situ

This example describes a workflow for analyzing genomic copy number based on RNA expression in a biological sample (e.g., a cell or tissue sample on a substrate).

A breast tissue sample for copy number variation analysis is sectioned and mounted on a slide, fixed (e.g., by incubating in paraformaldehyde (PFA)), washed, and permeabilized (e.g., using Triton-X). In one example, the tissue sample is suspected of having CNV and comprises both cancer cells and non-cancer cells. After permeabilization, the tissue sections are washed, dehydrated, and rehydrated. The sample comprises target RNA molecules expressed from one or more genomic regions of interest for copy number variation analysis.

The example method comprises contacting the biological sample with a plurality of circularizable probes comprising different targeting sequences complementary to different RNAs expressed from a plurality of subregions within a genomic region of interest (as shown in right panel of FIG. 1). Probes are designed to detect a plurality of genes on chromosome 17, including genes ERBB2, RPS6K1, and STARD3. Based on a reference data base or previous sequencing results, at least one of the genes being probed is identified as associated with copy number aberrations (CNAs). The different circularizable probes for detecting the three genes comprise a common barcode sequence corresponding to the genomic region. The circularizable probes are ligated and amplified by rolling circle amplification to generate rolling circle amplification products comprising multiple copies of the complement of the common barcode sequence.

The complement of the common barcode sequence is then detected by binding detectably labeled probes directly or indirectly (e.g., via an intermediate probe) to the complement of the common barcode sequence and imaging the biological sample to determine a barcode count corresponding to the genomic region. In an example, the barcode count is the number of spots detected for the common barcode sequence (e.g., the number of fluorescent spots detected for the common barcode sequence using fluorescently labeled probes). The barcode count for the genomic region in a cancer cell is compared to the barcode count for the genomic region in a non-cancer cell to determine whether the cancer cell contains a copy number variation. For the comparison, the barcode count for the genomic region in a cancer cell is divided by the barcode count for the genomic region in a non-cancer cell to determine a fold copy number change. An increase (e.g., doubling or greater than 1 fold change) of the barcode count in the cancer cell relative to the non-cancer cell corresponds to copy number gain (e.g., duplication), and a decrease (e.g., halving or less than 1 fold change) of the barcode count in the cancer cell relative to the non-cancer cell corresponds to copy number loss. The comparison further comprises performing a statistical calculation to determine a confidence interval. The cancer cell and non-cancer cell are identified based on histology and/or expression of one or more cancer biomarkers (e.g., detected in situ in the biological sample).

The present disclosure is not intended to be limited in scope to the particular disclosed embodiments, which are provided, for example, to illustrate various aspects of the present disclosure. Various modifications to the compositions and methods described will become apparent from the description and teachings herein. Such variations may be practiced without departing from the true scope and spirit of the disclosure and are intended to fall within the scope of the present disclosure.

## Claims

1. A method, comprising:
(a) contacting the biological sample with a plurality of probes for detecting RNA expression from a plurality of subregions within a genomic region,
wherein the biological sample is a cell or tissue sample,
wherein the plurality of probes comprises at least two different probes capable of hybridizing to at least two different RNA molecules expressed from different subregions within the genomic region,
wherein the plurality of probes individually comprise a common barcode sequence that identifies the genomic region; and
(b) detecting the common barcode sequence or a complement thereof in hybridized probes of the plurality of probes or in products thereof, wherein detecting the barcode sequence or the complement thereof provides a barcode count corresponding to the genomic region for a cell in the biological sample.

2. The method of claim 1, wherein the method comprises inferring a copy number variation for the genomic region in the cell based on the barcode count corresponding to the genomic region.

3. The method of claim 1 or 2, wherein the cell in (b) is a cancer cell in the biological sample.

4. The method of claim 3, wherein the biological sample further comprises a non-cancer cell, and wherein detecting the common barcode sequence or a complement thereof in hybridized probes of the plurality of probes or in products thereof provides a barcode count corresponding to the genomic region for the non-cancer cell in the biological sample.

5. The method of claim 4, wherein the method comprises identifying the cancer cell and the non-cancer cell based on RNA or protein expression of one or more cancer biomarker detected *in situ* in the biological sample.

6. The method of claim 4 or claim 5, wherein the method comprises comparing the barcode count for the genomic region in the cancer cell with the barcode count for the genomic region in the non-cancer cell or in a cell of a healthy control sample.

7. The method of any of claims 2-6, wherein the copy number variation is gain or loss of a chromosome.

8. The method of any of claims 2-6, wherein the copy number variation is gain or deletion of a chromosomal segment.

9. The method of any of claims 1-8, wherein the different subregions of the contiguous genomic region are separated by at least one gene.

10. The method of any of claims 1-9, wherein the different subregions are housekeeping genes within the genomic region.

11. The method of any of claims 1-10, wherein:
(a) the plurality of probes comprises at least 5 different probes capable of hybridizing to different RNA molecules expressed from different subregions within the genomic region; and/or
(b) the different subregions span at least 1 megabase within the genomic region.

12. The method of any of claims 1-11, wherein individual probes of the plurality of probes further comprise a gene-specific barcode sequence.

13. The method of any of claims 1-12, wherein the genomic region is a first genomic region and the plurality of probes is a first plurality of probes, and wherein (a) further comprises contacting the biological sample with a second plurality of probes for detecting RNA expression from a plurality of subregions within a second genomic region,
wherein the second plurality of probes comprises at least two different probes capable of hybridizing to different RNA molecules expressed from different subregions within the second genomic region,
wherein the second plurality of probes individually comprise a second common barcode sequence that identifies the second genomic region, and
wherein the method comprises analyzing the copy number of the second genomic region based on the barcode count corresponding to the second genomic region for the cell in the biological sample.

14. The method of any of claims 1-13, wherein the plurality of probes comprises circular probes or circularizable probes or probe sets capable of hybridizing to each of the different RNA molecules expressed from the different subregions within the genomic region, and wherein the method comprises performing rolling circle amplification to generate rolling circle amplification products of the hybridized circular probes or of circularized probes generated from the hybridized circularizable probes or probe sets in the biological sample, and wherein (b) comprises detecting the complement of the common barcode sequence in the rolling circle amplification products.

15. The method of any of claims 1-13, wherein the plurality of probes comprises at least two different probe sets, wherein each probe set is capable of hybridizing to a different RNA molecule, and wherein each probe set comprises at least 10, at least 20, or at least 30 probes that hybridize to tiled regions of an individual RNA molecule.
